(19) Europäisches Patentamt / European Patent Office / Office européen des brevets

(11) **EP 3 365 062 B1**

(12) **EUROPEAN PATENT SPECIFICATION**

(45) Date of publication and mention of the grant of the patent: **18.09.2024 Bulletin 2024/38**

(21) Application number: **16858069.4**

(22) Date of filing: **18.10.2016**

(51) International Patent Classification (IPC):
***A61K 45/06*** *(2006.01)* ***A61K 38/19*** *(2006.01)*
***A61K 39/00*** *(2006.01)* ***A61K 35/76*** *(2015.01)*
***A61K 35/761*** *(2015.01)* ***A61K 39/395*** *(2006.01)*
***A61K 38/20*** *(2006.01)* ***A61K 38/21*** *(2006.01)*
***A61P 35/00*** *(2006.01)* ***C12N 7/01*** *(2006.01)*
***C07K 16/28*** *(2006.01)*

(52) Cooperative Patent Classification (CPC):
(C-Sets available)
**A61K 35/761; A61K 35/74; A61K 38/193; A61K 38/2013; A61K 38/21; A61K 39/39558; A61K 45/06; A61P 35/00; C07K 16/2818;**
C12N 2710/10332; C12N 2710/10343 (Cont.)

(86) International application number:
**PCT/US2016/057526**

(87) International publication number:
**WO 2017/070110 (27.04.2017 Gazette 2017/17)**

(54) **METHODS OF TREATING SOLID OR LYMPHATIC TUMORS BY COMBINATION THERAPY**

VERFAHREN ZUR BEHANDLUNG VON SOLIDEN ODER LYMPHATISCHEN TUMOREN DURCH KOMBINATIONSTHERAPIE

PROCÉDÉS DE TRAITEMENT DE TUMEURS SOLIDES OU LYMPHATIQUES PAR POLYTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2015 US 201562243512 P**

(43) Date of publication of application:
**29.08.2018 Bulletin 2018/35**

(73) Proprietor: **CG Oncology, Inc.**
**Irvine, CA 92618 (US)**

(72) Inventors:
- **YEUNG, Alex Wah, Hin**
  **Irvine, CA 92612 (US)**
- **KUAN, Arthur**
  **Newport Coast, CA 92657 (US)**

(74) Representative: **J A Kemp LLP**
**80 Turnmill Street**
**London EC1M 5QU (GB)**

(56) References cited:
**US-A1- 2003 039 633** **US-A1- 2010 247 440**
**US-A1- 2015 190 505** **US-A1- 2015 190 505**

- **MIKIYA ISHIHARA ET AL: "Systemic CD8+ T Cell-Mediated Tumoricidal Effects by Intratumoral Treatment of Oncolytic Herpes Simplex Virus with the Agonistic Monoclonal Antibody for Murine Glucocorticoid-Induced Tumor Necrosis Factor Receptor", PLOS ONE, vol. 9, no. 8, 8 August 2014 (2014-08-08), pages e104669, XP055465537, DOI: 10.1371/journal.pone.0104669**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SIMONA BRAMANTE: "ONCOLYTIC ADENOVIRUS CODING FOR GM-CSF IN TREATMENT OF CANCER", ACADEMIC DISSERTATION, 2 October 2015 (2015-10-02), HELSINKI, pages 1 - 101, XP002791040, ISSN: 2342-3161, ISBN: 978-951-51-1505-8, Retrieved from the Internet <URL:https://helda.helsinki.fi/bitstream/handle/10138/156397/oncolyti.pdf?sequence=1> [retrieved on 20190506]**
- **DONG ET AL.: "Adenovirus-Mediated E2F-1 Gene Transfer Sensitizes Melanoma Cells to Apoptosis Induced by Topoisomerase II Inhibitors''.", CANCER RESEARCH, vol. 62, no. 6, 15 March 2002 (2002-03-15), pages 1776 - 83, XP001149157**
- **MELERO ET AL.: "Therapeutic Vaccines for Cancer: an Overview of Clinical Trials''.", NATURE REVIEWS CLINICAL ONCOLOGY., vol. 11, no. 9, 1 September 2014 (2014-09-01), pages 509 - 24, XP055377938**


Remarks:

The complete document including Reference Table(s) and the Sequence Listing(s) can be downloaded from the EPO website

(52) Cooperative Patent Classification (CPC): (Cont.)

C-Sets

**A61K 35/74, A61K 2300/00;**
**A61K 35/761, A61K 2300/00;**
**A61K 38/193, A61K 2300/00;**
**A61K 38/2013, A61K 2300/00;**
**A61K 38/21, A61K 2300/00;**
**A61K 39/39558, A61K 2300/00**

**Description**

FIELD OF THE INVENTION

The present invention relates to local administration of a combination of an infectious agent and one or more immunomodulators for cancer immunotherapy.

BACKGROUND OF THE INVENTION

The human immune system of innate and adaptive immunity is an extremely complex system which has not yet been successfully utilized to fight against cancer. One explanation is that, since cancers are usually developed within the later part of life, the development of an immunological response to counteract cancer is not vital to the survival of the fittest theory in the evolutionary process. In all likelihood, the different aspects of the human immune system are not designed specifically for that purpose, meaning to kill cells that are considered as "self". Even after extensive removal of the primary tumor it is still a problem to prevent the formation of metastases either due to growing out of micro-metastases already present at the time of surgery, or to the formation of new metastases by tumor cells or tumor stem cells that have not been removed completely or being re-attached after surgery. In essence, for later stages of cancer, surgery and/or radiotherapy can only take care of the macroscopic lesions, while most patients will have their cancers recurring and not amenable to further therapies.

More recently FDA has approved two immunotherapeutic agents against prostate cancer and melanoma. The first agent, Provenge, utilizes a GM-CSF fusion molecule with a prostatic antigen to activate the mononuclear or antigen presenting cells of late-stage cancer patients in vitro and is able to prolong the overall survival of these patients. The second agent is an anti-CTLA-4 monoclonal antibody, which was shown to produce a profoundly enhancing effect in T effector cell generation. An oncolytic virus CG0070 has also been shown to trigger a long-term complete response among bladder cancer patients after one series of six weekly intravesical treatments (*see* Burke JM, et al. Journal of Urology Dec, 188 (6) 2391-7, 2012).

Current cancer immunotherapy methods face various fundamental challenges. For example, normally tumor-specific immune T lymphocytes in cancer patients, even when they are present, only occur at low frequency systemically. The likely reason is that the antigenicity and specific immunogenicity of common cancers' tumor antigens are generally weak, as well as the presence of an overwhelming amount of suppressor activities through cytokines and regulatory cells, such as Treg, tumor associated macrophages, etc. Additionally, the older concepts of using nonspecific components to boost immune response against specific components were found to have little success, as the ability for a human body to generate very specific immunological responses against its own cells is limited by nature. After all, most cancer cells are not immunogenic enough to be different from normal cells. Such an immune response derived from non-specific immunological components, even if generated, will also be short-lived.

For at least the reasons discussed above, in vitro and pre-formulated therapeutic cancer vaccines using available tumor antigens and adjuvants have been tried for decades without much success. There is a clear need for cancer immunotherapy methods with improved efficacy.

Ishihara M. et al., (2014) PLOS ONE, 9, p e104669 describes systemic CD8+ T cell-mediated tumoricidal effects by intratumoral treatment of oncolytic herpes simplex virus with the agonistic monoclonal antibody for murine glucocorticoid-induced tumor necrosis factor receptor.

Bramante S. Faculty of Medicine of the University of Helsinki Academic Dissertation, 2 October 2015, pages 1-101 describes oncolytic adenovirus coding for GM-CSF in treatmenr of cancer.

US 2015/190505 describes a cancer vaccine to be used to treat bladder cancer comprising administering an oncolytic virus and an anti-CTLA4 antibody.

BRIEF SUMMARY OF THE INVENTION

The present application provides in combination, an infectious agent and immunomodulator for use in a method of treating a solid or lymphatic tumor in an individual, wherein said method comprises: a) locally administering to the site of the tumor an effective amount of said infectious agent, wherein the infectious agent is an oncolytic virus; and b) locally administering to the site of the tumor said effective amount of an immunomodulator, wherein the immunomodulator is an inhibitor of PD-L1. Local administration of the therapeutic components (e.g., an infectious agent, immunomodulator(s), and inactivated tumor cells) to the site of the tumor is a key requirement of this invention.

Without being bound by any theory or hypothesis, the present invention is based in part on an "at" tumor site concept for delivering therapeutic components and eliciting immune response, which can be used to overcome the presumably insurmountable obstacles in treating solid or resistant lymphatic tumors. According to the "at" tumor site concept, the therapeutic components are delivered "at" tumor site, at the right effective amounts, at the right timing, and in the right sequences. The effective amounts, timing, and sequences of the therapeutic components are each independently adjustable based on the specific condition of the tumor. For example, administration of a combination of an infectious agent, immunomodulators and optionally inactivated tumor cells using the "at" tumor site concept can give rise to the right amount of immune-related molecules (such as GM-CSF) secreted from the infectious agent, from the individual's own bodily reactions, and/or from the optionally administered live tumor cells, leading to a release of tolerance breaking antigens (TBAs), which contribute to the immune signal confirmations (such as the 1, 2, 3 signals of CD4 and CD8 T cells) and generation of the effector cells, also "at" the tumor sites. Thereby, a "whole" specific cancer immunotherapy response having strong and durable effects is believed to happen right "at" the tumor site.

Without being bound by any theory or hypothesis, it is believed that the release of the previously unknown tumor-specific or selective tolerance breaking antigens (TBA) by the real time infectious process at tumor sites may play a critical role in this process, as these antigens can only be released at the exact time and site of cell death. The TBAs may consist of antigens derived from tumors or even from structures vital to tumors (such as stromal cells), and the TBAs may not be previously transcribed by the AIRE (autoimmune regulator genes in the thymus) gene. In addition, the release of TBAs is believed to be a transient phenomenon that must be captured at the tumor site.

Under the "at" tumor site concept framework, it is additionally believed that use of the immunomodulators, such as immune checkpoint inhibitors and immune-stimulating agents, can be of immense help to provide synergistic effects with the use of an infectious agent and the inactivated tumor cells right "at" the tumor sites. Depending on the dose, route of administration, and other pharmacokinetic and pharmacodynamic factors, immunomodulators can exert different effects on the body and in particular, on the immune system. The "at" tumor site concept in this invention requires the immunomodulators to be administered with an adjustable dose and schedule, rather than, for example, being expressed at a fixed dosage from a transgene. For example, an increasing dose of IV administration of the anti-CTLA-4 antagonist antibody is associated with a systemic increase of immune suppressor cells, such as Treg. Patients can only derive benefits in local tumor sites and the draining lymph nodes, such as an increase of the CD8/CD4 ratio and upregulation of IL12 and IFNy etc., at a high enough systemic level of the anti-CTLA-4 antibody, which is associated with significant immune related adverse events and exacerbation of auto-immune conditions, including irreversible and fatal events. By contrast, in the present invention, the immunomodulators, such as anti-CTLA-4 antibody, are administered "at" tumor sites so that the "whole" specific cancer immunotherapy response is happening right "at" the tumor sites, including specific cancer cell death "live" mixture and release of TBAs (less normal cell death to confuse the system), "real time" maturation and migration of antigen presenting cells and immune cells, confirmation of immune signals via the immunomodulators (e.g., co-stimulating factors, antagonists of inhibitory checkpoint molecules, and agonists in immune cells activation, functions, survival, expansion and memory). All of the immune events described above are happening right at the tumor site, which is in contrast with the traditional view of the field that relies exclusively on the central or systematic immune response via the secondary lymphoid organs to eradicate tumor cells.

The "at" tumor site concept is further supported by unpublished results from our previous clinical trials of CG0070, which showed constant release of IL6 and not any other cytokine during the course of the treatment. Without being bound by any theory or hypothesis, it is believed that IL6, in combination with TGFβ, shifts Treg and other CD4 cells towards commitment to the Th17 immune pathway. If such shift happens at the right instant of cancer cell death and activation of antigen presenting and immune cells, the Th1 pathway will be confirmed on an auto-immune basis, which is required for the effector T cells to destroy the so-called "self' cancer cells. Otherwise, cancer cells are easily "tolerated" by the effector T cells, when the Th1 pathway is only temporarily conferred without the release of IL6 and commitment to the Th17 pathway. As the expression of IL6 and the shift to the Th17 pathway were observed only at the tumor sites, such results further confirm the importance of an "at" tumor site therapy.

Thus, one aspect of the present application provides in combination, an infectious agent and immunomodulator for use in a method of treating a solid or lymphatic tumor in an individual (e.g., a human individual), comprising: a) locally administering to the site of the tumor an effective amount of said infectious agent, wherein the infectious agent is an oncolytic virus; and b) locally administering to the site of the tumor an effective amount of an immunomodulator (including combination of immunomodulators), wherein the immunomodulator is an inhibitor of PD-L1. A method of inhibiting metastasis of a solid or lymphatic tumor in an individual (e.g., a human individual), comprising: a) locally administering to the site of the tumor an effective amount of an infectious agent; and b) locally administering to the site of the tumor an effective amount of an immunomodulator (including combination of immunomodulators) is described.

In some embodiments according to any one of the methods provided above, the infectious agent is an oncolytic virus, such as a virus selected from the group consisting of adenovirus, herpes simplex virus, vaccinia virus, mumps virus, newcastle disease virus, polio virus, measles virus, Seneca valley virus, coxsackie virus, reo virus, vesicular stomatitis virus, maraba and rhabdovirus, and parvovirus.

In some embodiments, the oncolytic virus is an oncolytic adenovirus. In some embodiments, the oncolytic virus preferentially replicates in a cancer cell. In some embodiments, the oncolytic virus comprises a viral vector comprising a tumor cell-specific promoter operably linked to a viral gene essential for replication of the virus. In some embodiments, the tumor-specific promoter is an E2F-1 promoter. In some embodiments, the tumor-specific promoter is a human E2F-1 promoter. In some embodiments, the E2F-1 promoter comprises the nucleotide sequence set forth in SEQ ID NO:1. In some embodiments, the viral gene essential for replication of the virus is selected from the group consisting of E1A, E1B, and E4.

In some embodiments according to any one of the methods provided above, the infectious agent and/or the immunomodulator is administered directly into the tumor. In some embodiments, the infectious agent is administered directly into the tumor. In some embodiments, the immunomodulator is administered directly into the tumor.

In some embodiments according to any one of the methods provided above, the infectious agent and/or the immunomodulator is administered to the tissue having the tumor. In some embodiments, the infectious agent is administered to the tissue having the tumor. In some embodiments, the immunomodulator is administered to the tissue having the tumor.

In some embodiments according to any one of the methods provided above, the infectious agent and the immunomodulator are administered sequentially. In some embodiments, the infectious agent is administered prior to the administration of the immunomodulator. In some embodiments, the infectious agent is administered after the administration of the immunomodulator.

In some embodiments according to any one of the methods provided above, the infectious agent and the immunomodulator are administered simultaneously. In some embodiments, the infectious agent and the immunomodulator are administered in the same composition.

In some embodiments according to any one of the methods provided above, the immunomodulator is a modulator of the immune checkpoint molecule PD-L1. In some embodiments, the method comprises local administration of at least two immunomodulators, wherein the method comprises local administration of a CTLA-4 inhibitor and a PD-L1 inhibitor. In some embodiments, the inhibitor of CTLA-4, is an anti-CTLA-4 antibody (e.g., Ipilimumab). In some embodiments, the anti-CTLA-4 antibody is selected from the group consisting of Ipilimumab, Tremilimumab, and a single chain anti-CTLA-4 antibody. In some embodiments, the inhibitor of CTLA-4 is an engineered lipocalin protein specifically recognizing CTLA-4, such as an anticalin molecule that specifically binds to CTLA-4.

In some embodiments according to any one of the methods provided above, the method further comprises locally administering to the site of the tumor an immune-related molecule (such as cytokine, chemokine, or a PRRago (i.e., pathogen recognition receptor agonist)). In some embodiments, the immune-related molecule is selected from the group consisting of GM-CSF, IL-2, IL-12, interferon (such as Type 1, Type 2 or Type 3 interferon, e.g., interferon γ), CCL4, CCL19, CCL21, CXCL13, TLR1, TLR2, TLR3, TLR4, TLR5, TLR6, TLR7, TLR8, TLR9, TLR10, RIG-I, MDA5, LGP2, and LTαβ. In some embodiments, the immune-related molecule is administered separately from the infectious agent. In some embodiments, the immune-related molecule is expressed by the infectious agent, wherein the infectious agent comprises a nucleic acid encoding the immune-related molecule. In some embodiments, the immune-related molecule is selected from the group consisting of STING (i.e., stimulator of interferon genes) activators (such as CDN), PRRago (such as CpG, Imiquimod, or Poly I:C), TLR stimulators (such as GS-9620, AED-1419, CYT-003-QbG10, AVE-0675, or PF-7909), and RLR (i.e., Rig-I-like receptor) stimulators (such as RIG-I, Mda5, or LGP2 stimulators).

In some embodiments according to any one of the methods provided above, the infectious agent is a virus comprising a viral vector, and wherein the viral vector comprises the nucleic acid encoding the immune-related molecule (such as cytokine or chemokine). In some embodiments, the nucleic acid encoding the immune-related molecule is operably linked to a viral promoter. In some embodiments, the virus is an adenovirus, and the viral promoter is an E3 promoter. In some embodiments, the infectious agent is an adenovirus serotype 5, wherein the endogenous E1a promoter and E3 19kD coding region of a native adenovirus is replaced by the human E2F-1 promoter and a nucleic acid encoding human GM-CSF. In some embodiments, the infectious agent is CG0070.

In some embodiments according to any one of the methods provided above, the method further comprises locally administering to the site of the tumor a pretreatment composition prior to the administration of the infectious agent. In some embodiments, the pretreatment composition comprises a transduction enhancing agent, such as N-Dodecyl-β-D-maltoside (DDM).

In some embodiments according to any one of the methods provided above, the individual (e.g., wholly or only at the site of the tumor) is subject to a prior therapy prior to the administration of the infectious agent and the immunomodulator. In some embodiments, the prior therapy is radiation therapy (e.g., with or without chemotherapy). In some embodiments, the prior therapy comprises administration of a therapeutic agent. In some embodiments, the therapeutic agent is an agent that increases the level of cytokines involved an immunogenic pathway. In some embodiments, the therapeutic agent is an agent that causes dysfunction or damage to a structural component of a tumor. In some embodiments, the therapeutic agent is selected from the group consisting of an anti-VEGF antibody, a hyaluronidase, CCL21, and N-dodecyl-β-maltoside. In some embodiments, the prior therapy is provided at a dose that is insufficient to eradicate the tumor cells.

In some embodiments according to any one of the methods provided above, the method further comprises locally administering to the site of the tumor an effective amount of inactivated tumor cells. In some embodiments, the inactivated tumor cells are autologous. In some embodiments, the inactivated tumor cells are allogenic. In some embodiments, the inactivated tumor cells are from a tumor cell line. In some embodiments, the inactivated tumor cells are inactivated by irradiation.

In some embodiments according to any one of the methods provided above, the infectious agent and the inactivated tumor cells are administered simultaneously. In some embodiments, the infectious agent and the inactivated tumor cells are administered as a single composition. In some embodiments, the infectious agent and the inactivated tumor cells are admixed immediately prior to the administration.

In some embodiments according to any one of the methods provided above, the solid or lymphatic tumor is bladder cancer (such as muscle invasive bladder cancer, or non-muscle invasive bladder cancer). In some embodiments, the infectious agent is administered intravesically. In some embodiments, the immunomodulator is administered intravesically.

In some embodiments according to any one of the methods provided above, the infectious agent and/or the immunomodulator is administered weekly.

In some embodiments according to any one of the methods provided above, the individual has high expression of one or more biomarkers selected from PD-1, PD-L1, and PD-L2 in the tumor (such as tumor cells or immune cells derived from the tumor). In some embodiments, the individual has high expression of one or more biomarkers selected from CD80, CD83, CD86, and HLA-Class II antigens in tumor-derived mature dendritic cells. In some embodiments, the individual has high expression of one or more biomarkers selected from the group consisting of CXCL9, CXCL10, CXCL11, CCR7, CCL5, CCL8, SOD2, MT2A, OASL, GBP1, HES4, MTIB, MTIE, MTIG, MTIH, GADD45A, LAMP3 and miR-155.

Also provided is an infectious agent for use in a method of treating a solid or lymphatic tumor in an individual, wherein said method comprises: a) locally administering to the site of the tumor an effective amount of said infectious agent, wherein the infectious agent is an oncolytic virus; and b) locally administering to the site of the tumor said effective amount of an immunomodulator, wherein the immunomodulator is an inhibitor of PD-L1. Also provided is an immunomodulator for use in a method of treating a solid or lymphatic tumor in an individual, wherein said method comprises: a) locally administering to the site of the tumor an effective amount of an infectious agent, wherein the infectious agent is an oncolytic virus; and b) locally administering to the site of the tumor said effective amount of said immunomodulator, wherein the immunomodulator is an inhibitor of PD-L1.

These and other aspects and advantages of the present invention will become apparent from the subsequent detailed description and the appended claims. It is to be understood that one, some, or all of the properties of the various embodiments described herein may be combined to form other embodiments of the present invention.

## BRIEF DESCRIPTION OF THE DRAWINGS

FIG. 1 is a schematic diagram of CG0070 and wild type (wt) adenovirus type 5. CG0070 is based on adenovirus serotype 5, but the endogenous E1a promoter and E3 19kD coding region have been replaced by the human E2F-1 promoter and a cDNA coding region of human GM-CSF, respectively.

FIG. 2 shows animal groups and dosing schemes in the *in vivo* study of Example 9.

FIG. 3 is a scatter diagram showing distribution of enumerated metastatic foci for each animal group on day 23 in the *in vivo* study of Example 9. The horizontal line corresponds to the mean value. Two-tailed statistical analyses were conducted at P = 0.05. Test results are considered not significant (ns) at P > 0.05, significant (symbolized by *) at 0.01 < P < 0.05, very significant (**) at 0.001 < P < 0.01, and extremely significant (***) at P < 0.001.

FIG. 4 is a box and whisker plot showing tumor volumes of each animal group on day 19 in the *in vivo* study of Example 9. The box represents the $25^{th}$ and $75^{th}$ percentile of observations, the line represents the median of observations, and the whisker represents extreme observations.

FIG. 5 is a diagram showing dosing schedule of the *in vivo* study in Example 10.

## DETAILED DESCRIPTION OF THE INVENTION

The present invention provides in combination, an infectious agent and immunomodulator for use in a method of treating a solid or lymphatic tumor in an individual, wherein said method comprises: a) locally administering to the site of the tumor an effective amount of said infectious agent, wherein the infectious agent is an oncolytic virus; and b) locally administering to the site of the tumor said effective amount of an immunomodulator, wherein the immunomodulator is an inhibitor of PD-L1. The combination for use, infectious agent for use, and immunomodulator for use, may further comprise local administration of inactivated tumor cells. The infectious agent and/or the immunomodulator and/or the inactivated tumor cells can be directly administered into the tumor. Alternatively, the infectious agent and/or the immunomodulator and/or the inactivated tumor cells are administered to the tissue having the tumor cell. For example, one exemplary tumor suitable for methods described herein is bladder cancer, and the infectious agent, and/or the immunomodulator can be administered intravesically.

The present invention provides a live and real time "in vivo" cancer vaccine system generated inside a human body by local (such as intratumoral) delivery of therapeutic components, including an infectious agent, one or more agents of immunomodulation, and live cancer cells. Without being bound by any theory or hypothesis, such an *in vivo* on site and real time infectious system is believed to give rise to a release of previously unknown tolerance breaking antigens ("TBAs"), which can be essentially a transient phenomenon. As such, when all three components described herein (infectious agent, immunomodulator(s), and live cancer cells either present at the tumor site or administered to the tumor site) are present, an effective adaptive immunotherapy against solid and lymphatic tumor can be achieved.

One requirement for the methods described herein is to locally administer the infectious agent, the immunomodulator (including combination of immunomodulators), and optionally the inactivated tumor cells to the site of the tumor. The direct effect of local administration is important because, if the components are not provided directly to tumor cells (e.g., when administered systematically), there would be pharmacokinetic and pharmacodynamics changes in these components by, or on, the human body. These changes would tip the fine balance between tumor suppression and activation in a wrong direction of the complicated as well as delicate immunological response required for success.

It is thus believed that the combination described herein would allow full exploitation of the oncolytic and immunogenic reactions in the individual, and increase the therapeutic potential of cancer immunotherapy. It is to be understood by a person of ordinary skill in the art that the combination therapy methods described herein requires that one agent or composition be administered in conjunction with another agent. The dosage, dosing schedule, routes of administration, and sequence of administration for each agent in the combination therapy provided herein (such as the infectious agent, each immunomodulator, and the inactivated tumor cells) can be independently optimized to provide optimal therapeutic results. The methods may also be further combined with pretreatment, such as local radiation, or local administration of cytokines, chemokines, or other beneficial therapeutic agent, to increase the chance of success for the therapy.

In one aspect, there is provided a in combination, an infectious agent and immunomodulator for use in method of treating a solid or lymphatic tumor in an individual, comprising: a) locally administering to the site of the tumor an effective amount of said infectious agent, wherein the infectious agent is an oncolytic virus; and b) locally administering to the site of the tumor an effective amount of an immunomodulator (including combination of immunomodulators), wherein the immunomodulator is an inhibitor of PD-L1. In some embodiments, there is provided said combination for use wherein the method further comprises method c) locally administering to the site of the tumor an effective amount of inactivated tumor cells. In some embodiments, there is provided said combination for use in a method of treating bladder cancer in an individual, comprising: a) intravesically administering an effective amount of an infectious agent, wherein the infectious agent is an oncolytic virus; and b) intravesically administering an effective amount of an immunomodulator (including combination of immunomodulators), wherein the immunomodulator is an inhibitor of PD-L1.

## Definitions

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (*e.g.*, preventing or delaying the worsening of the disease), preventing or delaying the spread (*e.g.*, metastasis) of the disease, preventing or delaying the recurrence of the disease, reducing recurrence rate of the disease, delay or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. Also encompassed by "treatment" is a reduction of pathological consequence of cancer. The methods of the invention contemplate any one or more of these aspects of treatment.

"Adjuvant setting" refers to a clinical setting in which an individual has had a history of cancer, and generally (but not necessarily) been responsive to therapy, which includes, but is not limited to, surgery (*e.g.*, surgery resection), radiotherapy, and chemotherapy. Treatment or administration in the "adjuvant setting" refers to a subsequent mode of treatment.

"Neoadjuvant setting" refers to a clinical setting in which the method is carried out before the primary/definitive therapy. Neoadjuvant setting herein also refers to any "tumor site preparation" therapy modality that is used in conjunction with, in a sequential manner, with the therapeutic components (e.g., infectious agent and immunomodulator(s); or infectious agent, immunomodulator(s) and inactivated tumor cells) as described in this invention.

"Infectious agent" as used herein can refer to a virus, including a non-oncolytic virus, or an oncolytic virus, including, but not limited to, adenovirus, herpes simplex virus, vaccinia virus, mumps virus, newcastle disease virus, polio virus, measles virus, Seneca valley virus, coxsackie virus, reo virus, vesicular stomatitis virus, maraba and rhabdovirus, and parvovirus. In addition, the infectious agent can also be a bacterium, such as Bacillus Calmette-Guerin (BCG), Mycobacterial cell wall-DNA complex ("MCNA"), or Listeria monocytogene.

The term "effective amount" used herein refers to an amount of a compound or composition sufficient to treat a specified disorder, condition or disease such as ameliorate, palliate, lessen, and/or delay one or more of its symptoms. In reference to cancer, an effective amount comprises an amount sufficient to cause a tumor to shrink and/or to decrease the growth rate of the tumor (such as to suppress tumor growth) or to prevent or delay other unwanted cell proliferation in cancer. In some embodiments, an effective amount is an amount sufficient to delay development of cancer. In some embodiments, an effective amount is an amount sufficient to prevent or delay recurrence. In some embodiments, an effective amount is an amount sufficient to reduce recurrence rate in the individual. In some embodiments, the effective amount is an amount sufficient to inhibit tumor metastasis in the individual. An effective amount can be administered in one or more administrations. The effective amount of the drug or composition may: (i) reduce the number of cancer cells; (ii) reduce tumor size; (iii) inhibit, retard, slow to some extent and preferably stop cancer cell infiltration into peripheral organs; (iv) inhibit (*i.e*., slow to some extent and preferably stop) tumor metastasis; (v) inhibit tumor growth; (vi) prevent occurrence and/or recurrence of tumor; (vii) delay occurrence and/or recurrence of tumor; (viii) reduce recurrence rate of tumor, and/or (ix) relieve to some extent one or more of the symptoms associated with the cancer. As is understood in the art, an "effective amount" may be in one or more doses, *i.e.,* a single dose or multiple doses may be required to achieve the desired treatment endpoint.

"In conjunction with" or "in combination with" refers to administration of one treatment modality in addition to another treatment modality, such as administration of an infectious agent described herein in addition to administration of the other agent (such as immunomodulator and/or inactivated tumor cells) to the same individual under the same treatment plan. As such, "in conjunction with" or "in combination with" refers to administration of one treatment modality before, during or after delivery of the other treatment modality to the individual.

The term "simultaneous administration," as used herein, means that a first therapy and second therapy in a combination therapy are administered at the same time. When the first and second therapies are administered simultaneously, the first and second therapies may be contained in the same composition (*e.g*., a composition comprising both a first and second therapy) or in separate compositions (*e.g*., a first therapy is contained in one composition and a second therapy is contained in another composition).

As used herein, the term "sequential administration" or "in sequence" means that the first therapy and second therapy in a combination therapy are administered with a time separation, for example, of more than about 1 minute, such as more than about any of 5, 10, 15, 20, 30, 40, 50, 60, or more minutes. In some cases, the term "sequential administration" means that the first therapy and second therapy in a combination therapy are administered with a time separation of more than about 1 day, such as more than about any of 1 day to 1 week, 2 weeks, 3 weeks, 4 weeks, 8 weeks, 12 weeks, or more weeks. Either the first therapy or the second therapy may be administered first.

The term "administered immediately prior to" means that the first therapy is administered no more than about 15 minutes, such as no more than about any of 10, 5 or 1 minutes before administration of the second therapy. The term "administered immediately after" means that the first therapy is administered no more than about 15 minutes, such as no more than about any of 15, 10 or 1 minutes after administration of the second therapy.

As used herein, "specific", "specificity", or "selective" or "selectivity" as used when describing a compound as an inhibitor, means that the compound preferably interacts with (*e.g.,* binds to, modulates, and inhibits) a particular target (*e.g.,* a protein and an enzyme) than a non-target.

The term "transduction" and "transfection" as used herein include all methods known in the art using an infectious agent (such as a virus) or other means to introduce DNA into cells for expression of a protein or molecule of interest. Besides a virus or virus like agent, there are chemical-based transfection methods, such as those using calcium phosphate, dendrimers, liposomes, or cationic polymers (e.g., DEAE-dextran or polyethylenimine); non-chemical methods, such as electroporation, cell squeezing, sonoporation, optical transfection, impalefection, protoplast fusion, delivery of plasmids, or transposons; particle-based methods, such as using a gene gun, magnectofection or magnet assisted transfection, particle bombardment; and hybrid methods, such as nucleofection.

The term "tumor site preparation" as used herein, describes single treatment modality or combination of more than one treatment modalities to be used in conjunction with the therapeutic components (e.g., infectious agent and immunomodulator(s); or infectious agent, immunomodulator(s) and inactivated tumor cells) in a sequential manner, and in which the treatment modality or modalities are being applied directly or indirectly (e.g., through an IV therapy) to the tumor site (such as cancer cells or the tissue containing the cancer cells). Exemplary treatment modalities for tumor site preparations include, but are not limited to, administration of immune-related molecules, irradiation, and administration of therapeutic agents. All tumor site preparations described herein may include administration of a single molecule or agent, or a combination of more than one molecules and/or agents.

It is understood that embodiments of the invention described herein include "consisting" and/or "consisting essentially of" embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat cancer of type X means the method is used to treat cancer of types other than X.

The term "about X-Y" used herein has the same meaning as "about X to about Y."

As used herein and in the appended claims, the singular forms "a," "or," and "the" include plural referents unless the context clearly dictates otherwise.

**Medical uses for treating a solid or lymphatic tumor**

The present invention provides in combination, an infectious agent and immunomodulator for use in a method of treating a solid or lymphatic tumor (such as bladder cancer) in an individual (such as a human), comprising: a) locally administering to the site of the tumor an effective amount of said infectious agent wherein the infectious agent is an oncolytic virus; and b) locally administering to the site of the tumor an effective amount of an immunomodulator (including combination of immunomodulators), wherein the immunomodulator is an inhibitor of PD-L1. In some embodiments, the infectious agent is a virus, such as a virus selected from the group consisting of adenovirus, herpes simplex virus, vaccinia virus, mumps virus, newcastle disease virus, polio virus, measles virus, Seneca valley virus, coxsackie virus, reo virus, vesicular stomatitis virus, maraba and rhabdovirus, and parvovirus. In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the infectious agent is only a part, or parts of the wild type infectious agent that can cause infection, inflammation or infection-like effects. In some embodiments, the immunomodulator is an immune checkpoint inhibitor. In some embodiments, the infectious agent and/or the immunomodulator (including combination of immunomodulators) are administered directly into the tumor. In some embodiments, the infectious agent and/or immunomodulator (including combination of immunomodulators) are administered to the tissue having the tumor. In some embodiments, both the infectious agent and the immunomodulator (including combination of immunomodulators) are administered directly into the tumor. In some embodiments, both the infectious agent and the immunomodulator (including combination of immunomodulators) are administered to the tissue having the tumor. In some embodiments, the infectious agent is administered weekly. In some embodiments, the immunomodulator (including combination of immunomodulators) is administered weekly. In some embodiments, the method further comprises administration of the infectious agent and/or the immunomodulator (including combination of immunomodulators) by an administration route other than local administration.

Exemplary viruses that are suitable for use as the infectious agent in the present invention include, but are not limited to, adenovirus, for example, H101 (ONCOCRINE®), CG-TG-102 (Ad5/3-D24-GM-CSF), and CG0070; herpes simplex virus, for example, Talimogene laherparapvec (T-VEC) and HSV-1716 (SEPREHVIR®); reo virus, for example, REOLYSIN®; vaccinia virus, for example, JX-594; Seneca valley virus, for example, NTX-010 and SVV-001; newcastle disease virus, for example, NDV-NS1 and GL-ONC1; polio virus, for example, PVS-RIPO; measles virus, for example, MV-NIS; coxsackie virus, for example, Cavatak™; vesicular stomatitis virus; maraba and rhabdoviruses; parvovirus and mumps virus. In some embodiments, the virus is an oncolytic virus. In some embodiments, the virus is replication competent. In some embodiments, the virus replicates preferentially in a tumor cell.

In some embodiments, the oncolytic virus is a wild type oncolytic virus. In some embodiments, the oncolytic virus is genetically modified. In some embodiments, the oncolytic virus is attenuated (for example through multiple passages, inactivation or genetic modification). In some embodiments, the oncolytic virus is replication competent. In some embodiments, the oncolytic virus preferentially replicates in a cancer cell. In some embodiments, the immunomodulator is an immune checkpoint inhibitor. In some embodiments, the oncolytic virus and/or the immunomodulator (including combination of immunomodulators) are administered directly into the tumor. In some embodiments, the oncolytic virus and/or the immunomodulator (including combination of immunomodulators) are administered to the tissue having the tumor. In some embodiments, both the oncolytic virus and the immunomodulator (including combination of immunomodulators) are administered directly into the tumor. In some embodiments, both the oncolytic virus and the immunomodulator (including combination of immunomodulators) are administered to the tissue having the tumor. In some embodiments, the oncolytic virus is administered weekly. In some embodiments, the immunomodulator (including combination of immunomodulators) is administered weekly. In some embodiments, the method further comprises administration of the oncolytic virus and/or the immunomodulator (including combination of immunomodulators) by an administration route other than local administration.

In some embodiments, there is provided in combination, an infectious agent and immunomodulator for use in a method of treating a solid or lymphatic tumor in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus; and b) locally administering to the site of the tumor an effective amount of an immunomodulator (including combination of immunomodulators),

wherein the immunomodulator is an inhibitor of PD-L1. In some embodiments, the immunomodulator is an immune checkpoint inhibitor. In some embodiments, the tumor-specific promoter is an E2F-1 promoter, such as a human E2F-1 promoter or an E2F-1 promoter comprising the nucleotide sequence set forth in SEQ ID NO:1. In some embodiments, the viral gene essential for replication of the virus is selected from the group consisting of E1A, E1B, and E4. In some embodiments, the oncolytic virus and/or the immunomodulator (including combination of immunomodulators) are administered directly into the tumor. In some embodiments, the oncolytic virus and/or immunomodulator (including combination of immunomodulators) are administered to the tissue having the tumor. In some embodiments, both the oncolytic virus and the immunomodulator (including combination of immunomodulators) are administered directly into the tumor. In some embodiments, both the oncolytic virus and the immunomodulator (including combination of immunomodulators) are administered to the tissue having the tumor. In some embodiments, the oncolytic virus is administered weekly. In some embodiments, the immunomodulator (including combination of immunomodulators) is administered weekly. In some embodiments, the method further comprises administration of the oncolytic virus and/or the immunomodulator (including combination of immunomodulators) by an administration route other than local administration.

In some embodiments, the methods described herein further comprise locally administering to the site of the tumor an immune-related molecule (such as cytokine, chemokine, or PRRago (i.e., pathogen recognition receptor agonist)). In some embodiments, the immune-related molecule is selected from the group consisting of GM-CSF, IL-2, IL-12, interferon (such as Type 1, Type 2 or Type 3 interferon, e.g., interferon $\gamma$), CCL4, CCL19, CCL21, CXCL13, TLR1, TLR2, TLR3, TLR4, TLR5, TLR6, TLR7, TLR8, TLR9, TLR10, RIG-I, MDA5, LGP2, and LT$\alpha\beta$. In some embodiments, the immune-related molecule is selected from the group consisting of STING (i.e., stimulator of interferon genes) activators (such as CDN, i.e., cyclic dinucleotides), PRRago (such as CpG, Imiquimod, or Poly I:C), TLR stimulators (such as GS-9620, AED-1419, CYT-003-QbG10, AVE-0675, or PF-7909), and RLR stimulators (such as RIG-I, Mda5, or LGP2 stimulators). In some embodiments, the immune-related molecule induces dendritic cells, T cells, B cells, and/or T follicular helper cells. In some embodiments, the immune-related molecule is administered separately from the infectious agent (e.g., in a separate composition or as a separate entity in the same composition). In some embodiments, the immune-related molecule is administered to the site of the tumor via transduction. Exemplary transduction methods known in the art include, but are not limited to, the use of calcium phosphate, dendrimers, liposomes, cationic polymers, electroporation, cell squeezing, sonoporation, optical transfection, protoplast fusion, impalefection, hydrodynamic delivery, gene gun, magnetofection, viral transfection and nucleofection. In some embodiments, the immune-related molecule is expressed by the infectious agent. For example, the infectious agent may comprise a nucleic acid encoding the immune-related molecule, and the nucleic acid can be in the viral vector or on a separate vector. In some embodiments, the infectious agent is a virus comprising a viral vector, and wherein the viral vector comprises the nucleic acid encoding the immune-related molecule. In some embodiments, the nucleic acid encoding the immune-related molecule is operably linked to a viral promoter, such as an E1 promoter, or an E3 promoter.

The present invention is based in part on unpublished results from our clinical trials performed between 2005 and 2008. Without being bound by any theory or hypothesis, it is believed that the viral infectious agent, CG0070, which is specifically designed to replicate only in cancer cells, provides the "right amount" of GM-CSF at tumor sites and in "real time" during cancer cell death. This "at" tumor site delivery of GM-CSF by the infectious agent during cancer cell death is believed to be vital for antigen presenting cells to both mature and to cross present established antigens, neoantigens, and tolerance breaking antigens (TBA) from this cell death mixture to the activated T cells. The right amount of GM-CSF is needed at the tumor site in this therapeutic scenario, because a high dose of GM-CSF would render the immune system without a focus, and trigger an instantaneous increase of local and system suppressors; whereas a low dose of GM-CSF would not be enough for the activation of the inflammatory process and the related immune cells. A delicate balance at the tumor site involving the right amount of GM-CSF and the on-site "live" cancer cell death mixture is believed to elicit an adaptive immune response that is specific to cancer cells. Therefore, an infectious agent that is cancer specific and oncolytic, and in combination with the right amount of GM-CSF or other appropriate immune-related molecules either expressed by the infectious agent or secreted by body defense in response to any infectious agent during cell death, infection or inflammation, delivered "at" the tumor sites, are believed to be an ideal choice for effective cancer immunotherapy.

In some embodiments, the immune-related molecule enhances an immune response in the individual. Immune-related molecules may include, but are not limited to, a cytokine, a chemokine, a stem cell growth factor, a lymphotoxin, an hematopoietic factor, a colony stimulating factor (CSF), erythropoietin, thrombopoietin, tumor necrosis factor-alpha (TNF), TNF-beta , granulocyte-colony stimulating factor (G-CSF), granulocyte macrophage-colony stimulating factor (GM-CSF), interferon-alpha, interferon-beta, interferon-gamma, interferon-lambda, stem cell growth factor designated "S1 factor", human growth hormone, N-methionyl human growth hormone, bovine growth hormone, parathyroid hormone, thyroxine, insulin, proinsulin, relaxin, prorelaxin, follicle stimulating hormone (FSH), thyroid stimulating hormone (TSH), luteinizing hormone (LH), hepatic growth factor, prostaglandin, fibroblast growth factor, prolactin, placental lactogen, OB protein, mullerian-inhibiting substance, mouse gonadotropin-associated peptide, inhibin, activin, vascular endothelial growth factor, integrin, NGF-beta , platelet-growth factor, TGF-alpha , TGF-beta , insulin-like growth factor-1, insulin-like growth factor-II, macrophage-CSF (M-CSF), IL-1, IL-1a, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, angiostatin, thrombospondin, endostatin, lymphotoxin, thalidomide, lenalidomide, or pomalidomide.

The immune-related molecule can be of any one of the molecular modalities known in the art, including, but not limited to, aptamer, mRNA, siRNA, microRNA, shRNA, peptide, antibody, anticalin, Spherical nucleic acid, TALEN, Zinc Finger Nuclease, CRISPR/Cas9, and small molecule.

The immune-related molecules can be used singly or in combination. For example, any number (such as any of 1, 2, 3, 4, 5, 6, or more) of immune-related molecules can be used simultaneously or sequentially.

Thus, for example, in some embodiments, there is provided a method of treating a solid or lymphatic tumor in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus and a nucleic acid encoding an immune-related molecule (such as cytokine or chemokine) operably linked to a viral promoter; and b) locally administering to the site of the tumor an effective amount of an immunomodulator (including combination of immunomodulators), wherein the immunomodulator is an inhibitor of PD-L1. In some embodiments, the immunomodulator is an immune checkpoint inhibitor. In some embodiments, the tumor-specific promoter is an E2F-1 promoter, such as a human E2F-1 promoter or an E2F-1 promoter comprising the nucleotide sequence set forth in SEQ ID NO:1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the immune-related molecule is GM-CSF. In some embodiments, the oncolytic virus and/or the immunomodulator (including combination of immunomodulators) are administered directly into the tumor. In some embodiments, the oncolytic virus and/or immunomodulator (including combination of immunomodulators) are administered to the tissue having the tumor. In some embodiments, both the oncolytic virus and the immunomodulator (including combination of immunomodulators) are administered directly into the tumor. In some embodiments, both the oncolytic virus and the immunomodulator (including combination of immunomodulators) are administered to the tissue having the tumor. In some embodiments, the oncolytic virus is administered weekly. In some embodiments, the immunomodulator (including combination of immunomodulators) is administered weekly. In some embodiments, the method further comprises administration of the oncolytic virus and/or the immunomodulator (including combination of immunomodulators) by an administration route other than local administration.

In some embodiments, the infectious agent is an adenovirus serotype 5. In some embodiments, the endogenous E1a promoter and E3 19kD coding region of a native adenovirus is replaced by the human E2F-1 promoter and a nucleic acid encoding human GM-CSF. In some embodiments, a polyadenylation signal (PA) is inserted 5' of the E2F-1 promoter. In some embodiments, the nucleic acid encoding human GM-CSF is operably linked to the E3 promoter. In some embodiments, the vector backbone of the adenovirus serotype 5 further comprises E2, E4, late protein regions or inverted terminal repeats (ITRs) identical to the wildtype adenovirus serotype 5 genome. In some embodiments, the infectious agent has the genomic structure as shown in Figure 1. In some embodiments, the infectious agent is conditionally replicating. In some embodiments, the infectious agent preferentially replicates in cancer cells. In some embodiments, the cancer cells are Rb pathway-defective cancer cells. In some embodiments, the infectious agent is CG0070.

Thus, for example, in some embodiments, there is provided in combination, an infectious agent and immunomodulator for use in a method of treating a solid or lymphatic tumor in an individual, comprising: a) locally administering to the site of the tumor an effective amount of an adenovirus serotype 5, wherein the endogenous E1a promoter and E3 19kD coding region of a native adenovirus is replaced by the human E2F-1 promoter and a nucleic acid encoding an immune-related molecule (such as cytokine or chemokine, for example, GM-CSF); and b) locally administering to the site of the tumor an effective amount of an immunomodulator (including combination of immunomodulators), wherein the immunomodulator is an inhibitor of PD-L1. In some embodiments, the immunomodulator is an immune checkpoint inhibitor. In some embodiments, the tumor-specific promoter is a human E2F-1 promoter or an E2F-1 promoter comprising the nucleotide sequence set forth in SEQ ID NO:1. In some embodiments, the adenovirus and/or the immunomodulator (including combination of immunomodulators) are administered directly into the tumor. In some embodiments, the adenovirus and/or immunomodulator (including combination of immunomodulators) are administered to the tissue having the tumor. In some embodiments, both the adenovirus and the immunomodulator (including combination of immunomodulators) are administered directly into the tumor. In some embodiments, both the adenovirus and the immunomodulator (including combination of immunomodulators) are administered to the tissue having the tumor. In some embodiments, the adenovirus is administered weekly. In some embodiments, the immunomodulator (including combination of immunomodulators) is administered weekly. In some embodiments, the method further comprises administration of the adenovirus and/or the immunomodulator (including combination of immunomodulators) by an administration route other than local administration.

In some embodiments, there is provided in combination, an infectious agent and immunomodulator for use in a method of treating a solid or lymphatic tumor in an individual, comprising: a) locally administering to the site of the tumor an effective amount of CG0070; and b) locally administering to the site of the tumor an effective amount of an immunomodulator (including combination of immunomodulators), wherein the immunomodulator is an inhibitor of PD-L1. In some embodiments, the immunomodulator is an immune checkpoint inhibitor. In some embodiments, the CG0070 and/or the immunomodulator (including combination of immunomodulators) are administered directly into the tumor. In some embodiments, the CG0070 and/or immunomodulator (including combination of immunomodulators) are administered to the tissue having the tumor. In some embodiments, both the CG0070 and the immunomodulator (including combination of immunomodulators) are administered directly into the tumor. In some embodiments, both the CG0070 and the immunomodulator (including combination of immunomodulators) are administered to the tissue having the tumor. In some embodiments, the CG0070 is administered weekly. In some embodiments, the immunomodulator (including combination of immunomodulators) is administered weekly. In some embodiments, the method further comprises administration of CG0070 and/or the immunomodulator (including combination of immunomodulators) by an administration route other than local administration.

In some embodiments, the infectious agent and the immunomodulator (including combination of immunomodulators) discussed above are administered sequentially, i.e., the administration of the infectious agent is administered before or after the administration of the immunomodulator (including combination of immunomodulators). In some embodiments, the infectious agent is administered prior to the administration of the immunomodulator (including combination of immunomodulators). In some embodiments, the infectious agent is administered no more than about any of 15 minutes, 30 minutes, 1 hour, 2 hours, 3 hours, 4 hours, 5 hours, 6 hours, 12 hours, or 24 hours prior to the administration of the immunomodulator (including combination of immunomodulators). In some embodiments, the infectious agent is administered about days or weeks (such as about any of 1 day, 2 days, 3 days, 4 days, 5 days, 6 days, 1 week, 2 weeks, 3 weeks, 4 weeks, or more) prior to the administration of the immunomodulator (including combination of immunomodulators). In some embodiments, the infectious agent is administered after the administration of the immunomodulator (including combination of immunomodulators). In some embodiments, the infectious agent is administered no more than about any of 15 minutes, 30 minutes, 1 hour, 2 hours, 3 hours, 4 hours, 5 hours, 6 hours, 12 hours, or 24 hours after the administration of the immunomodulator (including combination of immunomodulators). In some embodiments, the infectious agent is administered about days or weeks (such as about any of 1 day, 2 days, 3 days, 4 days, 5 days, 6 days, 1 week, 2 weeks, 3 weeks, 4 weeks, or more) after the administration of the immunomodulator (including combination of immunomodulators). In some embodiments, the infectious agent and the immunomodulator (including combination of immunomodulators) are administered with one immediately after another (e.g., within 5 minutes or less between the two administrations). For example, in some embodiments, the infectious agent is administered immediately before the administration of the immunomodulator (including combination of immunomodulators). In some embodiments, the infectious agent is administered immediately after the administration of the immunomodulator (including combination of immunomodulators).

In some embodiments, the infectious agent and the immunomodulator (including combination of immunomodulators) are administered simultaneously. In some embodiments, the infectious agent and the immunomodulator (including combination of immunomodulators) are administered simultaneously via separate compositions. In some embodiments, the infectious agent and the immunomodulator (including combination of immunomodulators) are administered as a single composition. In some embodiments, the infectious agent and the immunomodulator (including combination of immunomodulators) are mixed prior to (such as immediately prior to, e.g., within less than about 10, 5, or 1 minutes before) the administration of the composition. In some embodiments, the composition comprising the infectious agent and the immunomodulator (including combination of immunomodulators) is pre-made and stored for at least about 1 hours, 2 hours, 3 hours, 4 hours, 5 hours, 6 hours, 12 hours, 24 hours, 2 days, 3 days, 4 days, 5 days, 6 days, 7 days, 2 weeks, 3 weeks, or more prior to the administration.

The immunomodulators discussed herein include immune checkpoint inhibitors. The immunomodulator can be of any one of the molecular modalities known in the art, including, but not limited to, aptamer, mRNA, siRNA, microRNA, shRNA, peptide, antibody, anticalin, Spherical nucleic acid, TALEN, Zinc Finger Nuclease, CRISPR/Cas9, and small molecule.

In some embodiments, the immunomodulator is an immune checkpoint inhibitor. In some embodiments, the immune-checkpoint inhibitor is a natural or engineered ligand of an inhibitory immune checkpoint molecule, including, for example, ligands of CTLA-4 (e.g., B7.1, B7.2), and ligands of PD-L1. In some embodiments, the immune checkpoint inhibitor is an antibody that targets an inhibitory immune checkpoint protein. In some embodiments, the immunomodulator is an antibody selected from the group consisting of anti-CTLA-4 (e.g., Ipilimumab, Tremelimumab, KAHR-102), and anti-PD-L1 (e.g., KY-1003 (EP20120194977)In some embodiments, the antibody is an antagonistic antibody. In some embodiments, the antibody is a monoclonal antibody. In some embodiments, the antibody is a monoclonal antibody. In some embodiments, the antibody is an antigen-binding fragment selected from the group consisting of Fab, Fab', $F(ab')_2$, Fv, scFv, and other antigen-binding subsequences of the full length antibody. In some embodiments, the antibody is a human, humanized, or chimeric antibody. In some embodiments, the antibody is a bispecific antibody, a multispecific antibody, a single domain antibody, a fusion protein comprising an antibody portion, or any other functional variants or derivatives thereof.

In some embodiments, the method comprises local administration of a single immunomodulator. In some embodiments, the immunomodulator is an immune checkpoint inhibitor.

In some embodiments, the method comprises local administration of at least two (such as any of 2, 3, 4, 5, 6, or more) immunomodulators. In some embodiments, all or part of the at least two immunomodulators are administered simultaneously, such as in a single composition. In some embodiments, all or part of the at least two immunomodulators are administered sequentially. In some embodiments, the method comprises local administration of a combination of immunomodulators comprising an immune checkpoint inhibitor and an immune-stimulating agent. In some embodiments, the method comprises local administration of a combination of immunomodulators comprising two or more (such as any of 2, 3, 4, 5, 6, or more) checkpoint inhibitors. In some embodiments, the method comprises local administration of a combination of immunomodulators comprising two or more (such as any of 2, 3, 4, 5, 6, or more) immune-stimulating agents. In some embodiments, the method comprises local administration of a combination of immunomodulators comprising any number (such as any of 1, 2, 3, 4, 5, 6, or more) of immune checkpoint inhibitors and any number (such as any of 2, 3, 4, 5, 6, or more) of immune-stimulating agents. A method may comprise: a) locally administering to the site of the tumor an effective amount of an infectious agent (such as a virus, for example an oncolytic virus); and b) locally administering to the individual an effective amount of a first immunomodulator (such as an immune checkpoint inhibitor); and c) locally administering to the site of the tumor an effective amount of a second immunomodulator (such as an immune-stimulating agent). A method may comprise administration of a CTLA-4 inhibitor (such as an anti-CTLA-4 antibody, for example Ipilimumab, or an engineered lipocalin protein, for example an anticalin that specifically recognizes CTLA-4) and a CD40 agonist (such as an agnostic anti-CD40 antibody, for example, APX005M). A method may comprise administration of a CTLA-4 inhibitor (such as an anti-CTLA-4 antibody, for example Ipilimumab, or an engineered lipocalin protein, for example an anticalin that specifically recognizes CTLA-4) and a 4-1BB agonist (such as an agonistic anti-4-1BB antibody, e.g., PF-05082566). In some embodiments, the method comprises administration of a CTLA-4 inhibitor (such as an anti-CTLA-4 antibody) and a PD-L1 inhibitor (such as an anti-PD-L1 antibody).

In some embodiments, an additional immune checkpoint inhibitor is an inhibitor of CTLA-4. In some embodiments, the inhibitor of CTLA-4 is an anti-CTLA-4 antibody. Any of the anti-CTLA-4 antibodies that are known in the art may be used in the present invention, including, but not limited to, Ipilimumab, Tremelimumab, and KAHR-102. In some embodiments, the anti-CTLA-4 antibody is YERVOY® (Ipilimumab). In some embodiments, the anti-CTLA-4 antibody is a monoclonal antibody or a polyclonal antibody. In some embodiments, the anti-CTLA-4 antibody is an antigen-binding fragment selected from the group consisting of Fab, Fab', $F(ab')_2$, Fv, scFv, and other antigen-binding subsequences of the full length anti-CTLA-4 antibody. In some embodiments, the anti-CTLA-4 antibody is a human, humanized, or chimeric antibody. In some embodiments, the anti-CTLA-4 antibody is a bispecific antibody, a multispecific antibody, a single domain antibody, a fusion protein comprising an antibody portion, or any other functional variants or derivatives thereof. In some embodiments, the inhibitor of CTLA-4 is an engineered lipocalin protein specifically recognizing CTLA-4 (such as an anticalin molecule that specifically binds to CTLA-4). In some embodiments, the inhibitor of CTLA-4 is a natural or engineered ligand of CTLA-4, such as B7.1 or B7.2.

In some embodiments, the immune checkpoint inhibitor is an inhibitor of PD-1. In some embodiments, the inhibitor of PD-1 is an anti-PD-1 antibody. Any of the anti-PD-1 antibodies known in the art may be used in the present invention, including, but not limited to, Nivolumab, pembrolizumab, pidilizumab, BMS-936559, and atezolizumab, Lambrolizumab, MK-3475, AMP-224, AMP-514, STI-A1110, and TSR-042. In some embodiments, the anti-PD-1 antibody is a monoclonal antibody or a polyclonal antibody. In some embodiments, the anti-PD-1 antibody is an antigen-binding fragment selected from the group consisting of Fab, Fab', $F(ab')_2$, Fv, scFv, and other antigen-binding subsequences of the full-length anti-PD-1 antibody. In some embodiments, the anti-PD-1 antibody is a human, humanized, or chimeric antibody. In some embodiments, the anti-PD-1 antibody is a bispecific antibody, a multispecific antibody, a single domain antibody, a fusion protein comprising an antibody portion, or any other variants or derivatives thereof. In some embodiments, the inhibitor of PD-1 is a natural or engineered ligand of PD-1, such as PD-L1 or PD-L2. In some embodiments, the inhibitor of PD-1 is an inhibitor of the interaction between PD-1 and its ligand, for example, an inhibitor of PD-1/PD-L1 interaction or an inhibitor of PD-1/PD-L2 interaction. In the invention, the inhibitor of PD-1 is an inhibitor of PD-L1 (e.g., anti-PD-L1 antibody). Any of the inhibitors of interaction between PD-1 and its ligand may be used in the present invention, see, for example, U.S. Patent No. US7709214, US7432059, US7722868, US8217149, US8383796, and US9102725. In some embodiments, the inhibitor of PD-1 is an Fc fusion protein comprising a PD-1 ligand, such as an Fc-fusion of PD-L2 (e.g., AMP-224).

Thus, for example, in some embodiments, there is provided in combination, an infectious agent and immunomodulator for use in a method of treating a solid or lymphatic tumor (such as inhibiting tumor metastasis) in an individual (such as a human), comprising: a) locally administering to the site of the tumor an effective amount of an infectious agent wherein the infectious agent is an oncolytic virus; and b) locally administering to the site of the tumor an effective amount of an inhibitor of PD-L1. In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the inhibitor of PD-1 is an inhibitor of the interaction between PD-1 and its ligand, such as an inhibitor of PD-1/PD-L1 interaction. In some embodiments, the method further comprises local administration of a second immunomodulator, such as an immune-stimulating agent (e.g., a CD40 activator or a 4-1BB activator). In some embodiments, the infectious agent and/or the inhibitor of PD-L1 are administered directly into the tumor. In some embodiments, the infectious agent and/or the inhibitor of PD-L1 are administered to the tissue having the tumor. In some embodiments, both the infectious agent and the inhibitor of PD-L1 are administered directly into the tumor. In some embodiments, both the infectious agent and the inhibitor of PD-L1 are administered to the tissue having the tumor. In some embodiments, the infectious agent is administered weekly. In some embodiments, the inhibitor of PD-L1 is administered weekly. In some embodiments, the infectious agent and the inhibitor of PD-L1 are administered sequentially. In some embodiments, the infectious agent is administered prior to (such as immediately prior to) the administration of the inhibitor of PD-L1. In some embodiments, the infectious agent is administered after (such as immediately after) the administration of the inhibitor of PD-L1. In some embodiments, the infectious agent and the inhibitor of PD-L1 are administered simultaneously (for example in a single composition).

For example, in some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an oncolytic virus (such as oncolytic adenovirus); and b) locally administering to the site of the tumor an effective amount of an inhibitor of PD-L1.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus; and b) locally administering to the site of the tumor an effective amount of an inhibitor of PD-L1. In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus and a nucleic acid encoding an immune-related molecule (such as cytokine or chemokine) operably linked to a viral promoter; and b) locally administering to the site of the tumor an effective amount of an inhibitor of PD-L1. In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the immune-related molecule is GM-CSF.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an adenovirus serotype 5, wherein the endogenous E1a promoter and E3 19kD coding region of a native adenovirus is replaced by the human E2F-1 promoter and a nucleic acid encoding an immune-related molecule (such as cytokine or chemokine, for example, GM-CSF); and b) locally administering to the site of the tumor an effective amount of an inhibitor of PD-L1. In some embodiments, the tumor-specific promoter is a human E2F-1 promoter or an E2F-1 promoter comprising the nucleotide sequence set forth in SEQ ID NO: 1.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of CG0070; and b) locally administering to the site of the tumor an effective amount of an inhibitor of PD-L1. In some embodiments, the inhibitor of PD-L1 is an inhibitor of the interaction between PD-1 and its ligand, such as an inhibitor of PD-1/PD-L1 interaction. In some embodiments, the CG0070 and/or the inhibitor of PD-L1 are administered directly into the tumor. In some embodiments, the oncolytic virus and/or the inhibitor of PD-L1 are administered to the tissue having the tumor. In some embodiments, both the CG0070 and the inhibitor of PD-L1 are administered directly into the tumor. In some embodiments, both the CG0070 and the inhibitor of PD-L1 are administered to the tissue having the tumor. In some embodiments, the CG007 is administered weekly. In some embodiments, the inhibitor of PD-L1 is administered weekly. In some embodiments, the CG0070 and the inhibitor of PD-L1 are administered sequentially. In some embodiments, the CG0070 is administered prior to (such as immediately prior to) the administration of the inhibitor of PD-L1. In some embodiments, the CG0070 is administered after (such as immediately after) the administration of the inhibitor of PD-L1. In some embodiments, the CG0070 and the inhibitor of PD-L1 are administered simultaneously (for example in a single composition). In some embodiments, the method further comprises administration of the CG0070 and/or the inhibitor of PD-L1 by an administration route other than local administration.

The immune checkpoint inhibitor is an inhibitor of PD-1 ligand PD-L1. In some embodiments, the inhibitor of PD-1 ligand is an anti-PD-L1 antibody. Exemplary anti-PD-L1 antibodies include, but are not limited to, KY-1003, MCLA-145, RG7446 (also known as atezolizumab), BMS935559 (also known as MDX-1105), MPDL3280A, MEDI4736, Avelumab (also known as MSB0010718C), and STI-A1010. In some embodiments, the anti-PD-L1 is a monoclonal antibody or a polyclonal antibody. In some embodiments, the anti-PD-L1 is an antigen-binding fragment selected from the group consisting of Fab, Fab', $F(ab')_2$, Fv, scFv, and other antigen-binding subsequences of the full-length anti-PD-L1 antibody. In some embodiments, the anti-PD-L1 antibody is a human, humanized, or chimeric antibody. In some embodiments, the anti-PD-L1 antibody is a bispecific antibody, a multispecific antibody, a single domain antibody, a fusion protein comprising an antibody portion, or any other variants or derivatives thereof. In some embodiments, the inhibitor of PD-1 ligand is an inhibitor (e.g., peptide, protein or small molecule) of both PD-L1 and PD-L2. Exemplary inhibitors of both PD-L1 and PD-L2 include, but are not limited to, AUR-012, and AMP-224. In some embodiments, the inhibitor of PD-L1 and the inhibitor of PD-L2 can be used interchangeably in any of the methods of treatment described herein.

Thus, for example, in some embodiments, there is provided in combination, an infectious agent and immunomodulator for use in a method of treating a solid or lymphatic tumor (such as inhibiting tumor metastasis) in an individual (such as a human), comprising: a) locally administering to the site of the tumor an effective amount of an infectious agent wherein the infectious agent is an oncolytic virus; and b) locally administering to the site of the tumor an effective amount of an inhibitor of the PD-1 ligand PD-L1(such as an anti-PD-L1 antibody, or an inhibitor of both PD-L1 and PD-L2). In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the inhibitor of PD-1 ligand is an anti-PD-L1 antibody, for example, KY-1003, MCLA-145, RG7446, BMS935559, MPDL3280A, MEDI4736, Avelumab, or STI-A1010. In some embodiments, the inhibitor of PD-1 ligand is an inhibitor (e.g., peptide, protein or small molecule) of both PD-L1 and PD-L2, such as AUR-012, and AMP-224. In some embodiments, the method further comprises local administration of a second immunomodulator, such as an immune-stimulating agent (e.g., a CD40 activator or a 4-1BB activator). In some embodiments, the infectious agent and/or the inhibitor of PD-1 ligand are administered directly into the tumor. In some embodiments, the infectious agent and/or the inhibitor of PD-1 ligand are administered to the tissue having the tumor. In some embodiments, both the infectious agent and the inhibitor of PD-1 ligand are administered directly into the tumor. In some embodiments, both the infectious agent and the inhibitor of PD-1 ligand are administered to the tissue having the tumor. In some embodiments, the infectious agent is administered weekly. In some embodiments, the inhibitor of PD-1 ligand is administered weekly. In some embodiments, the infectious agent and the inhibitor of PD-1 ligand are administered sequentially. In some embodiments, the infectious agent is administered prior to (such as immediately prior to) the administration of the inhibitor of PD-1 ligand. In some embodiments, the infectious agent is administered after (such as immediately after) the administration of the inhibitor of PD-1 ligand. In some embodiments, the infectious agent and the inhibitor of PD-1 ligand are administered simultaneously (for example in a single composition). In some embodiments, the method further comprises administration of the infectious agent and/or the inhibitor of PD-1 ligand by an administration route other than local administration.

For example, in some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an oncolytic virus (such as oncolytic adenovirus); and b) locally administering to the site of the tumor an effective amount of an inhibitor of PD-1 ligand PD-L1 (such as an anti-PD-L1 antibody, or an inhibitor of both PD-L1 and PD-L2).

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus; and b) locally administering to the site of the tumor an effective amount of an inhibitor of PD-1 ligand PD-L1 (such as an anti-PD-L1 antibody, or an inhibitor of both PD-L1 and PD-L2). In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus and a nucleic acid encoding an immune-related molecule (such as cytokine or chemokine) operably linked to a viral promoter; and b) locally administering to the site of the tumor an effective amount of an inhibitor of PD-1 ligand PD-L1 (such as an anti-PD-L1 antibody, or an inhibitor of both PD-L1 and PD-L2). In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the immune-related molecule is GM-CSF.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of an adenovirus serotype 5, wherein the endogenous E1a promoter and E3 19kD coding region of a native adenovirus is replaced by the human E2F-1 promoter and a nucleic acid encoding an immune-related molecule (such as cytokine or chemokine, for example, GM-CSF); and b) locally administering to the site of the tumor an effective amount of an inhibitor of PD-1 ligand PD-L1 (such as an anti-PD-L1 antibody, or an inhibitor of both PD-L1 and PD-L2). In some embodiments, the tumor-specific promoter is a human E2F-1 promoter or an E2F-1 promoter comprising the nucleotide sequence set forth in SEQ ID NO: 1.

In some embodiments, there is provided in combination, an infectious agent and immunomodulator for use in a method of treating a solid or lymphatic tumor (such as inhibiting tumor metastasis) in an individual, comprising: a) locally administering to the site of the tumor an effective amount of CG0070; and b) locally administering to the site of the tumor an effective amount of an inhibitor of PD-1 ligand PD-L1 (such as an anti-PD-L1 antibody, or an inhibitor of both PD-L1 and PD-L2). In some embodiments, the inhibitor of PD-1 ligand PD-L1 is an anti-PD-L1 antibody, for example, KY-1003, MCLA-145, RG7446, BMS935559, MPDL3280A, MEDI4736, Avelumab, or STI-A1010. In some embodiments, the inhibitor of PD-1 ligand PD-L1 is an inhibitor (e.g., peptide, protein or small molecule) of both PD-L1 and PD-L2, such as AUR-012, and AMP-224. In some embodiments, the CG0070 and/or the inhibitor of PD-1 ligand are administered directly into the tumor. In some embodiments, the oncolytic virus and/or the inhibitor of PD-1 ligand are administered to the tissue having the tumor. In some embodiments, both the CG0070 and the inhibitor of PD-1 ligand are administered directly into the tumor. In some embodiments, both the CG0070 and the inhibitor of PD-1 ligand are administered to the tissue having the tumor. In some embodiments, the CG007 is administered weekly. In some embodiments, the inhibitor of PD-1 ligand is administered weekly. In some embodiments, the CG0070 and the inhibitor of PD-1 ligand are administered sequentially. In some embodiments, the CG0070 is administered prior to (such as immediately prior to) the administration of the inhibitor of PD-1 ligand. In some embodiments, the CG0070 is administered after (such as immediately after) the administration of the inhibitor of PD-1 ligand. In some embodiments, the CG0070 and the inhibitor of PD-1 ligand are administered simultaneously (for example in a single composition). In some embodiments, the method further comprises administration of CG0070 and/or the inhibitor of PD-1 ligand by an administration route other than local administration.

The methods described herein may further comprise a step of locally administering to the site of the tumor a pretreatment composition prior to the administration of the infectious agent. In some embodiments, the pretreatment composition comprises a transduction enhancing agent, such as N-Dodecyl-β-D-maltoside (DDM). DDM is a nonionic surfactant comprised of a maltose derivatized with a single twelve-carbon chain, and acts as a mild detergent and solubilizing agent. It has been used as a food additive and is known to enhance mucosal surface permeation in rodents, probably due to its effect on membrane associated GAG and tight junctions.

The pretreatment composition can be administered directly into the tumor or to a tissue having the tumor. In some embodiments, the pretreatment composition comprises a solution of the transduction enhancing agent (such as DDM). Suitable concentration of the pretreatment composition (such as DDM solution) include, but are not limited to, about any one of 0.01%, 0.05%, 0.1%, 0.2%, 0.3%, 0.4%, 0.5%, 1%, 2%, 3%, 4%, or 5% of the transducing enchanting agent (such as DDM). In some embodiments, the pretreatment composition comprises any of about 0.01% to about 0.05%, about 0.05% to about 0.1%, about 0.1% to about 0.5%, about 0.5% to about 1%, about 1% to about 2%, about 2% to about 3%, about 3% to about 4%, about 4% to about 5%, about 0.01% to about 1%, about 0.05% to about 2%, about 1% to about 5%, or about 0.1% to about 5% of the transduction enhancing agent (such as DDM).

In some embodiments, the pretreatment (such as DDM) is administered immediately (such as no more than 5 minutes) prior to the administration of the infectious agent. In some embodiments, the pretreatment (such as DDM) is administered no more than about any of 5 minutes, 10 minutes, 15 minutes, 20 minutes, 30 minutes, 45 minutes, 1 hour, 90 minutes, 2 hours, 3 hours or 4 hours before the administration of the infectious agent. In some embodiments, the pretreatment (such as DDM) is administered no more than about 2 hours before the administration of the infectious agent.

Suitable dosages for the pretreatment composition (such as DDM) include, but are not limited to, about any of 0.1 mg/kg, 0.5 mg/kg, 1 mg/kg, 1.5mg/kg, 2 mg/kg, 2.5 mg/kg, 5mg/kg, 10 mg/kg, 25 mg/kg, 50 mg/kg, 100 mg/kg, 150 mg/kg, 200 mg/kg, 250 mg/kg, 300 mg/kg, 400 mg/kg, 500 mg/kg, 0.1 mg/kg to 0.5 mg/kg, 0.5 mg/kg to 1 mg/kg, 1 mg/kg to 2 mg/kg, 2 mg/kg to 5mg/kg, 5mg/kg to 10 mg/kg, 10 mg/kg to 25 mg/kg, 25 mg/kg to 50 mg/kg, 50 mg/kg to 100 mg/kg, 100 mg/kg to 150 mg/kg, 150 mg/kg to 200 mg/kg, 200 mg/kg to 250 mg/kg, 250 mg/kg to 500 mg/kg, or 0.5 mg/kg to about 5 mg/kg. In some embodiments, a suitable dosage for the pretreatment composition is about any one of 0.1 g, 0.2 g, 0.5g, 0.75 g, 1 g, 1.5 g, 2 g, 2.5 g, 5 g, or 10 g of the transduction enhancing agent (such as DDM).

In some embodiments, the individual (e.g., wholly or only at the site of the tumor) is subject to a prior therapy prior to the administration of the infectious agent and the immunomodulator (including combination of immunomodulators). In some embodiments, the prior therapy is tumor site preparation using one or more (such as 1, 2, 3, 4, 5, or more) treatment modalities, including, but are not limited to radiation therapy, administration of one or more immune-related molecules, administration of other therapeutic agents, and combination thereof. It is believed that adding other pre-treatment preparations can increase the chance of success for the methods described above. Without being bound by any theory or hypothesis, for example, local radiation, with or without lymphodepletion effects, or chemotherapy, may increase the chance of the infectious process, and may deplete the more sensitive Treg at the tumor sites, thereby reviving the exhausted or telorized T memory cells. Similarly, tumor site preparations prior to or in concomitant with the administration of the invention combination "at" tumor site can involve cytokines, chemokines, small molecules and other well-known beneficial immunomodulators, such as IL2, IL12, OX40, CD40 and 4-1BB agonist. These tumor site preparation modalities can be given in conjunction with or in sequence depending on needs.

In some embodiments, the prior therapy is radiation therapy (e.g., with or without chemotherapy). In some embodiments, the radiation therapy is in combination with chemotherapy. In some embodiments, the prior therapy is radiation therapy to the whole body. In some embodiments, the prior therapy is radiation therapy to only tumor sites. In some embodiments, the prior therapy is radiation therapy to tissues having the tumor. In some embodiments, the prior therapy is radiation therapy to only the site of the tumor selected for local administration of the infectious agent and the immunomodulator. In some embodiments, the prior therapy is radiation therapy to only a tissue having the tumor selected for local administration of the infectious agent and the immunomodulator. In some embodiments, the dose of the radiation therapy is insufficient to eradicate the tumor cells. For example, a suitable dosage of the radiation therapy is about any one of 1 Gy, 5 Gy, 10 Gy, 15 Gy, 20 Gy, 25 Gy, 30 Gy, 35 Gy, 40 Gy, 45 Gy, 50 Gy, 55 Gy, 60 Gy, 65 Gy, 70 Gy, 75 Gy, 80 Gy, 90 Gy or 100 Gy. In some embodiments, the dose of the radiation therapy is no more than about any one of 1 Gy, 5 Gy, 10 Gy, 15 Gy, 20 Gy, 25 Gy, 30 Gy, 35 Gy, 40 Gy, 45 Gy, 50 Gy, 55 Gy, 60 Gy, 65 Gy, 70 Gy, 75 Gy, 80 Gy, 90 Gy or 100 Gy. In some embodiments, the dose of the radiation therapy is any one of about 1 Gy to about 5 Gy, about 5 Gy to about 10 Gy, about 10 Gy to about 15 Gy, about 15 Gy to about 20 Gy, about 20 Gy to about 25 Gy, about 25 Gy to about 30 Gy, about 30 Gy to about 35 Gy, about 5 Gy to about 15 Gy, about 10 Gy to about 20 Gy, about 20 Gy to about 30 Gy, about 30 Gy to about 40 Gy, about 40 Gy to about 50 Gy, about 50 Gy to about 60 Gy, about 60 Gy to about 70 Gy, about 70 Gy to about 80 Gy, about 80 Gy to about 100 Gy, about 10 Gy to about 30 Gy, about 20 Gy to about 40 Gy, about 1Gy to about 25 Gy, about 25 Gy to about 50 Gy, about 30 Gy to about 60 Gy, about 60 Gy to about 80 Gy, or about 10 Gy to about 60 Gy. The suitable dosage of the radiation therapy may also depend on the type, stage and location of the tumor.

In some embodiments, the radiation therapy is administered in more than one fraction, such as about any one of 2, 3, 4, 5, 6, 7, 8, 9, 10, 12, 15, 16, 18, 20 or more fractions. In some embodiments, the radiation therapy fractions are administered over the course of about any one of 1 day, 2 days, 3 days, 4 days, 5 days, 6 days, 1 week, 2 weeks, 3 weeks, 4 weeks, 5 weeks, 6 weeks, 7 weeks or more. In some embodiments, the radiation therapy fractions are administered over the course of any one of about 1 day to about 5 days, about 1 week to about 2 weeks, about 2 weeks to about 3 weeks, about 3 weeks to about 4 weeks, about 4 weeks to about 5 weeks, about 5 weeks to about 6 weeks, about 6 weeks to about 7 weeks, about 2 weeks to about 4 weeks, about 4 weeks to about 6 weeks, or about 1 week to about 6 weeks. In some embodiments, the radiation therapy is administered about two fractions per day. In some embodiments, each fraction of the radiation therapy is about 1.8 Gy to about 2 Gy per day, five days a week, for an adult, or about 1.5 Gy to about 1.8 Gy per day, five days a week for a child. In some embodiments, each fraction of the radiation therapy is about any one of 1 Gy, 1.5 Gy, 2 Gy, 2.5 Gy, 5 Gy, 10 Gy, 15 Gy, 20 Gy, 30 Gy, 40 Gy, 50 Gy or more. In some embodiments, each fraction of the radiation therapy is any one of about 1 Gy to about 1.5 Gy, about 1.5 Gy to about 2 Gy, about 1 Gy to about 2.5 Gy, about 2.5 Gy to about 5 Gy, about 5 Gy to about 10 Gy, about 10 Gy to about 15 Gy, about 15 Gy to about 20 Gy, about 20 Gy to about 30 Gy, about 25 Gy to about 50 Gy, about 1 Gy to about 10 Gy, or about 2 Gy to about 20 Gy. In some embodiments, the radiation therapy is administered in a single fraction.

In some embodiments, the radiation therapy is aim at lymphodepletion, either as a single dose fraction per day or in multiple fractions over days to weeks. In some embodiments, the lymphodepletion radiation therapy is given as a total body irradiation. In some embodiments, the lymphodepletion is only given to local tumor sites, or to tissues with the tumor. In some embodiments, the lymphodepletion radiation therapy is administered two fractions per day. In some embodiments, each fraction of the lymphodepletion radiation therapy is about 1 Gy to about 2 Gy per day, five days a week, for an adult, or about 0.5 Gy to about 1.8 Gy per day, five days a week for a child. In some embodiments, each fraction of the radiation therapy is about any one of 1 Gy, 1.5 Gy, 2 Gy, 2.5 Gy, 5 Gy, 10 Gy, 15 Gy, 20 Gy, 30 Gy, 40 Gy, 50 Gy or more. In some embodiments, each fraction of the radiation therapy is any one of about 1 Gy to about 1.5 Gy, about 1.5 Gy to about 2 Gy, about 1 Gy to about 2.5 Gy, about 2.5 Gy to about 5 Gy, about 5 Gy to about 10 Gy, about 10 Gy to about 15 Gy, about 15 Gy to about 20 Gy, about 20 Gy to about 30 Gy, about 25 Gy to about 50 Gy, about 1 Gy to about 10 Gy, or about 2 Gy to about 20 Gy. In some embodiments, lymphodepletion radiation therapy is administered with or without the use of a chemotherapeutic agent, such as but not limited to, cyclophosphamide and fludarabine.

Any of the known methods of radiation therapy may be used in the present invention, including, but not limited to external beam radiation therapy (EBRT or XRT), tele therapy, brachytherapy, sealed source radiation therapy, systemic radioisotope therapy (RIT), unsealed source radiation therapy, intraoperative radiation therapy (IORT), targeted intraoperative radiation therapy (TARGIT), intensity-modulated radiation therapy (IMRT), volumetric modulated arc therapy (VMAT), particle therapy, and auger therapy.

In some embodiments, the prior therapy comprises administration of a therapeutic agent. In some embodiments, the dosage of the therapeutic agent is sufficient to eradicate the tumor cells. In some embodiments, the dosage of the therapeutic agent is insufficient to eradicate the tumor cells. In some embodiments, the therapeutic agent is any one or combination of chemotherapeutic agents known in the art, for example, cyclosphamide. In some embodiments, the therapeutic agent is any one or combination of agents targeting or blocking a cellular signaling pathway known in the art, for example, a BRAF inhibitor. In some embodiments, the therapeutic agent is any one or combination of cell therapies known in the art, for example, TIL cells, CAR/T cells, and/or TCR/T cells. In some embodiments, the therapeutic agent is an agent that increases the level of cytokines involved an immunogenic pathway. Any of the immune-related molecules described herein may be used as the therapeutic agent, including, but are not limited to, cytokines such as IL6, IL8 and IL18 (these cytokines can either have pro and/or antiinflammatory actions, or some may promote new blood vessels formation and tumor growth), chemokines (such as CCL21 that can promote tumor spread by increase of lymphatic structures), growth factors (such as FLT3L), heat shock proteins, small molecule kinase inhibitors (such as JAK2 inhibitor), IAP inhibitors, STING activators (such as CDN), PRRago (such as CpG ODN (oligodeoxynucleotides), Imiquimod, or Poly I:C), TLR stimulators (such as GS-9620, AED-1419, CYT-003-QbG10, AVE-0675, or PF-7909), and RLR stimulators (such as RIG-I, Mda5, or LGP2 stimulators). In some embodiments, the therapeutic agent is an agent that causes dysfunction or damage to a structural component of a tumor. Exemplary agents include, but are not limited to, anti-VEGF antibody, a hyaluronidase, and n-dodecyl-β-maltoside. In some embodiments, the therapeutic agent induces immune cells, such as dendritic cells, B cells, and T cells (such as follicular T helper cells).

Any of the therapeutic agent/s described herein, e.g. chemotherapeutic agents, agents targeting or blocking cell signaling pathways, cytokines, chemokines, cell therapies, etc., can be administered directly or indirectly (e.g. through intravenous administration) to the tumor sites, either singly or in combination.

Suitable dosages for the infectious agent depend on factors such as the nature of the infectious agent, type of the solid or lymphatic tumor being treated, and routes of administration. As used herein, "particles" as related to an infectious agent mean the collective number of physical singular units of the infectious agent (such as a virus or bacterium). This number can be converted to, or is equivalent to, another number meaning infectious titer units, e.g., plaque forming unit (pfu) or international unit, by infectivity assays as known in the art. In some embodiments, the infectious agent is administered at a dose of about any one of $1\times10^5$ particles, $1\times10^6$ particles, $1\times10^7$ particles, $1\times10^8$ particles, $1\times10^9$ particles, $1\times10^{10}$ particles, $2\times10^{10}$ particles, $5\times10^{10}$ particles, $1\times10^{11}$ particles, $2\times10^{11}$ particles, $5\times10^{11}$ particles, $1\times10^{12}$ particles, $2\times10^{12}$ particles, $5\times10^{12}$ particles, $1\times10^{13}$ particles, $2\times10^{13}$ particles, $5\times10^{13}$ particles, $1\times10^{14}$ particles, or $1\times10^{15}$ particles. In some embodiments, the infectious agent is administered at a dose of any one of about $1\times10^5$ particles to about $1\times10^6$ particles, about $1\times10^6$ particles to about $1\times10^7$ particles, about $1\times10^7$ particles to about $1\times10^8$ particles, about $1\times10^8$ particles to about $1\times10^9$ particles, about $1\times10^9$ particles to about $1\times10^{10}$ particles, about $1\times10^{10}$ particles to about $1\times10^{11}$ particles, about $1\times10^{11}$ particles to about $5\times10^{11}$ particles, about $5\times10^{11}$ particles to about $1\times10^{12}$ particles, about $1\times10^{12}$ particles to about $2\times10^{12}$ particles, about $2\times10^{12}$ particles to about $5\times10^{12}$ particles, about $5\times10^{12}$ particles to about $1\times10^{13}$ particles, about $1\times10^{13}$ particles to about $1\times10^{14}$ particles, or about $1\times10^{14}$ particles to about $1\times10^{15}$ particles.

In some embodiments, the infectious agent is administered daily. In some embodiments, the infectious agent is administered is administered at least about any one of 1x, 2x, 3x, 4x, 5x, 6x, or 7x (i.e., daily) a week. In some embodiments, the infectious agent is administered weekly. In some embodiments, the infectious agent is administered weekly without break; weekly, two out of three weeks; weekly three out of four weeks; once every two weeks; once every 3 weeks; once every 4 weeks; once every 6 weeks; once every 8 weeks, monthly, or every two to 12 months. In some embodiments, the intervals between each administration are less than about any one of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any one of 1 month, 2 months, 3 months, 4 months, 5 months, 6 months, 8 months, or 12 months. In some embodiments, there is no break in the dosing schedule. In some embodiments, the interval between each administration is no more than about a week.

The administration of the infectious agent can be over an extended period of time, such as from about a month up to about seven years. In some embodiments, the infectious agent is administered over a period of at least about any one of 1, 2, 3, 4, 5, 6, 7, 8, 9, 10, 11, 12, 18, 24, 30, 36, 48, 60, 72, or 84 months. In some embodiments, the infectious agent is administered over a period of at least 4 weeks or 6 weeks. In some embodiments, the infectious agent is administered weekly for four weeks every 3 months. In some embodiments, the infectious agent is administered weekly for 6 weeks every 3 months.

Suitable dosages for the immunomodulator (including combination of immunomodulators) depend on factors such as the nature of the immunomodulator or combination of immunomodulators, type of the solid or lymphatic tumor being treated, and the routes of administration. Exemplary doses of the immunomodulator (including combination of immunomodulators) include, but are not limited to, about any one of 1 mg/$m^2$, 5 mg/$m^2$, 10 mg/$m^2$, 20 mg/$m^2$, 50 mg/$m^2$, 100 mg/$m^2$, 200 mg/$m^2$, 300 mg/$m^2$, 400 mg/$m^2$, 500 mg/$m^2$, 750 mg/$m^2$, 1000 mg/$m^2$, or more. In some embodiments, the dose of the immunomodulator (including combination of immunomodulators) is included in any one of the following ranges: about 1 to about 5 mg/$m^2$, about 5 to about 10 mg/$m^2$, about 10 to about 20 mg/$m^2$, about 20 to about 50 mg/$m^2$, about 50 to about 100 mg/$m^2$, about 100 mg/$m^2$ to about 200 mg/$m^2$, about 200 to about 300 mg/$m^2$, about 300 to about 400 mg/$m^2$, about 400 to about 500 mg/$m^2$, about 500 to about 750 mg/$m^2$, or about 750 to about 1000 mg/$m^2$. In some embodiments, the dose of the immunomodulator is about any one of 1 μg/kg, 2 μg/kg, 5 μg/kg, 10 μg/kg, 20 μg/kg, 50 μg/kg, 0.1 mg/kg, 0.2 mg/kg, 0.3 mg/kg, 0.4 mg/kg, 0.5 mg/kg, 1 mg/kg, 2 mg/kg, 5 mg/kg, 10 mg/kg, 20 mg/kg, 50 mg/kg, 100 mg/kg, or more. In some embodiments, the dose of the immunomodulator (including combination of immunomodulators) is any one of about 1 μg/kg to about 5 μg/kg, about 5 μg/kg to about 10 μg/kg, about 10 μg/kg to about 50 μg/kg, about 50 μg/kg to about 0.1 mg/kg, about 0.1 mg/kg to about 0.2 mg/kg, about 0.2 mg/kg to about 0.3 mg/kg, about 0.3 mg/kg to about 0.4 mg/kg, about 0.4 mg/kg to about 0.5 mg/kg, about 0.5 mg/kg to about 1 mg/kg, about 1 mg/kg to about 5 mg/kg, about 5 mg/kg to about 10 mg/kg, about 10 mg/kg to about 20 mg/kg, about 20 mg/kg to about 50 mg/kg, about 50 mg/kg to about 100 mg/kg, or about 1 mg/kg to about 100 mg/kg. In some embodiments, the dose of the immunomodulatory (including combination of immunomodulators) is about any one of 1 μg, 10 μg, 50 μg, 100 μg, 500 μg, 1 mg, 2 mg, 4 mg, 6 mg, 12 mg, 18 mg, 24 mg, 50 mg, 100 mg, 500 mg or 1000 mg. In some embodiments, the dose of the immunomodulatory (including combination of immunomodulators) is any one of about 1 μg to about 10 μg, about 10 μg to about 50 10 μg, about 50 μg to about 100 μg, about 100 μg to about 500 μg, about 500 μg to about 1 mg, about 1 mg to about 5 mg, about 5 mg to about 10 mg, about 10 mg to about 25 mg, about 25 mg to about 50 mg, about 50 mg to about 100 mg, about 100 mg to about 500 mg, about 500 mg to about 1000 mg, about 1 μg to about 1 mg, about 1 mg to about 1000 mg, or about 1 μg to about 1000 mg. In some embodiments, the dose of the immunomodulator (including combination of immunomodulators) administered per tumor site is no more than about any of 10 μg, 50 μg, 100 μg, 500 μg, 1 mg, 2 mg, 4 mg, 6 mg, 12 mg, 18 mg, 24 mg, 50 mg, or 100 mg. In some embodiments, the dose of the immunomodulator (including combination of immunomodulators) administered per tumor site is any one of about 10 μg to about 50 μg, about 50 μg to about 100 μg, about 100 μg to about 500 μg, about 100 μg to about 1 mg, about 1 mg to about 2 mg, about 2 mg to about 5 mg, about 5 mg to about 10 mg, about 10 mg to about 15 mg, about 10 mg to about 25 mg, about 25 mg to about 50 mg, about 50 mg to about 100 mg, about 1 mg to about 50 mg, or about 100 μg to about 10 mg. In some embodiments, the dose of the immunomodulatory (including combination of immunomodulators) administered per tumor site is based on the size of the tumor.

In some embodiments, the immunomodulator (including combination of immunomodulators) is administered daily. In some embodiments, the immunomodulator (including combination of immunomodulators) is administered is administered at least about any one of 1x, 2x, 3x, 4x, 5x, 6x, or 7x (i.e., daily) a week. In some embodiments, the immunomodulator (including combination of immunomodulators) is administered weekly. In some embodiments, the immunomodulator (including combination of immunomodulators) is administered weekly without break; weekly, two out of three weeks; weekly three out of four weeks; once every two weeks; once every 3 weeks; once every 4 weeks; once every 6 weeks; once every 8 weeks, monthly, or every two to 12 months. In some embodiments, the intervals between each administration are less than about any one of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any one of 1 month, 2 months, 3 months, 4 months, 5 months, 6 months, 8 months, or 12 months. In some embodiments, there is no break in the dosing schedule. In some embodiments, the interval between each administration is no more than about a week. In some embodiments, the immunomodulator (including combination of immunomodulators) is administered with the same dosing schedule as the infectious agent. In some embodiments, the immunomodulator (including combination of immunomodulators) is administered with a different dosing schedule as the infectious agent. In some embodiments, the infectious agent is administered weekly for four weeks, and the immunomodulator (including combination of immunomodulators) is administered weekly for three out of four weeks.

The administration of the immunomodulator (including combination of immunomodulators) can be over an extended period of time, such as from about a month up to about seven years. In some embodiments, the immunomodulator (including combination of immunomodulators) is administered over a period of at least about any one of 1, 2, 3, 4, 5, 6, 7, 8, 9, 10, 11, 12, 18, 24, 30, 36, 48, 60, 72, or 84 months. In some embodiments, the immunomodulator (including combination of immunomodulators) is administered over a period of at least 3 weeks or 6 weeks. In some embodiments, the immunomodulator (including combination of immunomodulators) is administered weekly for three out of four weeks every 3 months. In some embodiments, the immunomodulator (including combination of immunomodulators) is administered weekly for 6 weeks every 3 months.

Exemplary routes of administration of the infectious agent, the immunomodulator (including combination of immunomodulators), the cytokine, and/or the pretreatment composition include, but are not limited to, intratumoral, intravesical, intramuscular, intraperitoneal, intravenous, intra-arterial, intracranial, intrapleural, subcutaneous, and epidermal routes, or be delivered into lymph glands, body spaces, organs or tissues known to contain such live cancer cells (such as intrahepatic or intrapancreatic injections). In some embodiments, the administration is carried out by direct injection of the agent(s) into the tumor. In some embodiments, the administration is carried out by direct injection of the agent(s) to a site close to the tumor cells. The specific route of the administration depends on the nature of the solid or lymphatic tumor and is discussed further below in the context of different types of solid or lymphatic tumor.

In some embodiments, wherein the infectious agent and/or the immunomodulator (including combination of immunomodulators) are administered intratumorally (e.g., intratumoral injection), the total volume administered is no more than about any one of 0.5 mL, 1 mL, 1.5 mL, 2 mL, 2.5 mL, 5 mL or 10 mL. In some embodiments, the volume of the infectious agent and/or the immunomodulator (including combination of immunomodulators) for intratumoral administration (such as intratumoral injection) per tumor site is dependent on the size of the tumor site. Tumor size can be measured as the tumor volume or the longest dimension of the tumor. For example, for a tumor with the longest dimension greater than about 5 cm, the intratumoral administration volume is no more than about 2 mL; for a tumor with the longest dimension of about 2 cm to about 5 cm, the intratumoral administration volume is about 1 mL; for a tumor with the longest dimension of about 0.75 cm to about 2 cm, the intratumoral administration volume is about 0.5 mL; and for a tumor with the longest dimension of smaller than about 0.75 cm, the intratumoral administration volume is about 0.1 mL. In some embodiments, the infectious agent and/or the immunomodulator (including combination of immunomodulators) are administered to all tumor sites. In some embodiments, the infectious agent and/or the immunomodulator (including combination of immunomodulators) are administered to about any one of 1, 2, 3, 4, 5, 6, or more tumor sites. In some embodiments, the infectious agent and/or the immunomodulator (including combination of immunomodulators) are administered to the tumor site with the largest size.

In some embodiments, the amount of the infectious agent in combination with the immunomodulator is effective to inhibit tumor metastasis in the individual. In some embodiments, at least about 10% (including for example at least about any of 20%, 30%, 40%, 60%, 70%, 80%, 90%, or 100%) metastasis is inhibited. In some embodiments, a method of inhibiting metastasis to lymph node is provided. In some embodiments, a method of inhibiting metastasis to the lung is provided. Metastasis can be assessed by any known methods in the art, such as by blood tests, bone scans, x-ray scans, CT scans, PET scans, and biopsy.

In some embodiments, the amount of the infectious agent in combination with the immunomodulator is effective to prolong survival (such as disease free survival) in the individual. In some embodiments, the survival is prolonged for at least about 2, 3, 4, 5, 6, 12, or 24 months. In some embodiments, there is provided a method of prolonging survival of an individual having a solid or lymphatic tumor, comprising: (a) locally administering to the site of the tumor an effective amount of an infectious agent (such as CG0070); and (b) locally administering to the site of the tumor an effective amount of an immunomodulator (including combination of immunomodulators).

In some embodiments, the amount of the infectious agent in combination with the immunomodulator is effective to cause disease remission (partial or complete) in the individual. In some embodiments, there is provided a method of causing disease remission (partial or complete) in an individual having a solid or lymphatic tumor, comprising: (a) locally administering to the site of the tumor an effective amount of an infectious agent (such as CG0070); and (b) locally administering to the site of the tumor an effective amount of an immunomodulator (including combination of immunomodulators).

In some embodiments, the amount of the infectious agent in combination with the immunomodulator is effective to improve quality of life in the individual. In some embodiments, there is provided a method of improving quality of life of an individual having a solid or lymphatic tumor, comprising: (a) locally administering to the site of the tumor an effective amount of an infectious agent (such as CG0070); and (b) locally administering to the site of the tumor an effective amount of an immunomodulator (including combination of immunomodulators).

In some embodiments, the amount of the infectious agent in combination with the immunomodulator is effective to inhibit growth or reducing the size of the solid or lymphatic tumor. In some embodiments, the size of the solid or lymphatic tumor is reduced for at least about 10% (including for example at least about any of 20%, 30%, 40%, 60%, 70%, 80%, 90%, or 100%). In some embodiments, there is provided a method of inhibiting growth or reducing the size of a solid or lymphatic tumor in an individual, comprising: (a) locally administering to the site of the tumor an effective amount of an infectious agent (such as CG0070); and (b) locally administering to the site of the tumor an effective amount of an immunomodulator (including combination of immunomodulators).

Solid or lymphatic tumors discussed herein include, but is not limited to, Hodgkin lymphoma, non-Hodgkin lymphoma, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine sacronomasynovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

In some embodiments, the solid or lymphatic tumor is selected from the group consisting of head and neck squamous cell cancer, breast cancer, colorectal cancer, pancreatic adenocarcinoma, ovarian cancer, non-small cell lung cancer, prostate cancer, and melanoma. The methods are applicable to solid or lymphatic tumors of all stages, including stages, I, II, III, and IV, according to the American Joint Committee on Cancer (AJCC) staging groups. In some embodiments, the solid or lymphatic tumor is an/a: early stage cancer, non-metastatic cancer, primary cancer, advanced cancer, locally advanced cancer, metastatic cancer, cancer in remission, cancer in an adjuvant setting, or cancer in a neoadjuvant setting. In some embodiments, the solid or lymphatic tumor is localized resectable, localized unresectable, or unresectable. In some embodiments, the solid or lymphatic tumor is localized resectable or borderline resectable. In some embodiments, the cancer has been refractory to prior therapy.

In some embodiments, the solid or lymphatic tumor is head and neck cancer. In some embodiments, the head and neck cancer is a squamous cell carcinoma in the head and neck. In some embodiments, the head and neck cancer is a hypopharyngeal cancer, laryngeal cancer, lip and oral cavity cancer, metastatic squamous neck cancer with occult primary, nasopharyngeal cancer, oropharyngeal cancer, paranasal sinus and nasal cavity cancer, or salivary gland cancer. In some embodiments, the head and neck squamous cell cancer is an early stage head and neck cancer, non-metastatic head and neck cancer, advanced head and neck cancer, locally advanced head and neck cancer, metastatic head and neck cancer, head and neck cancer in remission, head and neck cancer in adjuvant setting, or head and neck cancer in neoadjuvant setting. In some embodiments, the head and neck cancer is in a neoadjuvant setting. In some embodiments, the administration of the infectious agent, the immunomodulator (including combination of immunomodulators) and/or the pretreatment composition is carried out by injection into the head and neck tissue having the head and neck tumor. In some embodiments, the administration of the infectious agent, the immunomodulator (including combination of immunomodulators) and/or the pretreatment composition is carried out by injection directly into the head and neck tumor. In some embodiments, the administration of the infectious agent, the immunomodulator (including combination of immunomodulators) and/or the pretreatment composition is carried out by injection directly into metastatic sites of the head and neck tumor. In some embodiments, the administration of the infectious agent, the immunomodulator (including combination of immunomodulators) and/or the pretreatment composition is carried out by injection into the head and neck tissue close to the head and neck tumor.

In some embodiments, the solid or lymphatic tumor is breast cancer. In some embodiments, the breast cancer is early stage breast cancer, non-metastatic breast cancer, advanced breast cancer, stage IV breast cancer, locally advanced breast cancer, metastatic breast cancer, breast cancer in remission, breast cancer in an adjuvant setting, or breast cancer in a neoadjuvant setting. In some embodiments, the breast cancer is in a neoadjuvant setting. In some embodiments, the breast cancer is at an advanced stage. In some embodiments, the breast cancer (which may be HER2 positive or HER2 negative) includes, for example, advanced breast cancer, stage IV breast cancer, locally advanced breast cancer, and metastatic breast cancer. In some embodiments, the breast cancer is a triple negative breast cancer. In some embodiments, the administration of the infectious agent, the immunomodulator (including combination of immunomodulators) and/or the pretreatment composition is carried out by intramammary injection into the mammary tissue having the breast tumor. In some embodiments, the administration of the infectious agent, the immunomodulator (including combination of immunomodulators) and/or the pretreatment composition is carried out by intramammary injection directly into the breast tumor. In some embodiments, the administration of the infectious agent, the immunomodulator (including combination of immunomodulators) and/or the pretreatment composition is carried out by injection directly into metastatic sites of the breast tumor. In some embodiments, the administration of the infectious agent, the immunomodulator (including combination of immunomodulators) and/or the pretreatment composition is carried out by intramammary injection into the mammary tissue close to the breast tumor.

In some embodiments, the cancer is renal cell carcinoma. In some embodiments, the renal cell carcinoma is an adenocarcinoma. In some embodiments, the renal cell carcinoma is a clear cell renal cell carcinoma, papillary renal cell carcinoma (also called chromophilic renal cell carcinoma), chromophobe renal cell carcinoma, collecting duct renal cell carcinoma, granular renal cell carcinoma, mixed granular renal cell carcinoma, renal angiomyolipomas, or spindle renal cell carcinoma. In some embodiments, the renal cell carcinoma is at any of stage I, II, III, or IV, according to the American Joint Committee on Cancer (AJCC) staging groups. In some embodiments, the administration of the infectious agent, the immunomodulator (including combination of immunomodulators) and/or the pretreatment composition is carried out by intrarenal injection into the renal tissue having the renal tumor. In some embodiments, the administration of the infectious agent, the immunomodulator (including combination of immunomodulators) and/or the pretreatment composition is carried out by intrarenal injection directly into the renal tumor. In some embodiments, the administration of the infectious agent, the immunomodulator (including combination of immunomodulators) and/or the pretreatment composition is carried out by injection directly into metastatic sites of the renal tumor. In some embodiments, the administration of the infectious agent, the immunomodulator (including combination of immunomodulators) and/or the pretreatment composition is carried out by intrarenal injection into the renal tissue close to the renal tumor.

In some embodiments, the solid or lymphatic tumor is prostate cancer. In some embodiments, the prostate cancer is an adenocarcinoma. In some embodiments, the prostate cancer is a sarcoma, neuroendocrine tumor, small cell cancer, ductal cancer, or a lymphoma. In some embodiments, the prostate cancer is at any of the four stages, A, B, C, or D, according to the Jewett staging system. In some embodiments, the administration of the infectious agent, the immunomodulator (including combination of immunomodulators) and/or the pretreatment composition is carried out by intraprostatic injection into the prostate tissue having the prostate tumor. In some embodiments, the administration of the infectious agent, the immunomodulator (including combination of immunomodulators) and/or the pretreatment composition is carried out by intraprostatic injection directly into the prostate tumor. In some embodiments, the administration of the infectious agent, the immunomodulator (including combination of immunomodulators) and/or the pretreatment composition is carried out by injection directly into metastatic sites of the prostate tumor. In some embodiments, the administration of the infectious agent, the immunomodulator (including combination of immunomodulators) and/or the pretreatment composition is carried out by intraprostatic injection into the prostate tissue close to the prostate tumor.

In some embodiments, the solid or lymphatic tumor is lung cancer. In some embodiments, the lung cancer is a non-small cell lung cancer (NSCLC). Examples of NSCLC include, but are not limited to, large-cell carcinoma, adenocarcinoma, neuroendocrine lung tumors, and squamous cell carcinoma. In some embodiments, the administration of the infectious agent, the immunomodulator (including combination of immunomodulators) and/or the pretreatment composition is carried out by intrapulmonary injection into the lung tissue having the lung tumor. In some embodiments, the lung cancer is small cell lung cancer (SCLC). In some embodiments, the administration of the infectious agent, the immunomodulator (including combination of immunomodulators) and/or the pretreatment composition is carried out by intrapulmonary injection directly into the lung tumor. In some embodiments, the administration of the infectious agent, the immunomodulator (including combination of immunomodulators) and/or the pretreatment composition is carried out by injection directly into metastatic sites of the lung tumor. In some embodiments, the administration of the infectious agent, the immunomodulator (including combination of immunomodulators) and/or the pretreatment composition is carried out by intrapulmonary injection into the lung tissue close to the lung tumor.

In some embodiments, the solid or lymphatic tumor is melanoma. In some embodiments, the melanoma is superficial spreading melanoma, lentigo maligna melanoma, nodular melanoma, mucosal melanoma, polypoid melanoma, desmoplastic melanoma, amelanotic melanoma, soft-tissue melanoma, or acral lentiginous melanoma. In some embodiments, the melanoma is at any of stage I, II, III, or IV, according to the American Joint Committee on Cancer (AJCC) staging groups. In some embodiments, the melanoma is recurrent. In some embodiments, the administration of the infectious agent, the immunomodulator (including combination of immunomodulators) and/or the pretreatment composition is carried out by injection into the skin tissue having the melanoma tumor. In some embodiments, the administration of the infectious agent, the immunomodulator (including combination of immunomodulators) and/or the pretreatment composition is carried out by injection directly into the melanoma tumor. In some embodiments, the administration of the infectious agent, the immunomodulator (including combination of immunomodulators) and/or the pretreatment composition is carried out by injection directly into metastatic sites of the melanoma tumor. In some embodiments, the administration of the infectious agent, the immunomodulator (including combination of immunomodulators) and/or the pretreatment composition is carried out by injection into the lung tissue close to the melanoma tumor.

In some embodiments, the solid or lymphatic tumor is ovarian cancer. In some embodiments, the ovarian cancer is ovarian epithelial cancer. In some embodiments, the ovarian cancer is stage I (e.g., stage IA, IB, or IC), stage II (e.g., stage HA, HB, or IIC), stage III (e.g., stage IIIA, HIB, or HIC), or stage IV. In some embodiments, the administration of the infectious agent, the immunomodulator (including combination of immunomodulators) and/or the pretreatment composition is carried out by intraovarian injection into the ovarian tissue having the ovarian tumor. In some embodiments, the administration of the infectious agent, the immunomodulator (including combination of immunomodulators) and/or the pretreatment composition is carried out by intraovarian injection directly into the ovarian tumor. In some embodiments, the administration of the infectious agent, the immunomodulator (including combination of immunomodulators) and/or the pretreatment composition is carried out by injection directly into metastatic sites of the ovarian tumor. In some embodiments, the administration of the infectious agent, the immunomodulator (including combination of immunomodulators) and/or the pretreatment composition is carried out by intraovarian injection into the ovarian tissue close to the ovarian tumor.

In some embodiments, according to any of the methods described above, the solid or lymphatic tumor is pancreatic cancer. In some embodiments, the pancreatic cancer is a seous cystic neoplasm, mucinous cystic neoplasm, intraductal papillary mucinous neoplasm, pancreatic adenocarcinoma, adenosquamous carcinoma, squamous cell carcinoma, signet ring cell carcinoma, undifferentiated carcinoma, undifferentiated carcinoma with giant cells, solid pseudopapillary neoplasm, ampullary cancer, or pancreatic neuroendocrine tumor. In some embodiments, the pancreatic cancer is a pancreatic adenocarcinoma. In some embodiments, the administration of the infectious agent, the immunomodulator (including combination of immunomodulators) and/or the pretreatment composition is carried out by intrapancreatic injection into the pancreatic tissue having the pancreatic tumor. In some embodiments, the administration of the infectious agent, the immunomodulator (including combination of immunomodulators) and/or the pretreatment composition is carried out by intrapancreatic injection directly into the pancreatic tumor. In some embodiments, the administration of the infectious agent, the immunomodulator (including combination of immunomodulators) and/or the pretreatment composition is carried out by injection directly into metastatic sites of the pancreatic tumor. In some embodiments, the administration of the infectious agent, the immunomodulator (including combination of immunomodulators) and/or the pretreatment composition is carried out by intrapancreatic injection into the pancreatic tissue close to the pancreatic tumor.

In some embodiments, the solid or lymphatic tumor is endometrial cancer. In some embodiments, the endometrial cancer is adenocarcinoma, carcinosarcoma, squamous cell carcinoma, undifferentiated carcinoma, small cell carcinoma, or transitional carcinoma. In some embodiments, the administration of the infectious agent, the immunomodulator (including combination of immunomodulators) and/or the pretreatment composition is carried out by intraendometrial injection into the endometrial tissue having the endometrial tumor. In some embodiments, the administration of the infectious agent, the immunomodulator (including combination of immunomodulators) and/or the pretreatment composition is carried out by intraendometrial injection directly into the endometrial tumor. In some embodiments, the administration of the infectious agent, the immunomodulator (including combination of immunomodulators) and/or the pretreatment composition is carried out by injection directly into metastatic sites of the endometrial tumor. In some embodiments, the administration of the infectious agent, the immunomodulator (including combination of immunomodulators) and/or the pretreatment composition is carried out by intraendometrial injection into the endometrial tissue close to the endometrial tumor.

In some embodiments, according to any of the methods described above, the solid or lymphatic tumor is colorectal cancer. In some embodiments, the colorectal cancer is adenocarcinoma, gastrointestinal carcinoid tumor, gastrointestimal stromal tumor, leiomysarcoma, melanoma, or squamous cell carcinoma. In some embodiments, the administration of the infectious agent, the immunomodulator (including combination of immunomodulators) and/or the pretreatment composition is carried out by injection into the colorectal tissue having the colorectal tumor. In some embodiments, the administration of the infectious agent, the immunomodulator (including combination of immunomodulators) and/or the pretreatment composition is carried out by injection directly into the colorectal tumor. In some embodiments, the administration of the infectious agent, the immunomodulator (including combination of immunomodulators) and/or the pretreatment composition is carried out by injection directly into metastatic sites of the colorectal tumor. In some embodiments, the administration of the infectious agent, the immunomodulator (including combination of immunomodulators) and/or the pretreatment composition is carried out by injection into the colorectal tissue close to the colorectal tumor.

In some embodiments, according to any of the methods described above, the solid or lymphatic tumor is hepatocellular carcinoma (HCC). In some embodiments, the HCC is early stage HCC, non-metastatic HCC, primary HCC, advanced HCC, locally advanced HCC, metastatic HCC, HCC in remission, or recurrent HCC. In some embodiments, the HCC is localized resectable (i.e., tumors that are confined to a portion of the liver that allows for complete surgical removal), localized unresectable (i.e., the localized tumors may be unresectable because crucial blood vessel structures are involved or because the liver is impaired), or unresectable (i.e., the tumors involve all lobes of the liver and/or has spread to involve other organs (e.g., lung, lymph nodes, bone). In some embodiments, the HCC is, according to TNM classifications, a stage I tumor (single tumor without vascular invasion), a stage II tumor (single tumor with vascular invasion, or multiple tumors, none greater than 5 cm), a stage III tumor (multiple tumors, any greater than 5 cm, or tumors involving major branch of portal or hepatic veins), a stage IV tumor (tumors with direct invasion of adjacent organs other than the gallbladder, or perforation of visceral peritoneum), N1 tumor (regional lymph node metastasis), or M1 tumor (distant metastasis). In some embodiments, the HCC is, according to AJCC (American Joint Commission on Cancer) staging criteria, stage T1, T2, T3, or T4 HCC. In some embodiments, the HCC is any one of liver cell carcinomas, fibrolamellar variants of HCC, and mixed hepatocellular cholangiocarcinomas. In some embodiments, the administration of the infectious agent, the immunomodulator (including combination of immunomodulators) and/or the pretreatment composition is carried out by intrahepatic injection into the liver tissue having the HCC. In some embodiments, the administration of the infectious agent, the immunomodulator (including combination of immunomodulators) and/or the pretreatment composition is carried out by intrahepatic injection directly into the HCC. In some embodiments, the administration of the infectious agent, the immunomodulator (including combination of immunomodulators) and/or the pretreatment composition is carried out by injection directly into metastatic sites of the HCC. In some embodiments, the administration of the infectious agent, the immunomodulator (including combination of immunomodulators) and/or the pretreatment composition is carried out by intrahepatic injection into the tissue close to the HCC.

In some embodiments, according to any of the methods described above, the solid or lymphatic tumor is lymphoma. In some embodiments, the lymphoma is a B-cell neoplasm, a T-cell neoplasm, and/or a putative NK-cell neoplasm. Examples of B-cell neoplasms include, but are not limited to, precursor B-cell neoplasms (e.g., precursor B-lymphoblastic leukemia/lymphoma) and peripheral B-cell neoplasms (e.g., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (e.g., cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (e.g., extranodal (e.g., MALT-type +/- monocytoid B cells) and/or Nodal (e.g., +/- monocytoid B cells)), splenic marginal zone lymphoma (e.g., +/- villous lymphocytes), Hairy cell leukemia, plasmacytoma/plasma cell myeloma (e.g., myeloma and multiple myeloma), diffuse large B-cell lymphoma (e.g., primary mediastinal (thymic) B-cell lymphoma), intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia). Examples of T-cell and/or putative NK-cell neoplasms include, but are not limited to, precursor T-cell neoplasm (precursor T-lymphoblastic lymphoma/leukemia) and peripheral T-cell and NK-cell neoplasms (e.g., T-cell chronic lymphocytic leukemia/prolymphocytic leukemia, and large granular lymphocyte leukemia (LGL) (e.g., T-cell type and/or NK-cell type), cutaneous T-cell lymphoma (e.g., mycosis fungoides/Sezary syndrome), primary T-cell lymphomas unspecified (e.g., cytological categories (e.g., medium-sized cell, mixed medium and large cell), large cell, lymphoepitheloid cell, subtype hepatosplenic $\gamma\delta$ T-cell lymphoma, and subcutaneous panniculitic T-cell lymphoma), angioimmunoblastic T-cell lymphoma (AILD), angiocentric lymphoma, intestinal T-cell lymphoma (e.g., +/- enteropathy associated), adult T-cell lymphoma/leukemia (ATL), anaplastic large cell lymphoma (ALCL) (e.g., CD30+, T- and null-cell types), anaplastic large-cell lymphoma, and Hodgkin's like). In some embodiments, the lymphoma is Hodgkin's disease or Non-Hodgkin Lymphoma (NHL). For example, the Hodgkin's disease may be lymphocyte predominance, nodular sclerosis, mixed cellularity, lymphocyte depletion, and/or lymphocyte-rich. In some embodiments, the administration of the infectious agent, the immunomodulator (including combination of immunomodulators) and/or the pretreatment composition is carried out by intralymphatic injection into the lymph node having the lymphatic tumor. In some embodiments, the administration of the infectious agent, the immunomodulator (including combination of immunomodulators) and/or the pretreatment composition is carried out by intralymphatic injection directly into the lymphatic tumor. In some embodiments, the administration of the infectious agent, the immunomodulator (including combination of immunomodulators) and/or the pretreatment composition is carried out by injection directly into metastatic sites of the lymphatic tumor. In some embodiments, the administration of the infectious agent, the immunomodulator (including combination of immunomodulators) and/or the pretreatment composition is carried out by intralymphatic injection into the tissue close to the lymphatic tumor.

In some embodiments, according to any of the methods described above, the solid or lymphatic tumor is mesothelioma. In some embodiments, the mesothelioma is pleural mesothelioma, peritoneal mesothelioma, pericardial mesothelioma, or mesothelioma affecting mesothelial tissue covering other organs. In some embodiments, the mesothelioma is benign mesothelioma or malignant mesothelioma. In some embodiments, the mesothelioma is epithelial mesothelioma, sarcomatoid mesothelioma, biphasic mesothelioma, or papillary mesothelioma. In some embodiments, the administration of the infectious agent, the immunomodulator (including combination of immunomodulators) and/or the pretreatment composition is carried out by injection into the mesothelial tissue having the mesothelioma. In some embodiments, the administration of the infectious agent, the immunomodulator (including combination of immunomodulators) and/or the pretreatment composition is carried out by injection directly into the mesothelioma. In some embodiments, the administration of the infectious agent, the immunomodulator (including combination of immunomodulators) and/or the pretreatment composition is carried out by injection directly into metastatic sites of the mesothelioma. In some embodiments, the administration of the infectious agent, the immunomodulator (including combination of immunomodulators) and/or the pretreatment composition is carried out by injection into the mesothelial tissue close to the mesothelioma.

In some embodiments, according to any of the methods described above, the solid or lymphatic tumor is brain tumor. In some embodiments, the brain tumor is primary brain tumor or secondary (or metastatic) brain tumor. In some embodiments, the brain tumor is glioma (such as astrocytoma, oligodendroglioma, or ependymoma), meningioma, Schwannoma, craniopharyngioma, germ cell tumor, or pineal region tumor. In some embodiments, the administration of the infectious agent, the immunomodulator (including combination of immunomodulators) and/or the pretreatment composition is carried out by injection into the brain tissue having the brain tumor. In some embodiments, the administration of the infectious agent, the immunomodulator (including combination of immunomodulators) and/or the pretreatment composition is carried out by injection directly into the brain tumor. In some embodiments, the administration of the infectious agent, the immunomodulator (including combination of immunomodulators) and/or the pretreatment composition is carried out by injection directly into metastatic sites of the brain tumor. In some embodiments, the administration of the infectious agent, the immunomodulator (including combination of immunomodulators) and/or the pretreatment composition is carried out by injection into the brain tissue close to the brain tumor.

In some embodiments, according to any of the methods described above, the solid or lymphatic tumor is gallbladder and bile duct tumor. In some embodiments, the gallbladder and bile duct tumor is carcinoma, adenocarcinoma, cholangiocarcinoma, papillary tumor, small cell (neuroendocrine) carcinoma, adenosquamous carcinoma, or rhabdomyosarcoma. In some embodiments, the gallbladder and bile duct tumor is gallbladder carcinoma, carcinoma of extrahepatic bile duct, or carcinoma of intrahepatic bile duct. In some embodiments, the administration of the infectious agent, the immunomodulator (including combination of immunomodulators) and/or the pretreatment composition is carried out by injection into the gallbladder or bile duct tissue having the gallbladder and bile duct tumor. In some embodiments, the administration of the infectious agent, the immunomodulator (including combination of immunomodulators) and/or the pretreatment composition is carried out by injection directly into the gallbladder and bile duct tumor. In some embodiments, the administration of the infectious agent, the immunomodulator (including combination of immunomodulators) and/or the pretreatment composition is carried out by injection directly into metastatic sites of the gallbladder and bile duct tumor. In some embodiments, the administration of the infectious agent, the immunomodulator (including combination of immunomodulators) and/or the pretreatment composition is carried out by injection into the gallbladder or bile duct tissue close to the gallbladder and bile duct tumor.

In some embodiments, according to any of the methods described above, the solid or lymphatic tumor is soft tissue sarcoma. In some embodiments, the soft tissue sarcoma is adult fibrosarcoma, alveolar soft-part sarcoma, angiosarcoma, clear cell sarcoma, desmoplastic small round cell tumor, epitheloid sarcoma, fibromyxoid sarcoma, liposarcoma, malignant mesenchymoma, malignant peripheral nerve sheath tumor (e.g., neurofibrosarcoma, malignant schwannoma, or neurogenic sarcoma), myxofibrosarcoma, synovial sarcoma, undifferentiated pleomorphic sarcoma, dermatofibrosarcoma protuberan, fibromatosis, hemangioendothelioma, infantile fibrosarcoma, solitary fibrous tumor, elastofibroma, fibroma, fibrous histocytoma, glomus tumor, granular cell tumor, hemangioma, hibernoma, lipoma, leiomyoma, leiomyoma, lipoblastoma, lymphangioma, myxoma, neurofibroma, neuroma, PEComa, rhabdomyoma, schwannoma, tenosynovial giant cell tumor, spindle cell tumor, or tumor-like conditions of soft tissue. In some embodiments, the administration of the infectious agent, the immunomodulator (including combination of immunomodulators) and/or the pretreatment composition is carried out by injection into the tissue having the soft tissue sarcoma. In some embodiments, the administration of the infectious agent, the immunomodulator (including combination of immunomodulators) and/or the pretreatment composition is carried out by injection directly into the soft tissue sarcoma. In some embodiments, the administration of the infectious agent, the immunomodulator (including combination of immunomodulators) and/or the pretreatment composition is carried out by injection directly into metastatic sites of the soft tissue sarcoma. In some embodiments, the administration of the infectious agent, the immunomodulator (including combination of immunomodulators) and/or the pretreatment composition is carried out by injection into the tissue close to the soft tissue sarcoma.

In some embodiments, according to any of the methods described above, the solid or lymphatic tumor is uterine tumor. In some embodiments, the uterine tumor is uterine carcinoma, uterine sarcoma (such as endometrial stromal sarcoma, undifferentiated sarcoma, or uterine leiomyosarcoma), or uterine carcinosarcoma (such as malignant mixed mesodermal tumor, or malignant mixed mullerian tumor). In some embodiments, the uterine tumor is a fibroid tumor, such as leiomyoma, adenofibroma, or adenomyoma. In some embodiments, the administration of the infectious agent, the immunomodulator (including combination of immunomodulators) and/or the pretreatment composition is carried out by intrauterine injection into the uterine tissue having the uterine tumor. In some embodiments, the administration of the infectious agent, the immunomodulator (including combination of immunomodulators) and/or the pretreatment composition is carried out by intrauterine injection directly into the uterine tumor. In some embodiments, the administration of the infectious agent, the immunomodulator (including combination of immunomodulators) and/or the pretreatment composition is carried out by injection directly into metastatic sites of the uterine tumor. In some embodiments, the administration of the infectious agent, the immunomodulator (including combination of immunomodulators) and/or the pretreatment composition is carried out by intrauterine injection into the uterine tissue close to the uterine tumor.

In some embodiments, according to any of the methods described above, the solid or lymphatic tumor is cervical tumor. In some embodiments, the cervical tumor is squamous cell carcinoma, adenocarcinoma, or adenosquamous carcinoma. In some embodiments, the administration of the infectious agent, the immunomodulator (including combination of immunomodulators) and/or the pretreatment composition is carried out by intracervical injection into the cervical tissue having the cervical tumor. In some embodiments, the administration of the infectious agent, the immunomodulator (including combination of immunomodulators) and/or the pretreatment composition is carried out by intracervical injection directly into the cervical tumor. In some embodiments, the administration of the infectious agent, the immunomodulator (including combination of immunomodulators) and/or the pretreatment composition is carried out by injection directly into metastatic sites of the cervical tumor. In some embodiments, the administration of the infectious agent, the immunomodulator (including combination of immunomodulators) and/or the pretreatment composition is carried out by intracervical injection into the cervical tissue close to the cervical tumor.

In some embodiments, according to any of the methods described above, the solid or lymphatic tumor is thyroid tumor. In some embodiments, the thyroid tumor is differentiated thyroid tumor (such as papillary carcinoma, follicular carcinoma, or Hurthle cell carcinoma), medullary thyroid carcinoma, anaplastic carcinoma, thyroid lymphoma, thyroid sarcoma, or parathyroid tumor. In some embodiments, the administration of the infectious agent, the immunomodulator (including combination of immunomodulators) and/or the pretreatment composition is carried out by injection into the thyroid tissue having the thyroid tumor. In some embodiments, the administration of the infectious agent, the immunomodulator (including combination of immunomodulators) and/or the pretreatment composition is carried out by injection directly into the thyroid tumor. In some embodiments, the administration of the infectious agent, the immunomodulator (including combination of immunomodulators) and/or the pretreatment composition is carried out by injection directly into metastatic sites of the thyroid tumor. In some embodiments, the administration of the infectious agent, the immunomodulator (including combination of immunomodulators) and/or the pretreatment composition is carried out by injection into the thyroid tissue close to the thyroid tumor.

In some embodiments, according to any of the methods described above, the solid or lymphatic tumor is nasopharyngeal carcinoma. In some embodiments, the nasopharyngeal carcinoma is keratinizing squamous cell carcinoma, non-keratinizing differentiated carcinoma, or undifferentiated carcinoma (e.g., lymphoepithelioma), oral cavity and oropharyngeal tumor, nasal cavity and paranasal sinus tumor, or salivary gland tumor. In some embodiments, the administration of the infectious agent, the immunomodulator (including combination of immunomodulators) and/or the pretreatment composition is carried out by injection into the nasopharyngeal tissue having the nasopharyngeal carcinoma. In some embodiments, the administration of the infectious agent, the immunomodulator (including combination of immunomodulators) and/or the pretreatment composition is carried out by injection directly into the nasopharyngeal carcinoma. In some embodiments, the administration of the infectious agent, the immunomodulator (including combination of immunomodulators) and/or the pretreatment composition is carried out by injection directly into metastatic sites of the nasopharyngeal carcinoma. In some embodiments, the administration of the infectious agent, the immunomodulator (including combination of immunomodulators) and/or the pretreatment composition is carried out by injection into the nasopharyngeal tissue close to the nasopharyngeal carcinoma.

In some embodiments, the individual is a human individual. In some embodiments, the individual being treated for solid or lymphatic tumor has been identified as having one or more of the conditions described herein. Identification of the conditions as described herein by a skilled physician is routine in the art (e.g., via blood tests, X-rays, ultrasound, CT scans, PET scans, PET/CT scans, MRI scans, PET/MRI scans, nuclear medicine radioisotope scans, endoscopy, biopsy, angiography, CT-angiography, etc.) and may also be suspected by the individual or others, for example, due to tumor growth, hemorrhage, ulceration, pain, enlarged lymph nodes, cough, jaundice, swelling, weight loss, cachexia, sweating, anemia, paraneoplastic phenomena, thrombosis, etc. In some embodiments, the individual is selected for any one of the treatment methods described herein based on any one or more of a number of risk factors and/or diagnostic approaches appreciated by the skilled artisan, including, but not limited to, genetic profiling, family history, medical history (e.g., appearance of related conditions and viral infection history), lifestyle or habits.

In some embodiments, the individual is selected for any one of the treatment methods described herein based on the expression level of one or more biomarkers, including, but not limited to, immune checkpoint molecules, co-stimulatory molecules, cytokines, chemokines, other immune-related molecules, and HLA-Class II antigens. In some embodiments, the individual is selected for the treatment based on the expression level (e.g., high expression level) of one or more inhibitory immune checkpoint molecules, including, but not limited to, CTLA-4, PD-1, PD-L1, PD-L2, TIM3, B7-H3, B7-H4, LAG-3, KIR, 2B4 and ligands thereof. In some embodiments, the individual is selected for the treatment methods based on the expression level (e.g., low expression level) of one or more stimulatory immune checkpoint molecules or co-stimulatory molecules, including, but not limited to, OX40, 4-1BB, CD40, and ligands thereof. In some embodiments, the individual is selected for the treatment based on the expression level (e.g., high expression level) of one or more biomarkers selected from the group consisting of PD-1, PD-L1, and PD-L2 in the tumor (such as tumor cells and/or immune cells inside the tumor). In some embodiments, the individual is selected for the treatment based on the expression level (e.g., high expression level) of one or more biomarkers selected from the group consisting of CD80, CD83, CD86 and HLA-Class II antigens in tumor-derived mature dendritic cells. In some embodiments, the individual is selected for the treatment based on the expression level (e.g., high expression level) of one or more biomarkers selected from the group consisting of CXCL9, CXCL10, CXCL11, CCR7, CCL5, CCL8, SOD2, MT2A, OASL, GBP1, HES4, MTIB, MTIE, MTIG, MTIH, GADD45A, LAMP3 and miR-155.

In some embodiments, the individual has high expression of one or more inhibitory immune checkpoint molecules. In some embodiments, the individual has low expression of one or more stimulatory immune checkpoint molecule and/or co-stimulatory molecules. In some embodiments, the individual has high expression of one or more biomarkers selected from the group consisting of PD-1, PD-L1, and PD-L2 in the tumor (such as tumor cells and/or immune cells inside the tumor). In some embodiments, PD-L1 and PD-L2 can be used interchangeably as a biomarker for selecting patients or as a ligand for inhibiting PD-1. In some embodiments, the individual has high expression of one or more biomarkers selected from the group consisting of CD80, CD83, CD86 and HLA-Class II antigens in tumor-derived mature dendritic cells. Exemplary HLA-Class II antigens include, but are not limited to, tumor-specific antigens and tumor-associated antigens expressed in the solid or lymphatic tumor, such as PSA for prostate tumor, alpha fetoprotein for HCC, CEA for adenocarcinoma. In some embodiments, the individual has high expression of one or more biomarkers selected from the group consisting of CXCL9, CXCL10, CXCL11, CCR7, CCL5, CCL8, SOD2, MT2A, OASL, GBP1, HES4, MTIB, MTIE, MTIG, MTIH, GADD45A, LAMP3 and miR-155. In some embodiments, the method further comprises assessing the expression level of one or more biomarkers in the individual. In some embodiments, the method is adjusted based on the expression level of the one or more biomarkers.

Expression level of a biomarker may be measured at the nucleic acid level (e.g., gene copy number, DNA methylation or chromatin remodeling level, mRNA level), or protein level, including post-translational modification level of the protein, such as phosphorylation level of the protein corresponding to the biomarker. Expression level can be determined using any of the known methods in the art. For example, suitable methods for determining the mRNA expression level of a biomarker include, but are not limited to, Reverse Transcription Polymerase Chain Reaction (RT-PCR), quantitative PCR, microarray, and RNA sequencing. For example, suitable methods for determining the protein expression level of a biomarker include, but are not limited to, immunohistochemistry, Western blotting, and mass spectroscopy methods.

The expression level of the biomarker may be determined using a fresh or archived sample from the individual, including, but not limited to, the solid or lymphatic tumor tissue, a normal tissue adjacent to the solid or lymphatic tumor tissue, a normal tissue distal to the solid or lymphatic tumor tissue, or peripheral blood lymphocytes. In some embodiments, the sample is solid or lymphatic tumor tissue. In some embodiments, the sample is a biopsy containing tumor cells, such as fine needle aspiration of tumor cells. In some embodiments, the biopsied cells are centrifuged into a pellet, fixed, and embedded in paraffin prior to the analysis. In some embodiments, the biopsied cells are flash frozen prior to the analysis. In some embodiments, the sample is a bodily fluid, such as a blood sample or a plasma sample. In some embodiments, the sample comprises a circulating metastatic cancer cell. In some embodiments, the sample is obtained by sorting circulating tumor cells (CTCs) from blood.

In some embodiments, the expression levels of the one or more biomarkers in a specific cell population of the individual are determined using a sample from the individual. In some embodiments, the sample comprises immune cells isolated or derived from the solid or lymphatic tumor. Exemplary immune cells that are relevant for biomarker expression determination include, but are not limited to, dendritic cells (such as immature or mature dendritic cells), B cells, T cells (such as Th1 cells, Th2 cells, Th17 cells, NK T cells, Treg cells, etc.), Natural Killer (NK) cells, monocytes, macrophages, neutrophils, and combinations thereof. In some embodiments, the sample comprises tumor infiltrating lymphocytes. In some embodiments, the sample comprises tumor-derived mature dendritic cells. The specific cell population can be isolated from a sample, such as a tumor sample (e.g., tumor biopsy or resection) or a body fluid (e.g., blood sample), using methods known in the art, such as flow cytometry methods based on expression of specific cell surface molecules in the cell population.

High or low expression level of a biomarker is determined as compared to a standard expression level of the biomarker known in the art (e.g., a clinically accepted normal level in a standardized test), or as compared to the expression level of the biomarker in a control sample. In some embodiments, the expression level of the biomarker in an individual is compared to the expression level of the biomarker in multiple control samples. In some embodiments, multiple control samples are used to generate a statistic that is used to classify the level of the biomarker in an individual with the solid or lymphatic tumor. Control samples can be obtained from the same sources (e.g., individual and tissue) and methods as non-control samples. In some embodiments, the control sample is obtained from a different individual (for example an individual not having the solid or lymphatic tumor; an individual having a benign or less advanced form of the solid or lymphatic tumor; and/or an individual sharing similar ethnic, age, and gender). In some embodiments, the control sample is a cultured tissue or cell that has been determined to be a proper control. In some embodiments, wherein the sample is solid or lymphatic tumor tissue sample, the control sample may be a non-cancerous sample from the same individual. In some embodiments, multiple control samples (for example from different individuals) are used to determine a range of levels of the biomarker in a particular tissue, organ, or cell population. In some embodiments, the expression level of the biomarker in a sample of the individual is classified as high, medium or low according to a scoring system, such as an immunohistochemistry-based scoring system. In some embodiments, high expression of the biomarker is at least about any one of 1.5 times, 2 times, 3 times, 5 times, 10 times, 20 times, 50 times, 100 times, 200 times, 500 times, 1000 times or more than the expression level of the biomarker in a sample from the individual as compared to a control sample. In some embodiments, low expression of the biomarker is no more than about any one of 90%, 80%, 70%, 60%, 50%, 40%, 30%, 20%, 10%, 5%, 1%, 0.1%, 0.01%, 0.001% or less than the expression level of the biomarker in a sample from the individual as compared to a control sample. In some embodiments, the expression levels of two or more biomarkers are combined, for example, using a statistic model to determine an expression score, for selecting or recommending the individual for the treatment.

**Medical uses fortreating bladder cancer by intravesical administrations**

One aspect of the present application relates to treatment of bladder cancer. In this context, local administration of the infectious agent and the immunomodulator (including combination of immunomodulators) may encompass intravesical administration of one or both components. Any of the methods described herein may be useful for inhibiting growth of a bladder tumor, inhibiting metastasis of a bladder tumor, prolonging survival (such as disease-free survival) of an individual having a bladder cancer, causing disease remission in an individual having a bladder cancer, and/or improving quality of life of an individual having a bladder cancer.

In some embodiments, there is provided in combination, an infectious agent and immunomodulator for use in a method of treating bladder cancer in an individual, comprising: a) intravesically administering an effective amount of an infectious agent wherein the infectious agent is an oncolytic virus; and b) intravesically administering an effective amount of an immunomodulator (including combination of immunomodulators), wherein the immunomodulator is an inhibitor of PD-L1. In some embodiments, the infectious agent is a virus, such as a virus selected from the group consisting of adenovirus, herpes simplex virus, vaccinia virus, mumps virus, newcastle disease virus, polio virus, measles virus, Seneca valley virus, coxsackie virus, reo virus, vesicular stomatitis virus, maraba and rhabdovirus, and parvovirus. In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the immunomodulator is an immune checkpoint inhibitor. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the infectious agent is administered weekly. In some embodiments, the immunomodulator (including combination of immunomodulators) is administered weekly. In some embodiments, the method further comprises administration of the infectious agent and/or the immunomodulator (including combination of immunomodulators) by an administration route other than intravesical administration.

In some embodiments, the oncolytic virus is a wild type oncolytic virus. In some embodiments, the oncolytic virus is genetically modified. In some embodiments, the oncolytic virus is attenuated (for example through multiple passages, inactivation or genetic modification). In some embodiments, the oncolytic virus is replication competent. In some embodiments, the oncolytic virus preferentially replicates in a cancer cell. In some embodiments, the immunomodulator is an immune checkpoint inhibitorIn some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the oncolytic virus is administered weekly. In some embodiments, the immunomodulator (including combination of immunomodulators) is administered weekly. In some embodiments, the method further comprises administration of the oncolytic virus and/or the immunomodulator (including combination of immunomodulators) by an administration route other than intravesical administration.

In some embodiments, there is provided in combination, an infectious agent and immunomodulator for use in a method of treating bladder cancer in an individual, comprising: a) intravesically administering an effective amount of an oncolytic virus (such as oncolytic adenovirus) comprising a viral vector comprising a tumor cell-specific promoter operably linked to a viral gene essential for replication of the virus; and b) intravesically administering an effective amount of an immunomodulator (including combination of immunomodulators), wherein the immunomodulator is an inhibitor of PD-L1. In some embodiments, the immunomodulator is an immune checkpoint inhibitor. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the tumor-specific promoter is an E2F-1 promoter, such as a human E2F-1 promoter or an E2F-1 promoter comprising the nucleotide sequence set forth in SEQ ID NO:1. In some embodiments, the viral gene essential for replication of the virus is selected from the group consisting of E1A, E1B, and E4. In some embodiments, the oncolytic virus is administered weekly. In some embodiments, the immunomodulator (including combination of immunomodulators) is administered weekly. In some embodiments, the method further comprises administration of the oncolytic virus and/or the immunomodulator (including combination of immunomodulators) by an administration route other than intravesical administration.

In some embodiments, there is provided in combination, an infectious agent and immunomodulator for use in a method of treating bladder cancer in an individual, comprising: a) intravesically administering an effective amount of an oncolytic virus (such as oncolytic adenovirus) comprising a viral vector comprising a tumor cell-specific promoter operably linked to a viral gene essential for replication of the virus and a nucleic acid encoding a cytokine operably linked to a viral promoter; and b) intravesically administering an effective amount of an immunomodulator (including combination of immunomodulators), wherein the immunomodulator is an inhibitor of PD-L1. In some embodiments, the immunomodulator is an immune checkpoint inhibitor. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4. In some embodiments, the viral promoter operably linked to the nucleic acid encoding the cytokine is the E3 promoter. In some embodiments, the cytokine is GM-CSF. In some embodiments, the oncolytic virus is administered weekly. In some embodiments, the immunomodulator (including combination of immunomodulators) is administered weekly. In some embodiments, the method further comprises administration of the oncolytic virus and/or the immunomodulator (including combination of immunomodulators) by an administration route other than intravesical administration.

In some embodiments, there is provided in combination, an infectious agent and immunomodulator for use in a method of treating bladder cancer in an individual, comprising: a) intravesically administering an effective amount of an adenovirus serotype 5, wherein the endogenous E1a promoter and E3 19kD coding region of a native adenovirus is replaced by the human E2F-1 promoter and a nucleic acid encoding an immune-related molecule (such as cytokine or chemokine); and b) intravesically administering an effective amount of an immunomodulator (including combination of immunomodulators), wherein the immunomodulator is an inhibitor of PD-L1. In some embodiments, the immunomodulator is an immune checkpoint inhibitor. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the tumor-specific promoter is a human E2F-1 promoter or an E2F-1 promoter comprising the nucleotide sequence set forth in SEQ ID NO:1. In some embodiments, the adenovirus is administered weekly. In some embodiments, the immunomodulator (including combination of immunomodulators) is administered weekly. In some embodiments, the method further comprises administration of the adenovirus and/or the immunomodulator (including combination of immunomodulators) by an administration route other than intravesical administration.

In some embodiments, there is provided in combination, an infectious agent and immunomodulator for use in a method of treating bladder cancer in an individual, comprising: a) intravesically administering an effective amount of CG0070; and b) intravesically administering an effective amount of an immunomodulator (including combination of immunomodulators), wherein the immunomodulator is an inhibitor of PD-L1. In some embodiments, the immunomodulator is an immune checkpoint inhibitor. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the CG0070 is administered weekly. In some embodiments, the immunomodulator (including combination of immunomodulators) is administered weekly. In some embodiments, the method further comprises administration of CG0070 and/or the immunomodulator (including combination of immunomodulators) by an administration route other than intravesical administration.

The methods described herein can be used to treat a variety of bladder cancer conditions. In some embodiments, the bladder cancer is a low grade bladder cancer. In some embodiments, the bladder cancer is a high grade bladder cancer. In some embodiments, the bladder cancer is muscle invasive (e.g., T2, T3 or T4). In some embodiments, the bladder cancer is non-invasive (e.g., Ta, T1 Cis, Cis with Ta and/or T1).

In some embodiments, the bladder cancer is transitional cell carcinoma or urothelial carcinoma (such as metastatic urothelial carcinoma), including, but not limited to, papillary tumors and flat carcinomas. In some embodiments, the bladder cancer is metastatic urothelial carcinoma. In some embodiments, the bladder cancer is urothelial carcinoma of the bladder. In some embodiments, the bladder cancer is urothelial carcinoma of the ureter. In some embodiments, the bladder cancer is urothelial carcinoma of the urethra. In some embodiments, the bladder cancer is urothelial carcinoma of the renal pelvis.

In some embodiments, the bladder cancer is squamous cell carcinoma. In some embodiments, the bladder cancer is non-squamous cell carcinoma. In some embodiments, the bladder cancer is adenocarcinoma. In some embodiments, the bladder cancer is small cell carcinoma.

In some embodiments, the bladder cancer is early stage bladder cancer, non-metastatic bladder cancer, non-invasive bladder cancer, non-muscle-invasive bladder cancer, primary bladder cancer, advanced bladder cancer, locally advanced bladder cancer (such as unresectable locally advanced bladder cancer), metastatic bladder cancer, or bladder cancer in remission. In some embodiments, the bladder cancer is localized resectable, localized unresectable, or unresectable. In some embodiments, the bladder cancer is a high grade, non-muscle-invasive cancer that has been refractory to standard intra-bladder infusion (intravesical) therapy.

The methods provided herein can be used to treat an individual (e.g., human) who has been diagnosed with or is suspected of having bladder cancer. In some embodiments, the individual has undergone a tumor resection. In some embodiments, the individual has refused surgery. In some embodiments, the individual is medically inoperable. In some embodiments, the individual is at a clinical stage of Ta, Tis, T1, T2, T3a, T3b, or T4 bladder cancer. In some embodiments, the individual is at a clinical stage of Tis, CIS, Ta, or T1.

In some embodiments, the individual has been previously treated for bladder cancer (also referred to as the "prior therapy"). In some embodiments, individual has been previously treated with a standard therapy for bladder cancer. In some embodiments, the prior standard therapy is treatment with BCG. In some embodiments, the prior standard therapy is treatment with mitomycin C. In some embodiments, the prior standard therapy is treatment with interferon (such as interferon-$\alpha$). In some embodiments, the individual has bladder cancer in remission, progressive bladder cancer, or recurrent bladder cancer. In some embodiments, the individual is resistant to treatment of bladder cancer with other agents (such as platinum-based agents, BCG, mitomycin C, and/or interferon). In some embodiments, the individual is initially responsive to treatment of bladder cancer with other agents (such as platinum-based agents, or BCG) but has progressed after treatment.

In some embodiments, the individual has recurrent bladder cancer (such as a bladder cancer at the clinical stage of Ta, Tis, T1, T2, T3a, T3b, or T4) after a prior therapy (such as prior standard therapy, for example treatment with BCG). For example, the individual may be initially responsive to the treatment with the prior therapy, but develops bladder cancer after about any of about 2, 3, 4, 5, 6, 7, 8, 9, 10, 11, 12, 24, 36, 48, or 60 months upon the cessation of the prior therapy.

Any of the immunomodulators described herein, including immune-stimulating agents and immune checkpoint inhibitors, may be used in the combination therapy for intravesical administration. The immunomodulator can be of any one of the molecular modalities known in the art, including, but not limited to, aptamer, mRNA, siRNA, microRNA, shRNA, peptide, antibody, anticalin, Spherical nucleic acid, TALEN, Zinc Finger Nuclease, CRISPR/Cas9, and small molecule.

An immune-stimulating agent can be a natural or engineered ligand of an immune stimulatory molecule, including, for example, ligands of OX40 (e.g., OX40L), ligands of CD-28 (e.g., CD80, CD86), ligands of ICOS (e.g., B7RP1), ligands of 4-1BB (e.g., 4-1BBL, Ultra4-1BBL), ligands of CD27 (e.g., CD70), ligands of CD40 (e.g., CD40L), and ligands of TCR (e.g., MHC class I or class II molecules, IMCgp100). In some embodiments, the immune-stimulating agent is an antibody selected from the group consisting of anti-CD28 (e.g., TGN-1412), anti-OX40 (e.g., MEDI6469, MEDI-0562), anti-ICOS (e.g., MEDI-570), anti-GITR (e.g., TRX518, INBRX-110, NOV-120301), anti-41-BB (e.g., BMS-663513, PF-05082566), anti-CD27 (e.g., BION-1402, Varlilumab and hCD27.15), anti-CD40 (e.g., CP870,893, BI-655064, BMS-986090, APX005, APX005M), anti-CD3 (e.g., blinatumomab, muromonab), and anti-HVEM. In some embodiments, the antibody is an agonistic antibody. In some embodiments, the antibody is a monoclonal antibody. In some embodiments, the antibody is an antigen-binding fragment selected from the group consisting of Fab, Fab', $F(ab')_2$, Fv, scFv, and other antigen-binding subsequences of the full length antibody. In some embodiments, the antibody is a human, humanized, or chimeric antibody. In some embodiments, the antibody is a bispecific antibody, a multispecific antibody, a single domain antibody, a fusion protein comprising an antibody portion, or any other functional variants or derivatives thereof.

In some embodiments, the immunomodulator is an immune checkpoint inhibitor. In some embodiments, the immune-checkpoint inhibitor is a natural or engineered ligand of an inhibitory immune checkpoint molecule, including, for example, ligands of CTLA-4 (e.g., B7.1, B7.2), ligands of PD-1 (PD-L1). In some embodiments, the immune checkpoint inhibitor is an antibody that targets an inhibitory immune checkpoint protein. In some embodiments, the immunomodulator is an antibody selected from the group consisting of anti-CTLA-4 (e.g., Ipilimumab, Tremelimumab, KAHR-102), and anti-PD-L1 (e.g., KY-1003 (EP20120194977). In some embodiments, the antibody is an antagonistic antibody. In some embodiments, the antibody is a monoclonal antibody. In some embodiments, the antibody is a monoclonal antibody. In some embodiments, the antibody is an antigen-binding fragment selected from the group consisting of Fab, Fab', $F(ab')_2$, Fv, scFv, and other antigen-binding subsequences of the full length antibody. In some embodiments, the antibody is a human, humanized, or chimeric antibody. In some embodiments, the antibody is a bispecific antibody, a multispecific antibody, a single domain antibody, a fusion protein comprising an antibody portion, or any other functional variants or derivatives thereof.

In some embodiments, the method comprises intravesical administration of a single immunomodulator. In some embodiments, the immunomodulator is an immune checkpoint inhibitor. In some embodiments, the immunomodulator is an immune-stimulating agent.

In some embodiments, the method comprises intravesical administration of at least two (such as any of 2, 3, 4, 5, 6, or more) immunomodulators. In some embodiments, all or part of the at least two immunomodulators are administered simultaneously, such as in a single composition. In some embodiments, all or part of the at least two immunomodulators are administered sequentially. In some embodiments, the method comprises intravesical administration of a combination of immunomodulators comprising an immune checkpoint inhibitor and an immune-stimulating agent. In some embodiments, the method comprises intravesical administration of a combination of immunomodulators comprising two or more (such as any of 2, 3, 4, 5, 6, or more) checkpoint inhibitors. In some embodiments, the method comprises intravesical administration of a combination of immunomodulators comprising two or more (such as any of 2, 3, 4, 5, 6, or more) immune-stimulating agents. In some embodiments, the method comprises intravesical administration of a combination of immunomodulators comprising any number (such as any of 1, 2, 3, 4, 5, 6, or more) of immune checkpoint inhibitors and any number (such as any of 2, 3, 4, 5, 6, or more) of immune-stimulating agents. For example, in some embodiment, the method comprises: a) intravesically administering to the site of the tumor an effective amount of an infectious agent (such as a virus, for example an oncolytic virus); and b) intravesically administering to the individual an effective amount of a first immunomodulator (such as an immune checkpoint inhibitor); and c) intravesically administering to the site of the tumor an effective amount of a second immunomodulator (such as an immune-stimulating agent). In some embodiments, the method comprises intravesical administration of a CTLA-4 inhibitor (such as an anti-CTLA-4 antibody, for example Ipilimumab, or an engineered lipocalin protein, for example an anticalin that specifically recognizes CTLA-4) and a CD40 agonist (such as an agnostic anti-CD40 antibody, for example, APX005M). In some embodiments, the method comprises intravesical administration of a CTLA-4 inhibitor (such as an anti-CTLA-4 antibody, for example Ipilimumab, or an engineered lipocalin protein, for example an anticalin that specifically recognizes CTLA-4) and a 4-1BB agonist (such as an agonistic anti-4-1BB antibody, e.g., PF-05082566).

In some embodiments, there is provided in combination, an infectious agent and immunomodulator for use in a method of treating bladder cancer in an individual (such as a human), comprising: a) intravesically administering an effective amount of an infectious agent wherein the infectious agent is an oncolytic virus; and b) intravesically administering an effective amount of an inhibitor of PD-1 ligand PD-L1 (such as an anti-PD-L1antibody, or an inhibitor of both PD-L1 and PD-L2). In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the inhibitor of PD-1 ligand is an anti-PD-L1 antibody, for example, KY-1003, MCLA-145, RG7446, BMS935559, MPDL3280A, MEDI4736, Avelumab, or STI-A1010. In some embodiments, the inhibitor of PD-1 ligand is an inhibitor (e.g., peptide, protein or small molecule) of both PD-L1 and PD-L2, such as AUR-012, and AMP-224. In some embodiments, the method further comprises intravesical administration of a second immunomodulator, such as an immune-stimulating agent (e.g., a CD40 activator or a 4-1BB activator). In some embodiments, the infectious agent is administered weekly. In some embodiments, the inhibitor of PD-1 ligand is administered weekly. In some embodiments, the infectious agent and the inhibitor of PD-1 ligand are administered sequentially. In some embodiments, the infectious agent is administered prior to (such as immediately prior to) the administration of the inhibitor of PD-1 ligand. In some embodiments, the infectious agent is administered after (such as immediately after) the administration of the inhibitor of PD-1 ligand. In some embodiments, the infectious agent and the inhibitor of PD-1 ligand are administered simultaneously (for example in a single composition). In some embodiments, the method further comprises administration of the infectious agent and/or the inhibitor of PD-1 ligand by an administration route other than intravesical administration. In some embodiments, the inhibitor of PD-L1 and the inhibitor of PD-L2 can be used interchangeably in any of the methods of treatment described herein.

For example, in some embodiments, there is provided in combination, an infectious agent and immunomodulator for use in a method of treating bladder cancer in an individual, comprising: a) intravesically administering an effective amount of an oncolytic virus (such as oncolytic adenovirus); and b) intravesically administering an effective amount of an inhibitor of PD-1 ligand PD-L1 (such as an anti-PD-L1 antibody, or an inhibitor of both PD-L1 and PD-L2).

In some embodiments, there is provided in combination, an infectious agent and immunomodulator for use in a method of treating bladder cancer in an individual, comprising: a) intravesically administering an effective amount of an oncolytic virus (such as oncolytic adenovirus) comprising a viral vector comprising a tumor cell-specific promoter operably linked to a viral gene essential for replication of the virus; and b) intravesically administering an effective amount of an inhibitor of PD-1 ligand PD-L1 (such as an anti-PD-L1antibody, or an inhibitor of both PD-L1 and PD-L2). In some embodiments, the tumor-specific promoter is an E2F-1 promoter, such as a human E2F-1 promoter or an E2F-1 promoter comprising the nucleotide sequence set forth in SEQ ID NO:1. In some embodiments, the viral gene essential for replication of the virus is selected from the group consisting of E1A, E1B, and E4.

In some embodiments, there is provided in combination, an infectious agent and immunomodulator for use in a method of treating bladder cancer in an individual, comprising: a) intravesically administering an effective amount of an oncolytic virus (such as oncolytic adenovirus) comprising a viral vector comprising a tumor cell-specific promoter operably linked to a viral gene essential for replication of the virus and a nucleic acid encoding an immune-related molecule (such as cytokine or chemokine) operably linked to a viral promoter; and b) intravesically administering an effective amount of an inhibitor of PD-1 ligand PD-L1 (such as an anti-PD-L1 antibody, or an inhibitor of both PD-L1 and PD-L2). In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the immune-related molecule is GM-CSF.

In some embodiments, there is provided in combination, an infectious agent and immunomodulator for use in a method of treating bladder cancer in an individual, comprising: a) intravesically administering an effective amount of an adenovirus serotype 5, wherein the endogenous E1a promoter and E3 19kD coding region of a native adenovirus is replaced by the human E2F-1 promoter and a nucleic acid encoding an immune-related molecule (such as cytokine or chemokine, for example, GM-CSF); and b) intravesically administering an effective amount of an inhibitor of PD-1 ligand PD-L1 (such as an anti-PD-L1 antibody, or an inhibitor of both PD-L1 and PD-L2). In some embodiments, the tumor-specific promoter is a human E2F-1 promoter or an E2F-1 promoter comprising the nucleotide sequence set forth in SEQ ID NO: 1.

In some embodiments, there is provided in combination, an infectious agent and immunomodulator for use in a method of treating bladder cancer in an individual, comprising: a) intravesically administering an effective amount of CG0070; and b) intravesically administering an effective amount of an inhibitor of PD-1 ligand PD-L1 (such as an anti-PD-L1antibody, or an inhibitor of both PD-L1 and PD-L2). In some embodiments, the inhibitor of PD-1 ligand is an anti-PD-L1 antibody, for example, KY-1003, MCLA-145, RG7446, BMS935559, MPDL3280A, MEDI4736, Avelumab, or STI-A1010. In some embodiments, the inhibitor of PD-1 ligand is an inhibitor (e.g., peptide, protein or small molecule) of both PD-L1 and PD-L2, such as AUR-012, and AMP-224. In some embodiments, the CG007 is administered weekly. In some embodiments, the inhibitor of PD-1 ligand is administered weekly. In some embodiments, the CG0070 and the inhibitor of PD-1 ligand are administered sequentially. In some embodiments, the CG0070 is administered prior to (such as immediately prior to) the administration of the inhibitor of PD-1 ligand. In some embodiments, the CG0070 is administered after (such as immediately after) the administration of the inhibitor of PD-1 ligand. In some embodiments, the CG0070 and the inhibitor of PD-1 ligand are administered simultaneously (for example in a single composition). In some embodiments, the method further comprises administration of CG0070 and/or the inhibitor of PD-1 ligand by an administration route other than intravesical administration.

The intravesical administration of the infectious agent and/or the immunomodulator (including combination of immunomodulators) provide a unique opportunity of a relatively convenient yet effective intravesical tumor exposure to the infectious agent and/or the immunomodulator (including combination of immunomodulators), as well as a potentially reduced toxicity to other tissues. Suitable dosages and dosing frequency of the infectious agents and the immunomodulator (including combination of immunomodulators) are within the same ranges as those described for local administration of the infectious agents and the immunomodulator (including combination of immunomodulators) respectively in the previous section.

In some embodiments, the infectious agent and/or the immunomodulator (including combination of immunomodulators) are administered by instillation as a solution via a catheter. In some embodiments, the total volume of the solution used for the intravesical installation is about any of 1 mL, 10 mL, 50 mL, 75 mL, 100 mL, 125 mL, 150 mL, 200 mL, 250 mL, 300 mL, 400 mL or 500 mL. In some embodiments, the total volume of the solution used for the intravesical installation is any of about 1 mL to about 10 mL, about 10 mL to about 50 mL, about 50 mL to about 75 mL, about 75 mL to about 100 mL, about 100mL to about 125 mL, about 75 mL to about 125 mL, about 100 mL to about 150 mL, about 150 mL to about 200 mL, about 200 mL to about 300 mL, about 300 mL to about 400 mL, about 400 mL to about 500 mL, about 50 mL to about 500 mL, about 50 mL to about 250 mL, or about 100 mL to about 250 mL.

In some embodiments, the infectious agent is administered at a dose of about $1\times10^9$ to about $1\times10^{15}$ particles (such as about $1\times10^{11}$ to about $1\times10^{14}$ particles, for example about$1\times10^{12}$ particles). In some embodiments, the infectious agent is administered at a volume of about 50 to about 500mL (such as about 100 mL) by instillation.

In some embodiments, the immunomodulator (including combination of immunomodulators) is administered at a dose of about 0.1 mg/Kg to about 100 mg/Kg (such as about 0.1 mg/Kg to about 0.3 mg/Kg, about 0.1 mg/Kg to about 0.5 mg/Kg, about 0.5 mg/Kg to about 1 mg/Kg, about 1 mg/Kg to about 10 mg/Kg, about 10 mg/Kg to about 50 mg/Kg, about 50 mg/Kg to about 100 mg/Kg, or about 1 mg/Kg to about 100 mg/Kg). In some embodiments, the immunomodulator (including combination of immunomodulators) is administered at a dose no more than about any of 500 mg, 400 mg, 300 mg, 200 mg, 100 mg, 80 mg, 60 mg, 40 mg, 20 mg, or 10 mg per administration. In some embodiments, the immunomodulator (including combination of immunomodulators) is administered at a volume of about 1 mL to about 500mL (such as about 100 mL) by instillation.

The solution of the infectious agent and/or the immunomodulator (including combination of immunomodulators) may be retained in the bladder for a certain amount of time before voiding, in order to achieve uniform distribution or sufficient exposure of the infectious agent and/or the immunomodulator (including combination of immunomodulators) among the bladder tumor cells. In some embodiments, the solution is retained in the bladder of the individual for at least about any of 5 minutes, 10 minutes, 15 minutes, 20 minutes, 30 minutes, 45 minutes, 1 hour, 2 hours, or more. In some embodiments, the solution is retained in the bladder of the individual for any of about 5 minutes to about10 minutes, about 10 minutes to about 15 minutes, about 10 minutes to about 20 minutes, about 20 minutes to about 30 minutes, about 30 minutes to about 45 minutes, about 45 minutes to about 50 minutes, about 50 minutes to about 1 hour, about 5 minutes to about 15 minutes, about 10 minutes to about 30 minutes, about 30 minutes to about 1 hour, or about 1 hour to about 2 hours. In some embodiments, the infectious agent (such as the oncolytic virus, e.g., CG0070) is retained in the bladder of the individual for about 45 minutes to about 50 minutes. In some embodiments, the immunomodulator (including combination of immunomodulators) is retained in the bladder for about 45 minutes to 1 hour. In some embodiments, the efficiency of the intravesical administration of the infectious agent is further enhanced by a pretreatment comprising intravesical administration of an effective amount of a transduction enhancing agent, such as DDM.

In some embodiments, the pretreatment step is carried out by contacting the luminal surface of the bladder in the individual with the pretreatment composition prior to the administration of the infectious agent and the immunomodulator (including combination of immunomodulators). For example, the pretreatment composition may comprise about 0.01% to about 0.5% (such as 0.05 to about 0.2%, for example about 0.1%) of the transduction enhancing agent (such as DDM). In some embodiments, the total volume of the pretreatment composition (such as DDM) is about 10 mL to about 1000 mL (such as about 10 mL to about 100 mL, about 100 mL to about 500 mL, or about 500 mL to about 1000 mL). In some embodiments, a suitable dosage for the pretreatment composition is about any one of 0.1 g, 0.2 g, 0.5g, 0.75 g, 1 g, 1.5 g, 2 g, 2.5 g, 5 g, or 10 g of the transduction enhancing agent (such as DDM). In some embodiments, the effective amount of the pretreatment composition is about 1g of DDM (e.g., 100 mL of 0.1% DDM solution).

In some embodiments, the pretreatment composition (such as DDM) is administered immediately (such as no more than 5 minutes) prior to the administration of the infectious agent. In some embodiments, the pretreatment composition (such as DDM) is administered no more than about any of 5 minutes, 10 minutes, 15 minutes, 20 minutes, 30 minutes, 45 minutes, 1 hour, 90 minutes, 2 hours, 3 hours or 4 hours before the administration of the infectious agent. In some embodiments, the pretreatment composition (such as DDM) is administered no more than about 2 hours before the administration of the infectious agent. In some embodiments, the pretreatment composition (such as DDM solution) is retained in the bladder for at least about any one of 5 minutes, 10 minutes, 15 minutes, or 20 minutes. In some embodiments, the pretreatment composition (such as DDM solution) is retained in the bladder for any of about 5 minutes to about 10 minutes, about 10 minutes to about 15 minutes, about 12 minutes to about 15 minutes, about 15 minutes to about 20 minutes, or about 10 minutes to about 20 minutes. In some embodiments, the pretreatment composition (such as DDM solution) is retained in the bladder for about 12 minutes to about 15 minutes.

In some embodiments, the pretreatment step is carried out by contacting the luminal surface of the bladder in the individual with the pretreatment composition prior to the administration of the infectious agent and the immunomodulator (including combination of immunomodulators).

In some embodiments, the method further comprises washing the luminal surface of the bladder contact with the pretreatment composition In some embodiments, the method further comprises washing the luminal surface of the bladder after contacting the bladder with the pretreatment composition prior to the administration of the infectious agent.

In some embodiments, the pretreatment step comprises one or more tumor site preparation steps as described in the "Methods of treating a solid or lymphatic tumor" section.

In some embodiments, the pretreatment comprises intravesical administration of an effective amount of an immune-related molecule (such as cytokine, chemokine or PRRago). In some embodiments, the immune-related molecule is selected from the group consisting of GM-CSF, IL-2, IL12, interferon (such as Type 1, Type 2 or Type 3 interferon, e.g., interferonγ), CCL4, CCL19, CCL21, CXCL13, TLR1, TLR2, TLR3, TLR4, TLR5, TLR6, TLR7, TLR8, TLR9, TLR10, RIG-I, MDA5, LGP2, LTαβ, STING activators (such as CDN), PRRago (such as CpG, Imiquimod, or Poly I:C), TLR stimulators (such as GS-9620, AED-1419, CYT-003-QbG10, AVE-0675, or PF-7909), and RLR stimulators (such as RIG-I, Mda5, or LGP2 stimulators). In some embodiments, the immune-related molecule is administered directly in its native format. In some embodiments, the immune-related molecule is administered in a format that would include an excipient or any compound known to the art that can delay its metabolism, release and/or decay within the tumor site. In some embodiments, the immune-related molecule can be combined with one or more additional immune-related molecules. In some embodiments, the immune-related molecules of two or more in combinations are administered in a format that would include an excipient or any compound known to the art that can affect its metabolism, release and/or decay within the tumor site. In some embodiments, the immune-related molecule induces dendritic cells, T cells, B cells, and/or T follicular helper cells. In some embodiments, the immune-related molecule is administered separately from the infectious agent (e.g., in a separate composition or as a separate entity in the same composition). In some embodiments, the immune-related molecule is administered to the site of the tumor via transduction. Exemplary transduction methods known in the art include, but are not limited to, the use of calcium phosphate, dendrimers, liposomes, cationic polymers, electroporation, cell squeezing, sonoporation, optical transfection, protoplast fusion, impalefection, hydrodynamic delivery, gene gun, magnetofection, viral transfection and nucleofection. In some embodiments, the immune-related molecule is expressed by the infectious agent. For example, the infectious agent may comprise a nucleic acid encoding the immune-related molecule, and the nucleic acid can be in the viral vector or on a separate vector. In some embodiments, the infectious agent is a virus comprising a viral vector, and wherein the viral vector comprises the nucleic acid encoding the immune-related molecule. In some embodiments, the nucleic acid encoding the immune-related molecule is operably linked to a viral promoter, such as an E1 promoter, or an E3 promoter.

In some embodiments, the pretreatment step comprises administering an effective amount of radiation therapy to the bladder of the individual prior to the administration of the infectious agent and the immunomodulator (including combination of immunomodulators). In some embodiments, the radiation therapy is in combination with chemotherapy. In some embodiments, the radiation therapy is administered without chemotherapy. In some embodiments, the radiation therapy comprises irradiation to the whole body. In some embodiments, the radiation therapy is irradiation to only tumor sites. In some embodiments, the radiation therapy is irradiation to tissues having the tumor. In some embodiments, the radiation therapy is irradiation to only the site of the tumor selected for local administration of the infectious agent and the immunomodulator. In some embodiments, the radiation therapy is irradiation to only a tissue having the tumor selected for local administration of the infectious agent and the immunomodulator. In some embodiments, the dose of the radiation therapy is insufficient to eradicate the tumor cells. For example, a suitable dosage of the radiation therapy is about any one of 1 Gy, 5 Gy, 10 Gy, 15 Gy, 20 Gy, 25 Gy, 30 Gy, 35 Gy, 40 Gy, 45 Gy, 50 Gy, 55 Gy, 60 Gy, 65 Gy, 70 Gy, 75 Gy, 80 Gy, 90 Gy or 100 Gy. In some embodiments, the dose of the radiation therapy is no more than about any one of 1 Gy, 5 Gy, 10 Gy, 15 Gy, 20 Gy, 25 Gy, 30 Gy, 35 Gy, 40 Gy, 45 Gy, 50 Gy, 55 Gy, 60 Gy, 65 Gy, 70 Gy, 75 Gy, 80 Gy, 90 Gy or 100 Gy. In some embodiments, the dose of the radiation therapy is any one of about 1 Gy to about 5 Gy, about 5 Gy to about 10 Gy, about 10 Gy to about 15 Gy, about 15 Gy to about 20 Gy, about 20 Gy to about 25 Gy, about 25 Gy to about 30 Gy, about 30 Gy to about 35 Gy, about 5 Gy to about 15 Gy, about 10 Gy to about 20 Gy, about 20 Gy to about 30 Gy, about 30 Gy to about 40 Gy, about 40 Gy to about 50 Gy, about 50 Gy to about 60 Gy, about 60 Gy to about 70 Gy, about 70 Gy to about 80 Gy, about 80 Gy to about 100 Gy, about 10 Gy to about 30 Gy, about 20 Gy to about 40 Gy, about 1Gy to about 25 Gy, about 25 Gy to about 50 Gy, about 30 Gy to about 60 Gy, about 60 Gy to about 80 Gy, or about 10 Gy to about 60 Gy. In some embodiments, the radiation therapy is administered in more than one fraction, such as about any one of 2, 3, 4, 5, 6, 7, 8, 9, 10, 12, 15, 16, 18, 20 or more fractions. In some embodiments, the radiation therapy fractions are administered over the course of about any one of 1 day, 2 days, 3 days, 4 days, 5 days, 6 days, 1 week, 2 weeks, 3 weeks, 4 weeks, 5 weeks, 6 weeks, 7 weeks or more. In some embodiments, the radiation therapy fractions are administered over the course of any one of about 1 day to about 5 days, about 1 week to about 2 weeks, about 2 weeks to about 3 weeks, about 3 weeks to about 4 weeks, about 4 weeks to about 5 weeks, about 5 weeks to about 6 weeks, about 6 weeks to about 7 weeks, about 2 weeks to about 4 weeks, about 4 weeks to about 6 weeks, or about 1 week to about 6 weeks. In some embodiments, the radiation therapy is administered about two fractions per day. In some embodiments, each fraction of the radiation therapy is about 1.8 Gy to about 2 Gy per day, five days a week, for an adult, or about 1.5 Gy to about 1.8 Gy per day, five days a week for a child. In some embodiments, each fraction of the radiation therapy is about any one of 1 Gy, 1.5 Gy, 2 Gy, 2.5 Gy, 5 Gy, 10 Gy, 15 Gy, 20 Gy, 30 Gy, 40 Gy, 50 Gy or more. In some embodiments, each fraction of the radiation therapy is any one of about 1 Gy to about 1.5 Gy, about 1.5 Gy to about 2 Gy, about 1 Gy to about 2.5 Gy, about 2.5 Gy to about 5 Gy, about 5 Gy to about 10 Gy, about 10 Gy to about 15 Gy, about 15 Gy to about 20 Gy, about 20 Gy to about 30 Gy, about 25 Gy to about 50 Gy, about 1 Gy to about 10 Gy, or about 2 Gy to about 20 Gy.

In some embodiments, the radiation therapy is administered in a single fraction. In some embodiments, the radiation therapy is aim at lymphodepletion, either as a single dose fraction per day or in multiple fractions over days to weeks. In some embodiments, the lymphodepletion radiation therapy is given as a total body irradiation. In some embodiments, the lymphodepletion is only given to local tumor sites, or to tissues with the tumor. In some embodiments, the lymphodepletion radiation therapy is administered two fractions per day. In some embodiments, each fraction of the lymphodepletion radiation therapy is about 1 Gy to about 2 Gy per day, five days a week, for an adult, or about 0.5 Gy to about 1.8 Gy per day, five days a week for a child. In some embodiments, each fraction of the radiation therapy is about any one of 1 Gy, 1.5 Gy, 2 Gy, 2.5 Gy, 5 Gy, 10 Gy, 15 Gy, 20 Gy, 30 Gy, 40 Gy, 50 Gy or more. In some embodiments, each fraction of the radiation therapy is any one of about 1 Gy to about 1.5 Gy, about 1.5 Gy to about 2 Gy, about 1 Gy to about 2.5 Gy, about 2.5 Gy to about 5 Gy, about 5 Gy to about 10 Gy, about 10 Gy to about 15 Gy, about 15 Gy to about 20 Gy, about 20 Gy to about 30 Gy, about 25 Gy to about 50 Gy, about 1 Gy to about 10 Gy, or about 2 Gy to about 20 Gy. In some embodiments, lymphodepletion radiation therapy is administered with or without the use of a chemotherapeutic agent, such as but not limited to, cyclophosphamide and fludarabine.

Any of the known methods of radiation therapy may be used in the present invention, including, but not limited to external beam radiation therapy (EBRT or XRT), tele therapy, brachytherapy, sealed source radiation therapy, systemic radioisotope therapy (RIT), unsealed source radiation therapy, intraoperative radiation therapy (IORT), targeted intraoperative radiation therapy (TARGIT), intensity-modulated radiation therapy (IMRT), volumetric modulated arc therapy (VMAT), particle therapy, and auger therapy.

In some embodiments, the pretreatment step comprises administrating directly or indirectly (e.g. through an intravenous route) to the luminal surface of the bladder in the individual an effective amount of a therapeutic agent prior to the administration of the infectious agent and the immunomodulator (including combination of immunomodulators). In some embodiments, the therapeutic agent is any one or combination of chemotherapeutic agents known in the art, for example, cyclosphamide. In some embodiments, the therapeutic agent is any one or combination of agents targeting or blocking a cellular signaling pathway known in the art, for example, a BRAF inhibitor. In some embodiments, the therapeutic agent is any one or combination of cell therapies known in the art, for example, TIL cells, CAR/T cells, and/or TCR/T cells. In some embodiments, the therapeutic agent is an agent that increases the level of cytokines involved an immunogenic pathway. Any of the immune-related molecules described herein may be used as the therapeutic agent, including, but are not limited to, cytokines such as IL6, IL8 and IL18 (these cytokines can either have pro and/or anti-inflammatory actions, or some may promote new blood vessels formation and tumor growth), chemokines (such as CCL21 that can promote tumor spread by increase of lymphatic structures), growth factors (such as FLT3L), heat shock proteins, small molecule kinase inhibitors (such as JAK2 inhibitor), and IAP inhibitors. In some embodiments, the therapeutic agent is an agent that causes dysfunction or damage to a structural component of a tumor. Exemplary agents include, but are not limited to, anti-VEGF antibody, a hyaluronidase, and n-dodecyl-β-maltoside. In some embodiments, the therapeutic agent induces immune cells, such as dendritic cells, B cells, and T cells (such as follicular T helper cells).

**Combination therapy with tumor cells**

One aspect of the present application provides in combination, an infectious agent and immunomodulator for use in a method of treating a solid or lymphatic tumor in an individual (such as a human), comprising: a) locally administering to the site of the tumor an effective amount of an infectious agent wherein the infectious agent is an oncolytic virus; b) locally administering to the site of the tumor an effective amount of an immunomodulator (including combination of immunomodulators) wherein the immunomodulator is an inhibitor of PD-L1; and c) locally administering to the site of the tumor an effective amount of inactivated tumor cells. This at least three-component combination therapy method may comprise any embodiment of the methods described above for the combination therapy comprising the infectious agent and the immunomodulator (including combination of immunomodulators). The present combination therapy method comprising the inactivated tumor cells is advantageous over other cancer immunotherapy methods involving similar components, because administration parameters, such as dosage, dosing frequency and/or route of administration, for each of the three components, namely, the infectious agent (such as oncolytic virus, for example, oncolytic adenovirus), the immunomodulator (including combination of immunomodulators), and the inactivated tumor cells can be independently adjusted to optimize the efficacy and minimize the toxicity of the therapy to the individual. Any of the methods described herein may be useful for inhibiting growth of a solid or lymphatic tumor, inhibiting metastasis of a solid or lymphatic tumor, prolonging survival (such as disease-free survival) of an individual having a solid or lymphatic tumor, causing disease remission in an individual having a solid or lymphatic tumor, and/or improving quality of life of an individual having a solid or lymphatic tumor.

Without being bound by any theory or hypothesis, it is believed that in this three-component combination therapy, an outside source of inactivated but live tumor cells (also referred herein as "live cancer cells" or "live tumor cells"), whether they are autologous or allogeneic in origin, could provide an additional, yet important source of new antigens when administered at the site of the tumor. Outside source in this context means that these tumor cells have already been removed previously, from the same individual or from another individual. The cells may have further been subjected to in vitro culture for expansion, cryopreservation, thawing and characterization. It is believed that this outside source of inactivated tumor cells can sometimes stimulate not only a T cell response, but may also solicit a B cell, and sometimes trigger a massive antibody response that is synergistic with the infectious agent (such as virus), and the immunomodulator (including combination of immunomodulators) as described previously.

Thus, in some embodiments, there is provided in combination, an infectious agent and immunomodulator for use in a method of treating a solid or lymphatic tumor in an individual, comprising: a) locally administering to the site of the tumor an effective amount of an infectious agent wherein the infectious agent is an oncolytic virus; b) locally administering to the site of the tumor an effective amount of an immunomodulator (including combination of immunomodulators) , wherein the immunomodulator is an inhibitor of PD-L1; and c) locally administering to the site of the tumor an effective amount of inactivated tumor cells. In some embodiments, the infectious agent is a virus, such as a virus selected from the group consisting of adenovirus, herpes simplex virus, vaccinia virus, mumps virus, newcastle disease virus, polio virus, measles virus, Seneca valley virus, coxsackie virus, reo virus, vesicular stomatitis virus, maraba and rhabdovirus, and parvovirus. In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the immunomodulator is an immune checkpoint inhibitor. In some embodiments, the immunomodulator is a modulator of the immune checkpoint molecule PD-L1In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the inactivated tumor cells are autologous. In some embodiments, the inactivated tumor cells are allogenic. In some embodiments, the inactivated tumor cells are from a tumor cell line. In some embodiments, the inactivated tumor cells are inactivated by irradiation. In some embodiments, the infectious agent and the inactivated tumor cells are administered simultaneously (for example, in a single composition). In some embodiments, the infectious agent and the inactivated tumor cells are admixed immediately prior to the administration. In some embodiments, the infectious agent and the inactivated tumor cells are administered in sequentially. In some embodiments, the infectious agent and the inactivated tumor cells are admixed at the administration site immediately after the administration. In some embodiments, the infectious agent, the immunomodulator (including combination of immunomodulators), and/or the inactivated tumor cells are administered to the tissue having the tumor. In some embodiments, the infectious agent, the immunomodulator (including combination of immunomodulators), and/or the inactivated tumor cells are administered directly into the tumor. In some embodiments, the method further comprises administration of the infectious agent and/or the immunomodulator (including combination of immunomodulators) and/or the inactivated tumor cells by an administration route other than local administration.

In some embodiments, there is provided in combination, an infectious agent and immunomodulator for use in a method of treating a solid or lymphatic tumor in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus; b) locally administering to the site of the tumor an effective amount of an immunomodulator (including combination of immunomodulators), wherein the immunomodulator is an inhibitor of PD-L1; and c) locally administering to the site of the tumor an effective amount of inactivated tumor cells. In some embodiments, the immunomodulator is an immune checkpoint inhibitor. In some embodiments, the immunomodulator is a modulator of the immune checkpoint molecule PD-L1In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the tumor-specific promoter is an E2F-1 promoter, such as a human E2F-1 promoter or an E2F-1 promoter comprising the nucleotide sequence set forth in SEQ ID NO:1. In some embodiments, the viral gene essential for replication of the virus is selected from the group consisting of E1A, E1B, and E4. In some embodiments, the inactivated tumor cells are autologous. In some embodiments, the inactivated tumor cells are allogenic. In some embodiments, the inactivated tumor cells are from a tumor cell line. In some embodiments, the inactivated tumor cells are inactivated by irradiation. In some embodiments, the oncolytic virus and the inactivated tumor cells are administered simultaneously (for example, in a single composition). In some embodiments, the oncolytic virus and the inactivated tumor cells are admixed immediately prior to the administration. In some embodiments, the oncolytic virus and the inactivated tumor cells are administered in sequentially. In some embodiments, the oncolytic virus and the inactivated tumor cells are admixed at the administration site immediately after the administration. In some embodiments, the oncolytic virus, the immunomodulator (including combination of immunomodulators), and/or the inactivated tumor cells are administered to the tissue having the tumor. In some embodiments, the method further comprises administration of the oncolytic virus and/or the immunomodulator (including combination of immunomodulators) and/or the inactivated tumor cells by an administration route other than local administration.

In some embodiments, there is provided in combination, an infectious agent and immunomodulator for use in a method of treating a solid or lymphatic tumor in an individual, comprising: a) locally administering to the site of the tumor an effective amount of an oncolytic virus (such as oncolytic adenovirus) comprising a viral vector comprising a tumor cell-specific promoter operably linked to a viral gene essential for replication of the virus and a nucleic acid encoding an immune-related molecule (such as cytokine or chemokine) operably linked to a viral promoter; b) locally administering to the site of the tumor an effective amount of an immunomodulator (including combination of immunomodulators), wherein the immunomodulator is an inhibitor of PD-L1; and c) locally administering to the site of the tumor an effective amount of inactivated tumor cells, wherein the inactivated tumor cells are inactivated. In some embodiments, the immunomodulator is an immune checkpoint inhibitor. In some embodiments, the immunomodulator is a modulator of the immune checkpoint molecule PD-L1. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the tumor-specific promoter is an E2F-1 promoter, such as a human E2F-1 promoter or an E2F-1 promoter comprising the nucleotide sequence set forth in SEQ ID NO:1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the immune-related molecule is GM-CSF. In some embodiments, the inactivated tumor cells are autologous. In some embodiments, the inactivated tumor cells are allogenic. In some embodiments, the inactivated tumor cells are from a tumor cell line. In some embodiments, the inactivated tumor cells are inactivated by irradiation. In some embodiments, the oncolytic virus and the inactivated tumor cells are administered simultaneously (for example, in a single composition). In some embodiments, the oncolytic virus and the inactivated tumor cells are admixed immediately prior to the administration. In some embodiments, the oncolytic virus and the inactivated tumor cells are administered in sequentially. In some embodiments, the oncolytic virus and the inactivated tumor cells are admixed at the administration site immediately after the administration. In some embodiments, the oncolytic virus, the immunomodulator (including combination of immunomodulators), and/or the inactivated tumor cells are administered to the tissue having the tumor. In some embodiments, the oncolytic virus, the immunomodulator (including combination of immunomodulators), and/or the inactivated tumor cells are administered directly into the tumor. In some embodiments, the method further comprises administration of the oncolytic virus and/or the immunomodulator (including combination of immunomodulators) and/or the inactivated tumor cells by an administration route other than local administration.

In some embodiments, there is provided in combination, an infectious agent and immunomodulator for use in a method of treating a solid or lymphatic tumor in an individual, comprising: a) locally administering to the site of the tumor an effective amount of an adenovirus serotype 5, wherein the endogenous E1a promoter and E3 19kD coding region of a native adenovirus is replaced by the human E2F-1 promoter and a nucleic acid encoding an immune-related molecule (such as cytokine, chemokine, for example, GM-CSF); b) locally administering to the site of the tumor an effective amount of an immunomodulator (including combination of immunomodulators), wherein the immunomodulator is an inhibitor of PD-L1; and c) locally administering to the site of the tumor an effective amount of inactivated tumor cells. In some embodiments, the immunomodulator is an immune checkpoint inhibitor. In some embodiments, the immunomodulator is a modulator of the immune checkpoint molecule PD-L1. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the tumor-specific promoter is a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the inactivated tumor cells are autologous. In some embodiments, the inactivated tumor cells are allogenic. In some embodiments, the inactivated tumor cells are from a tumor cell line. In some embodiments, the inactivated tumor cells are inactivated by irradiation. In some embodiments, the adenovirus and the inactivated tumor cells are administered simultaneously (for example, in a single composition). In some embodiments, the adenovirus and the inactivated tumor cells are admixed immediately prior to the administration. In some embodiments, the adenovirus and the inactivated tumor cells are administered in sequentially. In some embodiments, the adenovirus and the inactivated tumor cells are admixed at the administration site immediately after the administration. In some embodiments, the adenovirus, the immunomodulator (including combination of immunomodulators), and/or the inactivated tumor cells are administered to the tissue having the tumor. In some embodiments, the adenovirus, the immunomodulator (including combination of immunomodulators), and/or the inactivated tumor cells are administered directly into the tumor. In some embodiments, the method further comprises administration of the adenovirus and/or the immunomodulator (including combination of immunomodulators) and/or the inactivated tumor cells by an administration route other than local administration.

In some embodiments, there is provided in combination, an infectious agent and immunomodulator for use in a method of treating a solid or lymphatic tumor in an individual, comprising: a) locally administering to the site of the tumor an effective amount of CG0070; and b) locally administering to the site of the tumor an effective amount of an immunomodulator (including combination of immunomodulators), wherein the immunomodulator is an inhibitor of PD-L1; and c) locally administering to the site of the tumor an effective amount of inactivated tumor cells. In some embodiments, the immunomodulator is an immune checkpoint inhibitor. In some embodiments, the immunomodulator is a modulator of the immune checkpoint molecule PD-L1. In some embodiments, the method comprises local administration of a combination of immunomodulators comprising one or more immune checkpoint inhibitors and/or one or more immune-stimulating agents (such as at least two immune checkpoint inhibitors, at least two immune-stimulating agents, or a combination of at least one immune checkpoint inhibitor and at least one immune-stimulating agent). In some embodiments, the inactivated tumor cells are autologous. In some embodiments, the inactivated tumor cells are allogenic. In some embodiments, the inactivated tumor cells are from a tumor cell line. In some embodiments, the inactivated tumor cells are inactivated by irradiation. In some embodiments, CG0070 and the inactivated tumor cells are administered simultaneously (for example, in a single composition). In some embodiments, CG0070 and the inactivated tumor cells are admixed immediately prior to the administration. In some embodiments, CG0070 and the inactivated tumor cells are administered in sequentially. In some embodiments, CG0070 and the inactivated tumor cells are admixed at the administration site immediately after the administration. In some embodiments, CG0070, the immunomodulator (including combination of immunomodulators), and/or the inactivated tumor cells are administered to the tissue having the solid or lymphatic tumor. In some embodiments, CG0070, the immunomodulator (including combination of immunomodulators), and/or the inactivated tumor cells are administered directly into the tumor. In some embodiments, the method further comprises administration of CG0070 and/or the immunomodulator (including combination of immunomodulators) and/or the inactivated tumor cells by an administration route other than local administration.

The inactivated tumor cells may be obtained from a variety of sources, including, but not limited to, autologous source, allogenic source, a tumor cell line and combinations thereof. Typically, the inactivated tumor cells are of the same type, or express one or more of the same tumor antigens and the solid or lymphatic tumor being treated. In some embodiments, the inactivated tumor cells consist of a single population of tumor cells. In some embodiments, the inactivated tumor cells comprise a plurality (such as 2, 3, 4, 5, 6, or more) of population of tumor cells.

In some embodiments, the inactivated tumor cells are derived from an allogenic source. In some embodiments, the inactivated tumor cells are derived from a different individual having a tumor (such as solid or lymphatic tumor of the same type). In some embodiments, the inactivated tumor cells and the solid or lymphatic tumor of the individual being treated express at least one common tumor antigen (such as tumor associated antigen and/or tumor specific antigen).

In some embodiments, the inactivated tumor cells are derived from a tumor cell line sharing the same or similar origin or genetic profile (such as tumor antigen expression profile) as the solid or lymphatic tumor of the individual. In some embodiments, the inactivated tumor cells and the individual having a tumor express at least one common tumor antigen (such as tumor associated antigen and/or tumor specific antigen). For example, when the solid or lymphatic tumor being treated is prostate cancer, the prostate tumor cell line may be selected from the group consisting of DU145, PC-3, and LnCaP.

In some embodiments, the inactivated tumor cells are derived from the same individual having the solid or lymphatic tumor. In some embodiments, the inactivated tumor cells are derived from the tissue having the solid or lymphatic tumor. In some embodiments, the inactivated tumor cells are derived from the solid or lymphatic tumor (e.g., from tumor biopsy or a resected tumor). In some embodiments, the inactivated tumor cells are derived from a metastatic site of the solid or lymphatic tumor from the individual. In some embodiments, the inactivated tumor cells provide one or more cellular, cytokine, chemokine, and/or antigenic components during death of the inactivated tumor cells in vivo, wherein the one or more components is sampled and cross-presented by the antigen presenting cells (such as dendritic cells) of the individual to stimulate an immune response against the solid or lymphatic tumor.

In some embodiments, the inactivated tumor cells are modified, such as genetically modified, for example, via transduction by an infectious agent harboring a vector encoding a transgene. The inactivated tumor cells may be transduced or transfected by the infectious agent in vitro, or in vivo. In some embodiments, the inactivated tumor cells are modified to express or secrete an immune-related molecule. In some embodiments, the immune-related molecule is a cytokine, a chemokine, or another immune-related molecule. In some embodiments, the immune-related molecule is selected from the group consisting of IL-2, IL-12, interferon (such as Type 1, Type 2 or Type 3 interferon, e.g., interferon $\gamma$), CCL4, CCL19, CCL21, CXCL13, TLR1, TLR2, TLR3, TLR4, TLR5, TLR6, TLR7, TLR8, TLR9, TLR10, RIG-I, MDA5, LGP2, and $LT\alpha\beta$. In some embodiments, the immune-related molecule is selected from the group consisting of STING activators (such as CDN), PRRago (such as CpG, Imiquimod, or Poly I:C), TLR stimulators (such as GS-9620, AED-1419, CYT-003-QbG10, AVE-0675, or PF-7909), and RLR stimulators (such as RIG-I, Mda5, or LGP2 stimulators).

In some embodiments, the inactivated tumor cells are modified to express or secrete one or more immunomodulators. In some embodiments, the one or more immunomodulators comprise an immune-stimulating agent. In some embodiments, the immune-stimulating agent is a natural or engineered ligand of an immune stimulatory molecule, including, for example, ligands of OX40 (e.g., OX40L), ligands of CD-28 (e.g., CD80, CD86), ligands of ICOS (e.g., B7RP1), ligands of 4-1BB (e.g., 4-1BBL, Ultra4-1BBL), ligands of CD27 (e.g., CD70), ligands of CD40 (e.g., CD40L), and ligands of TCR (e.g., MHC class I or class II molecules, IMCgp100). In some embodiments, the immune-stimulating agent is an antibody selected from the group consisting of anti-CD28 (e.g., TGN-1412), anti-OX40 (e.g., MEDI6469, MEDI-0562), anti-ICOS (e.g., MEDI-570), anti-GITR (e.g., TRX518, INBRX-110, NOV-120301), anti-41-BB (e.g., BMS-663513, PF-05082566), anti-CD27 (e.g., BION-1402, Varlilumab and hCD27.15), anti-CD40 (e.g., CP870,893, BI-655064, BMS-986090, APX005, APX005M), anti-CD3 (e.g., blinatumomab, muromonab), and anti-HVEM. In some embodiments, the antibody is an agonistic antibody. In some embodiments, the antibody is a monoclonal antibody. In some embodiments, the antibody is an antigen-binding fragment selected from the group consisting of Fab, Fab', $F(ab')_2$, and Fv, scFv, or other antigen-binding subsequences of the full length antibody. In some embodiments, the antibody is a human, humanized, or chimeric antibody. In some embodiments, the antibody is a bispecific antibody, a multispecific antibody, a single domain antibody, a fusion protein comprising an antibody portion, or any other functional variants or derivatives thereof.

In some embodiments, the one or more immunomodulators comprise an immune checkpoint inhibitor. In some embodiments, the immune-checkpoint inhibitor is a natural or engineered ligand of an inhibitory immune checkpoint molecule, including, for example, ligands of CTLA-4 (e.g., B7.1, B7.2), ligands of TIM3 (e.g., Galectin-9), ligands of A2a Receptor (e.g., adenosine, Regadenoson), ligands of LAG3 (e.g., MHC class I or MHC class II molecules), ligands of BTLA (e.g., HVEM, B7-H4), ligands of KIR (e.g., MHC class I or MHC class II molecules), ligands of PD-1 (e.g., PD-L1, PD-L2), ligands of IDO (e.g., NKTR-218, Indoximod, NLG919), and ligands of CD47 (e.g., SIRP-alpha receptor). In some embodiments, the immune checkpoint inhibitor is an antibody that targets an inhibitory immune checkpoint protein. In some embodiments, the immunomodulator is an antibody selected from the group consisting of anti-CTLA-4 (e.g., Ipilimumab, Tremelimumab, KAHR-102), anti-TIM3 (e.g., F38-2E2, ENUM005), anti-LAG3 (e.g., BMS-986016, IMP701, IMP321, C9B7W), anti-KIR (e.g., Lirilumab and IPH2101), anti-PD-1 (e.g., Nivolumab, Pidilizumab, Pembrolizumab, BMS-936559, atezolizumab, Lambrolizumab, MK-3475, AMP-224, AMP-514, STI-A1110, TSR-042), anti-PD-L1 (e.g., KY-1003 (EP20120194977), MCLA-145, RG7446, BMS-936559, MEDI-4736, MSB0010718C, AUR-012, STI-A1010, PCT/US2001/020964, MPDL3280A, AMP-224, Dapirolizumab pegol (CDP-7657), MEDI-4920), anti-CD73 (e.g., AR-42 (OSU-HDAC42,HDAC-42,AR42,AR 42,OSU-HDAC 42,OSU-HDAC-42,NSC D736012,HDAC-42,HDAC 42,HDAC42,NSCD736012,NSC-D736012), MEDI-9447), anti-B7-H3 (e.g., MGA271, DS-5573a, 8H9), anti-CD47 (e.g., CC-90002, TTI-621, VLST-007), anti-BTLA, anti-VISTA, anti-A2aR, anti-B7-1, anti-B7-H4, anti-CD52 (such as alemtuzumab), anti-IL-10, anti-IL-35, and anti-TGF-$\beta$ (such as Fresolumimab). In some embodiments, the antibody is an antagonistic antibody. In some embodiments, the antibody is a monoclonal antibody. In some embodiments, the antibody is a monoclonal antibody. In some embodiments, the antibody is an antigen-binding fragment selected from the group consisting of Fab, Fab', $F(ab')_2$, and Fv, scFv, or other antigen-binding subsequences of the full length antibody. In some embodiments, the antibody is a human, humanized, or chimeric antibody. In some embodiments, the antibody is a bispecific antibody, a multispecific antibody, a single domain antibody, a fusion protein comprising an antibody portion, or any other functional variants or derivatives thereof.

In some embodiments, the inactivated tumor cells are transduced and genetically modified by the infectious agent used in the combination therapy.

Tumor cells may be isolated from a tissue, a resected tumor, or a tumor biopsy by any of the methods known in the art, including, but not limited to mechanical, enzymatic separation methods, and combinations thereof. For example, a mixture of collagenase, DNase and hyaluronidase can be used to incubate tumor specimen to obtain the inactivated tumor cells. In some embodiments, multiple batches of isolated autologous tumor cells are obtained from the solid or lymphatic tumor or metastatic sites of the individual during the course of treatment. In some embodiments, the inactivated tumor cells are cryopreserved prior to inactivation.

Since cancer cells, particular in metastatic sites, are heterogeneous mixtures of different clones of cells undergoing rapid replications and frequent mutations, it is sometimes preferable to have a specific component that may adapt to these changes while or when they do occur. Autologous tumor cells can be prepared from the original surgical specimen, biopsies or from removal of metastatic lesions later on. One of the advantages of this method is that the autologous tumor cells can be changed according to the patient's response and the availability of tumor samples. For example, a tumor- infectious agent (e.g. virus) live and *in vivo* vaccine system generated in the primary tumor phase may be different from the one generated later on, using tumor cells from metastatic sites. The ultimate goal, in some embodiments, is to adapt the immunotherapeutic response according to the prevailing tumor types, an advantage that cannot be found in recent development of pathway- targeted therapy or monoclonal antibody-directed therapy.

The inactivated tumor cells are inactivated prior to the administration. Typically, the inactivated tumor cells are proliferation incompetent. Tumor cells can be inactivated with any of the known method in the art. In some embodiments, the inactivated tumor cells are inactivated by irradiation. In some embodiments, the inactivated tumor cells are irradiated at a dose of from about 50 to about 200 rads/min, or from about 120 to about 140 rads/min prior to administration to the patient. In some embodiments, the inactivated tumor cells are irradiated with a total dose of about any one of 2,500 rads, 5,000 rads, 10,000 rads, 15,000 rads or 20,000 rads. In some embodiments, the inactivated tumor cells are irradiated with a total dose of from about 10,000 to about 20,000 rads. In some embodiments, the inactivated tumor cells are irradiated with a total dose sufficient to inhibit substantially 100% of the cells, from further proliferation. In some embodiments, wherein the inactivated tumor cells are genetically modified, the total dose of irradiation is insufficient to inhibit expression or secretion of the immune-related molecule, such as GM-CSF. In some embodiments, the total dose of irradiation is insufficient to inhibit transduction or genetic modification of the inactivated tumor cells by the infectious agent upon administration. In some embodiments, the inactivated tumor cells are cryopreserved prior to the administration.

The inactivated tumor cells are administered intratumorally, for example, by intratumoral injection. Suitable dosage of the inactivated tumor cells for administration depends on the status (e.g., microenvironment, type, stage etc.) of the solid or lymphatic tumor and other diagnostic and risk factors of the individual. In some embodiments, a suitable dosage of the inactivated tumor cells is about any one of $1\times10^3$, $1\times10^4$, $1\times10^5$, $2\times 10^5$, $5 \times 10^5$, $1\times10^6$, $2\times10^6$, $5\times10^6$, $1\times10^7$, $5 \times10^7$, or $1\times 10^8$ cells. In some embodiments, a suitable dosage of the inactivated tumor cells is any one of about $1\times10^3$ to about $1\times10^4$, about $1\times10^4$ to about $1\times10^5$, about $1\times10^5$ to about $2\times 10^5$, about $2\times 10^5$ to about $5 \times10^5$, about $5 \times10^5$ to about $10^6$, about $10^6$ to about $2\times10^6$, about $2\times10^6$ to about $5\times10^6$, about $5\times10^6$ to about 1x10', about $1\times10^7$ to about 5x $10^7$, or about 5x $10^7$ to about $1 \times10^8$ tumor cells. In some embodiments, the dosage of the inactivated tumor cells is calculated as cells/Kg of body weight.

In some embodiments, the relative ratio of the infectious agent (such as virus) to the inactivated tumor cells is based on the multiplicity of infection (MOI) index calculated using the number of infectious agent particles to the number of the inactivated tumor cells alone or to the total number of live tumor cells including the inactivated tumor cells and the estimated number of live tumor cells at the administration site. In some embodiments, the MOI is at least about any one of 1, 2, 5, 10, 50, 100, 200, 500, 1000, 5000, $10^4$, $10^5$, $10^6$, or more. In some embodiments, the infectious agent is provided in an amount proportional to the volume of the estimated tumor sites. In some embodiments, the inactivated tumor cells are provided in an amount limited by preparations from tumor biopsy, tumor resection, tumor cell culture and other methods for isolating tumor cells known to the art. In some embodiments, the infectious agent is provided in the composition at about $1\times10^5$ particles to about $1\times10^{14}$ particles (for example, about $1\times10^{12}$ particles). In some embodiments, the inactivated tumor cells are provided in the composition at about $1\times10^3$ cells to about $1\times10^8$ cells (for example, about $1\times10^5$ inactivated tumor cells).

In some embodiments, the inactivated tumor cells are administered daily. In some embodiments, the inactivated tumor cells are at least about any one of 1x, 2x, 3x, 4x, 5x, 6x, or 7x (i.e., daily) a week. In some embodiments, the inactivated tumor cells are administered weekly. In some embodiments, the inactivated tumor cells are administered biweekly; weekly without break; weekly, two out of three weeks; weekly three out of four weeks; once every two weeks; once every 3 weeks; once every 4 weeks; once every 6 weeks; once every 8 weeks, monthly, or every two to 12 months. In some embodiments, the intervals between each administration are less than about any one of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any one of 1 month, 2 months, 3 months, 4 months, 5 months, 6 months, 8 months, or 12 months. In some embodiments, there is no break in the dosing schedule. In some embodiments, the interval between each administration is no more than about a week. In some embodiments, the inactivated tumor cells are administered with the same dosing schedule as the infectious agent. In some embodiments, the inactivated tumor cells are administered with a different dosing schedule as the infectious agent.

The administration of the inactivated tumor cells can be over an extended period of time, such as from about a month up to about seven years. In some embodiments, the inactivated tumor cells are administered over a period of at least about any one of 1, 2, 3, 4, 5, 6, 7, 8, 9, 10, 11, 12, 18, 24, 30, 36, 48, 60, 72, or 84 months. In some embodiments, the inactivated tumor cells are administered over a period of at least 3 weeks or 6 weeks. In some embodiments, the inactivated tumor cells are administered weekly for three out of four weeks every 3 months. In some embodiments, the inactivated tumor cells are administered weekly for 6 weeks every 3 months.

In some embodiments, the infectious agent is administered weekly. In some embodiments, the immunomodulator is administered weekly. In some embodiments, the inactivated tumor cells are administered weekly.

In some embodiments, the infectious agent is administered daily. In some embodiments, the immunomodulator is administered daily. In some embodiments, the inactivated tumor cells are administered daily.

In some embodiments, the infectious agent is administered first daily or weekly for a number of times (such as any of 1, 2, 3, 4, 5, 6, 7, 10, or more) in a first treatment course, followed by a second treatment course of daily or weekly administration for a number of times (such as any of 1, 2, 3, 4, 5, 6, 7, 10, or more), and then followed by maintenance treatment courses every month or every few (such as any of 2, 3, 4, 5, 6, or more) months. In some embodiments, the immunomodulator is administered first daily or weekly for a number of times (such as any of 1, 2, 3, 4, 5, 6, 7, 10, or more) in a first treatment course, followed by a second treatment course of daily or weekly administration for a number of times (such as any of 1, 2, 3, 4, 5, 6, 7, 10, or more), and then followed by maintenance treatment courses every month or every few (such as any of 2, 3, 4, 5, 6, or more) months. In some embodiments, the inactivated tumor cells are administered first daily or weekly for a number of times (such as any of 1, 2, 3, 4, 5, 6, 7, 10, or more) in a first treatment course, followed by a second treatment course of daily or weekly administration for a number of times (such as any of 1, 2, 3, 4, 5, 6, 7, 10, or more), and then followed by maintenance treatment courses every month or every few (such as any of 2, 3, 4, 5, 6, or more) months.

In some embodiments, the infectious agent, the immunomodulator and the inactivated cells are administered with any combination of the dosing schedules described above. Each treatment course may comprise administration over the course of days, weeks, or months. The treatment course may be repeated for as long as needed.

In some embodiments, the infectious agent and the inactivated tumor cells discussed above are administered sequentially, i.e., the administration of the infectious agent is administered before or after the administration of the inactivated tumor cells. In some embodiments, the infectious agent is administered prior to the administration of the inactivated tumor cells. In some embodiments, the infectious agent is administered no more than about any of 15 minutes, 30 minutes, 1 hour, 2 hours, 3 hours, 4 hours, 5 hours, 6 hours, 12 hours, or 24 hours prior to the administration of the inactivated tumor cells. In some embodiments, the infectious agent is administered about days or weeks (such as about any of 1 day, 2 days, 3 days, 4 days, 5 days, 6 days, 1 week, 2 weeks, 3 weeks, 4 weeks, or more) prior to the administration of the inactivated tumor cells. In some embodiments, the infectious agent is administered after the administration of the inactivated tumor cells. In some embodiments, the infectious agent is administered no more than about any of 15 minutes, 30 minutes, 1 hour, 2 hours, 3 hours, 4 hours, 5 hours, 6 hours, 12 hours, or 24 hours after the administration of the inactivated tumor cells. In some embodiments, the infectious agent is administered about days or weeks (such as about any of 1 day, 2 days, 3 days, 4 days, 5 days, 6 days, 1 week, 2 weeks, 3 weeks, 4 weeks, or more) after the administration of the inactivated tumor cells. In some embodiments, the infectious agent and the inactivated tumor cells are administered with one immediately after another (i.e., within 5 minutes or less between the two administrations). For example, in some embodiments, the infectious agent is administered immediately before the administration of the inactivated tumor cells. In some embodiments, the infectious agent is administered immediately after the administration of the inactivated tumor cells.

In some embodiments, the infectious agent and the inactivated tumor cells are administered simultaneously. In some embodiments, the infectious agent and the inactivated tumor cells are administered simultaneously via separate compositions. In some embodiments, the infectious agent and the inactivated tumor cells are administered as a single composition. In some embodiments, the infectious agent and the inactivated tumor cells are mixed prior to (such as immediately prior to, e.g., within less than about 10, 5, or 1 minutes before) the administration of the composition. In some embodiments, the composition comprising the infectious agent and the inactivated tumor cells is pre-made and stored for at least about any of 1 hours, 2 hours, 3 hours, 4 hours, 5 hours, 6 hours, 12 hours, 24 hours, 2 days, 3 days, 4 days, 5 days, 6 days, 7 days, 2 weeks, 3 weeks, or more prior to the administration. In some embodiments, the inactivated tumor cells and the infectious agent are completely separated until the moment of administration to the individual. In some embodiments, the infectious agent and the inactivated tumor cells do not need to be pre-incubated prior to the administration.

**Infectious agents**

The methods and compositions described herein are related to infectious agents that are oncolytic viruses, including, but not limited to, oncolytic adenoviruses. The infectious agent may be a naturally occurring infectious agent, or a genetically modified infectious agent, for example an attenuated infectious agent, and/or an infectious agent with additional favorable features (e.g., preferential replication in cancer cells, or encoding an immune-related molecule).

Exemplary viruses that are suitable for use in the present invention include, but are not limited to, adenovirus, for example, H101 (ONCOCRINE®), CG-TG-102 (Ad5/3-D24-GM-CSF), and CG0070; herpes simplex virus, for example, Talimogene laherparapvec (T-VEC) and HSV-1716 (SEPREHVIR®); reo virus, for example, REOLYSIN®; vaccinia virus, for example, JX-594; Seneca valley virus, for example, NTX-010 and SVV-001; newcastle disease virus, for example, NDV-NS1 and GL-ONC1; polio virus, for example, PVS-RIPO; measles virus, for example, MV-NIS; coxsackie virus, for example, Cavatak™; vesicular stomatitis virus; maraba and rhabdoviruses; parvovirus and mumps virus.

In some embodiments, the infectious agent is a wild type infectious agent. In some embodiments, the infectious agent is genetically modified. In some embodiments, the infectious agent is attenuated (for example through multiple passages, inactivation or genetic modification). In some embodiments, the infectious agent is only a part, or parts of the wild type infectious agent that can cause infection, inflammation or infection-like effects.

In some embodiments, the infectious agent is an oncolytic virus, such as an oncolytic adenovirus. In some embodiments, the oncolytic virus is a wild type oncolytic virus. In some embodiments, the oncolytic virus is genetically modified. In some embodiments, the oncolytic virus is attenuated (for example through multiple passages, inactivation or genetic modification). In some embodiments, the oncolytic virus is replication competent. In some embodiments, the oncolytic virus preferentially replicates in a cancer cell.

In some embodiments, the infectious agent is an oncolytic virus (such as oncolytic adenovirus) comprising a viral vector comprising a tumor cell-specific promoter operably linked to a viral gene essential for replication of the virus. In some embodiments, the tumor-specific promoter is an E2F-1 promoter, such as a human E2F-1 promoter or an E2F-1 promoter comprising the nucleotide sequence set forth in SEQ ID NO: 1 as shown below. In some embodiments, the viral gene essential for replication of the virus is selected from the group consisting of E1A, E1B, and E4.

SEQ ID NO: 1

gggcccaaaa ttagcaagtg accacgtggt tctgaagcca gtggcctaag gaccaccctt 60
gcagaaccgt ggtctccttg tcacagtcta ggcagcctct ggcttagcct ctgtttcttt 120
cataaccttt ctcagcgcct gctctgggcc agaccagtgt tgggaggagt cgctactgag 180
ctcctagatt ggcaggggag gcagatggag aaaaggagtg tgtgtggtca gcattggagc 240
agaggcagca gtgggcaata gaggaagtga gtaaatcctt gggagggctc cctagaagtg 300
atgtgttttc ttttttgtt ttagagacag gatctcgctc tgtcgcccag gctggtgtgc 360
agtggcatga tcatagctca ctgcagcctc gacttctcgg gctcaagcaa tcctcccacc 420
tcagcctccc aagtagctgg gactacgggc acacgccacc atgcctggct aatttttgta 480
tttttgtag agatgggtct tcaccatgtt gatcaggctg gtctcgaact cctgggctca 540
tgcgatccac cccgccagct gattacaggg attccggtgg tgagccaccg cgcccagacg 600
ccacttcatc gtattgtaaa cgtctgttac ctttctgttc ccctgtctac tggactgtga 660
gctccttagg gccacgaatt gaggatgggg cacagagcaa gctctccaaa cgtttgttga 720
atgagtgagg gaatgaatga gttcaagcag atgctatacg ttggctgttg gagattttgg 780
ctaaaatggg acttgcagga aagcccgacg tccccctcgc catttccagg caccgctctt 840
cagcttgggc tctgggtgag cgggataggg ctgggtgcag gattaggata atgtcatggg 900
tgaggcaagt tgaggatgga agaggtggct gatggctggg ctgtggaact gatgatcctg 960
aaaagaagag gggacagtct ctggaaatct aagctgaggc tgttgggggc tacaggttga 1020
gggtcacgtg cagaagagag gctctgttct gaacctgcac tatagaaagg tcagtgggat 1080
gcgggagcgt cggggcgggg cggggcctat gttcccgtgt ccccacgcct ccagcagggg 1140
acgcccgggc tgggggcggg gagtcagacc gcgcctggta ccatccggac aaagcctgcg 1200
cgcgccccgc cccgccattg gccgtaccgc cccgcgccgc cgccccatcc cgcccctcgc 1260
cgccgggtcc ggcgcgttaa agccaatagg aaccgccgcc gttgttcccg tcacggacgg 1320
ggcagccaat tgtggcggcg ctcggcggct cgtggctctt tcgcggcaaa aaggatttgg 1380
cgcgtaaaag tggccgggac tttgcaggca gcggcggccg gggcggagc gggatcgagc 1440
cctcgccgag gcctgccgcc atgggcccgc gccgccgccg ccgcctgtca cccgggccgc 1500
gcgggccgtg agcgtcatg 1519

In some embodiments, the infectious agent is an oncolytic virus (such as oncolytic adenovirus) comprising a viral vector comprising a tumor cell-specific promoter operably linked to a viral gene essential for replication of the virus and a nucleic acid encoding an immune-related molecule (such as cytokine or chemokine) operably linked to a viral promoter. In some embodiments, the tumor-specific promoter is an E2F-1 promoter, such as a human E2F-1 promoter or an E2F-1 promoter comprising the nucleotide sequence set forth in SEQ ID NO: 1. In some embodiments, the viral gene essential for replication of the virus is selected from the group consisting of E1A, E1B, and E4. In some embodiments, the viral promoter operably linked to the nucleic acid encoding the immune-related molecule is the E3 promoter. In some embodiments, the immune-related molecule is GM-CSF.

In some embodiments, the infectious agent is an adenovirus serotype 5, wherein the endogenous E1a promoter and E3 19kD coding region of a native adenovirus is replaced by the human E2F-1 promoter and a nucleic acid encoding an immune-related molecule (such as cytokine or chemokine, for example, GM-CSF). In some embodiments, the tumor-specific promoter is a human E2F-1 promoter or an E2F-1 promoter comprising the nucleotide sequence set forth in SEQ ID NO: 1.

In some embodiments, the infectious agent is CG0070, an adenovirus serotype 5 which has an E2F promoter at the E1a gene and a GM-CSF expression at the E3 gene.

CG0070 is a conditionally replicating oncolytic adenovirus (serotype 5) designed to preferentially replicate in and kill Rb pathway-defective cancer cells. This vector is transcriptionally regulated by a promoter (e.g., E2F-1 promoter) that is up-regulated in Rb-pathway-detective tumor cells. In approximately 85% of all cancers, one or more genes of the Rb pathway, such as the tumor suppressor Rb gene, are mutated. In addition to its restricted propagation, CG0070 also encodes the human cytokine GM-CSF, which is expressed selectively in the infected tumor cells to stimulate immune responses against uninfected distant (such as metastases) and local tumor foci.

The genomic structure of the oncolytic adenoviral vector CG0070 is shown schematically in Figure 1. Products of the adenoviral early E1A gene are essential for efficient expression of other regions of the adenoviral genome. CG0070 has been engineered to express the E1A gene under control of the human E2F-1 promoter, which provides tumor specificity to the E1A gene product. To protect from transcriptional read-through activating E1A expression, a polyadenylation signal (PA) was inserted 5' of the E2F-1 promoter. CG0070 includes the entire wild type E3 region except for the 19kD-coding region. A direct comparison of E3-containing to E3-deleted oncolytic adenovirus vectors showed superiority of E3-containing vectors in tumor spread and efficacy. In place of the 19kD gene, CG0070 carries the cDNA for human GM-CSF under the control of the endogenous E3 promoter (E3P). Since the E3 promoter is in turn activated by E1A, both viral replication and GM-CSF expression are ultimately under the control of the E2F-1 promoter. The rest of the viral vector backbone, including the E2, E4, late protein regions and inverted terminal repeats (ITRs), is identical to the wild type Ad5 genome.

CG0070 is manufactured in HeLa-S3 cells, and released from infected HeLa-S3 cells by detergent lysis. CG0070 is purified from the lysate by chromatography, and then formulated in 5% sucrose, 10 mM Tris, 0.05% polysorbate-80, 1 % glycine, 1 mM magnesium chloride, pH 7.8.

CG0070 is supplied as a sterile, slightly opalescent, frozen liquid in stoppered glass vials. The particle concentration per mL (vp/mL) is stated on the Certificate of Analysis for each lot of CG0070.

CG0070 has additional potential anti-tumor activity in that it carries the cDNA for human GM-CSF, a key cytokine for generating long-lasting anti-tumor immunity. Thus, CG0070 is a selectively replicating oncolytic vector with the potential for attacking the tumor by two mechanisms: direct cytotoxicity as a replicating vector and induction of a host immune response. Summarized in the following sections are in vitro and in vivo studies conducted to characterize the tumor selectivity and anti-tumor activity and safety of CG0070.

**Immunomodulators**

The methods of the present invention in some embodiments comprise administration of infectious agents with an immunomodulator.

"Immunomodulator" refers to an agent that when present, alters, suppresses or stimulates the body's immune system. Immunomodulators can target specific molecules, such as the checkpoint molecules, or non-specifically modulate the immune response. Immunomodulators can include compositions or formulations that activate the immune system (e.g., adjuvants or activators), or downregulate the immune system. Adjuvants can include aluminum-based compositions, as well as compositions that include bacterial or mycobacterial cell wall components. Activators can include molecules that activate antigen presenting cells to stimulate the cellular immune response. For example, activators can be immunostimulant peptides. Activators can include, but are not limited to, agonists of toll-like receptors TLR-2, 3, 4, 6, 7, 8, or 9, granulocyte macrophage colony stimulating factor (GM-CSF); TNF; CD40L; CD28; FLT-3 ligand; or cytokines such as IL-1, IL-2, IL-4, IL-7, IL-12, IL-15, or IL-21. Activators can include agonists of activating receptors (including co-stimulatory receptors) on T cells, such as an agonist (e.g., agonistic antibody) of CD28, OX40, GITR, CD137, CD27, CD40, or HVEM. Activators can also include compounds that inhibit the activity of an immune suppressor, such as an inhibitor of the immune suppressors IL-10, IL-35, TGF-β, IDO, or cyclophosphamide, or inhibit the activity of an immune checkpoint such as an antagonist (e.g., antagonistic antibody) of CTLA-4, PD-1, PD-L1, PD-L2, LAG3, B7-1, B7-H3, B7-H4, BTLA, VISTA, KIR, A2aR, or TIM3. Activators can also include costimulatory molecules such as CD40, CD80, or CD86. Immunomodulators can also include agents that downregulate the immune system such as antibodies against IL-12p70, antagonists of toll-like receptors TLR-2, 3, 4, 5, 6, 8, or 9, or general suppressors of immune function such as cyclophosphamide, cyclosporin A or FK506. These agents (e.g., adjuvants, activators, or downregulators) can be combined to achieve an optimal immune response.

Immunomodulators of particular interest in the present invention include immune-stimulating agents and immune checkpoint inhibitors. As used herein, the term "immune checkpoint inhibitors," "checkpoint inhibitors," and the like refers to compounds that inhibit the activity of control mechanisms of the immune system. Immune system checkpoints, or immune checkpoints, are inhibitory pathways in the immune system that generally act to maintain self-tolerance or modulate the duration and amplitude of physiological immune responses to minimize collateral tissue damage. Checkpoint inhibitors can inhibit an immune system checkpoint by stimulating the activity of a stimulatory checkpoint molecule, or inhibiting the activity of an inhibitory checkpoint molecule in the pathway. Stimulatory checkpoint molecules are molecules, such as proteins, that stimulate or positively regulate the immune system. Inhibitory checkpoint molecules are molecules, such as proteins, that inhibit or negatively regulate the immune system. Immune system checkpoint molecules include, but are not limited to, cytotoxic T-lymphocyte antigen 4 (CTLA-4), programmed cell death 1 protein (PD-1), programmed cell death 1 ligand 1 (PD-L1), programmed cell death 1 ligand 2 (PD-L2), lymphocyte activation gene 3 (LAG3), B7-1, B7-H3, B7-H4, T cell membrane protein 3 (TIM3), B- and T-lymphocyte attenuator (BTLA), V-domain immunoglobulin (Ig)-containing suppressor of T-cell activation (VISTA), Killer-cell immunoglobulin-like receptor (KIR), and A2A adenosine receptor (A2aR). As such, checkpoint inhibitors include antagonists of CTLA-4, PD-1, PD-L1, PD-L2, LAG3, B7-1, B7-H3, B7-H4, BTLA, VISTA, KIR, A2aR, or TIM3. For example, antibodies that bind to CTLA-4, PD-1, PD-L1, PD-L2, LAG3, B7-1, B7-H3, B7-H4, BTLA, VISTA, KIR, A2aR, or TIM3 and antagonize their function are checkpoint inhibitors. Moreover, any molecule (e.g., peptide, nucleic acid, small molecule, etc.) that inhibits the inhibitory function of an immune system checkpoint is a checkpoint inhibitor.

The immunomodulator can be of any one of the molecular modalities known in the art, including, but not limited to, aptamer, mRNA, siRNA, microRNA, shRNA, peptide, antibody, anticalin, Spherical nucleic acid, TALEN, Zinc Finger Nuclease, CRISPR/Cas9, and small molecule.

In some embodiments, the immunomodulator is an immune checkpoint inhibitor. In some embodiments, the immune-checkpoint inhibitor is a natural or engineered ligand of the inhibitory immune checkpoint molecule PD-L1. In some embodiments, the immune checkpoint inhibitor is an antibody that targets an inhibitory immune checkpoint protein. In some embodiments, the immunomodulator is an antibody selected from the group consisting of anti-PD-L1 (e.g., KY-1003 (EP20120194977). In some embodiments, the antibody is an antagonistic antibody. In some embodiments, the antibody is a monoclonal antibody. In some embodiments, the antibody is a monoclonal antibody. In some embodiments, the antibody is an antigen-binding fragment selected from the group consisting of Fab, Fab', $F(ab')_2$, Fv, scFv, and other antigen-binding subsequences of the full length antibody. In some embodiments, the antibody is a human, humanized, or chimeric antibody. In some embodiments, the antibody is a bispecific antibody, a multispecific antibody, a single domain antibody, a fusion protein comprising an antibody portion, or any other functional variants or derivatives thereof.

The immunomodulators can be used singly or in combination. For example, any number (such as any of 1, 2, 3, 4, 5, 6, or more) of immune checkpoint inhibitors can be used simultaneously or sequentially, or any number (such as any of 2, 3, 4, 5, 6, or more) of immune-stimulating agents can be used simultaneously or sequentially. Alternatively, any number (such as any of 1, 2, 3, 4, 5, 6, or more) of immune checkpoint inhibitors in combination with any number (such as any of 2, 3, 4, 5, 6, or more) of immune-stimulating agents can be used simultaneously or sequentially. Sequential administration of immunomodulators can be separated by hours, days or weeks. The administration route(s) for two or more immunomodulators can be the same or different. For example, one immunomodulator can be administered intratumorally, and a second immunomodulator can be administered intravenously; or two immunomodulators can be administered both intratumorally.

Exemplary immune checkpoint molecules and immunomodulators thereof are discussed below. It is understood that other suitable immune checkpoint molecules and immunomodulators known in the art are also within the scope of the present application.

## CTLA-4

CTLA-4 is an immune checkpoint molecule, which is up-regulated on activated T-cells. An anti-CTLA-4 mAb can block the interaction of CTLA-4 with CD80/86 and switch off the mechanism of immune suppression and enable continuous stimulation of T-cells by DCs. Examples of anti-CTLA-4 antibodies are Ipilimumab (see U.S. Patent Nos. 6,984,720, 7,452,535, 7,605,238, 8,017,114 and 8,142,778), Tremilimumab (see U.S. Patent No. 6,68,736, 7,109,003, 7,132,281, 7,411,057, 7,807,797, 7,824,679 and 8,143,379) and other anti-CTLA-4 antibodies, including single chain antibodies (e.g., see U.S. Patent Nos. 5,811,097, 6051,227 and 7,229,628, and US Patent Publication No. US20110044953).

Two IgG mAb directed against CTLA-4, Ipilimumab and Tremelimumab, have been tested in clinical trials for a number of indications. Ipilimumab is approved by the FDA for the treatment of melanoma, e.g., for late stage melanoma patients. The complete prescribing information is fully described in the packaging insert of YERVOY® (Bristol Meyers). YERVOY® (Ipilimumab) comes in 50mg single use vials.

Anticalins are engineered proteins that are able to recognize and bind specific targets with high affinity. They are antibody mimetics, but they are not structurally related to antibodies. Instead, they are derived from human lipocalins, which are a family of naturally binding proteins. Anticalins are being used in lieu of monoclonal antibodies, but are about eight times smaller than monoclonal antibodies with a size of about 180 amino acids and a mass of about 20 kDa. Anticalins have been described in U.S. Patent No. 7,250, 297. Anticalins that bind CTLA-4 with high affinity and specificity have been developed, which are described in, for example, International Patent Application Publication No. WO2012072806. Any of the CTLA-4-binding anticalins may be used in the present application. In some embodiments, the CTLA-4 binding anticalin is PRS-010 (Piers AG).

PD-1

PD-1 is a part of the B7/CD28 family of co-stimulatory molecules that regulate T-cell activation and tolerance, and thus antagonistic anti-PD-1 antibodies can be useful for overcoming tolerance. PD-1 has been defined as a receptor for B7-4. B7-4 can inhibit immune cell activation upon binding to an inhibitory receptor on an immune cell. Engagement of the PD-1/PD-L1 pathway results in inhibition of T-cell effector function, cytokine secretion and proliferation. (Turnis et al., OncoImmunology 1(7):1172-1174, 2012). High levels of PD-1 are associated with exhausted or chronically stimulated T cells. Moreover, increased PD-1 expression correlates with reduced survival in cancer patients.

Agents for down modulating PD-1, B7-4, and the interaction between B7-4 and PD-1 inhibitory signal in an immune cell resulting in enhancement of the immune response. Any of the anti-PD-1 antibodies known in the art may be used in the present invention, for example, see US Patent Nos. US7101550, US5698520, US6808710, US7029674, US7794710, US7892540, US8008449, US8088905, US8163503, US8168757, US8354509, US8460927, US8609089, US8747833, US8779105, US8900587, US8952136, US8981063, US8993731, US9062112, US9067999, US9073994, US9084776, US9102728, and US7488802; and U.S. Patent Publication Nos. US20020055139, US20140044738. For example, Nivolumab is a human mAb to PD-1 that is FDA approved for the treatment of unresectable or metastatic melanoma, as well as squamous non-small cell lung cancer.

PD-L1/PD-L2

PD-L1 (Programmed cell death-ligand 1) is also known as cluster of differentiation 274 (CD274) or B7 homolog 1 (B7-H1). PD-L1 serves as a ligand for PD-1 to play a major role in suppressing the immune system during particular events such as pregnancy, tissue allographs, autoimmune disease and other disease states such as hepatitis and cancer. The formation of PD-1 receptor/PD-L1 ligand complex transmits an inhibitory signal which reduces the proliferation of CD8+ T cells at the lymph nodes.

Any of the known anti-PD-L1 antibodies may be used in the present invention, see, for example, U.S. Patent Nos. US7943743, US7722868, US8217149, US8383796, US8552154, and US9102725; and U.S. Patent Application Publication Nos. US20140341917, and US20150203580; and International Patent Application No. PCT/US2001/020964. For example, anti-PD-L1 antibodies that are in clinical development include BMS935559 (also known as MDX-1105), MPDL3280A, MEDI4736, Avelumab (also known as MSB0010718C), KY-1003, MCLA-145, RG7446 (also known as atezolizumab), and STI-A1010.

PD-L2 (Programmed cell death 1 ligand 2) is also known as B7-DC. PD-L2 serves as a ligand for PD-1. Under certain circumstances, PD-L2 and its inhibitor can be used as a substitute for PD-L1 and its inhibitor respectively.

EXAMPLES

The examples below are intended to be purely exemplary of the invention and should therefore not be considered to limit the invention in any way. The following examples and detailed description are offered by way of illustration and not by way of limitation.

**Example 1: A Phase I/II Clinical Study of Intravesical Administration of CG0070 in Combination with a CTLA-4 inhibitor in Patients with Muscle Invasive Bladder Cancer**

This example describes a clinical study of intravesical administration of CG0070 in combination with an anti-CTLA-4 antibody in patients with muscle invasive bladder cancer (MIBC). Muscle invasive bladder cancer is chosen herein as an example because CG0070 has shown to be active in bladder cancer. Furthermore all muscle invasive bladder cancer patients need to have a cystectomy, thus providing a good tumor specimen to prepare the tumor cells needed for this vaccine system. In addition the prognosis of muscle invasive bladder cancer patients (T3-4) has been poor despite the use of neo-adjuvant chemotherapy. Most of these patients are over 60 years of age and few can undergo the serious side effects of chemotherapy. An effective agent that can minimize the risk of disease recurrence in this patient population is an unmet need.

This clinical study is a phase VII, single-arm, open-label, interventional dose-escalation safety and efficacy study of intravesical CG0070 in combination with a CTLA-4 inhibitor as a neo-adjuvant therapy in patients with transitional cell muscle-invasive bladder cancer disease, who have been selected for radical cystectomy and pelvic lymphadenectomy. The primary safety objective of the study is to investigate whether CG0070 and CTLA 4 blockade is safe and tolerable for the neo-adjuvant treatment of MIBC patients prior to cystectomy. The primary efficacy objective of the study is to measure tumor PD-L1 or PD-1 level changes after CG0070 and CTLA-4 inhibitor neo-adjuvant treatment. Secondary study objectives include evaluation of 2-year Disease Free Survival (DFS), 2-year Progression Free Survival (PFS), Overall Survival (OS), Pathological Complete Response proportion at Cystectomy (p0 proportion), Pathological Down Staging Proportion at Cystectomy, and Organ Confined Disease Proportion at Cystectomy.

In the Phase I portion of the study, cohorts of (e.g., three to six) patients receive intravesical CG0070 and CTLA 4 Blockade at one of four dose levels. The first dose level consists of CG0070 alone. Each patient receives 4 weekly installations of intravesical CG0070 (e.g., on Day 1 of each week), and 3 weekly CTLA-4 inhibitor (e.g., Ipilimumab) at one of four dose levels from the second week (e.g., on Days 8, 15, and 22) with administration of the CTLA-4 inhibitor following CG0070.

Dose escalation follows a modified Fibonacci sequence in which the dose increments become smaller as the dose increases. For example, if none of the first three patients in a cohort experiences a dose-limiting toxicity, another three patients will be treated at the next higher dose level. However, if one of the first three patients experiences a dose-limiting toxicity, three more patients will be treated at the same dose level. The dose escalation continues until at least two patients among a cohort of three to six patients experience dose-limiting toxicities (i.e., ≥33% of patients with a dose-limiting toxicity at that dose level). The recommended dose for the next stage or phase of the trial is conventionally defined as the dose level just below this toxic dose level. Dose-limiting toxicity (DLT) is defined with the use of the Common Terminology Criteria for Adverse Events (CTCAE) version 4. A DLT is defined as a ≥ Grade 3 drug-related Adverse Events (AE) from day 1 of week 1 to day 1 of week 4 of treatment, including any grade 3 or higher toxicity which requires interruption of study treatment for more than 3 consecutive weeks and/or permanent discontinuation of the drug(s) due to immune-related toxicities, but excluding Grade 3 AE of tumor flare (defined as local pain, irritation, or rash localized at sites of known or suspected tumor) and Grade 3 immune-mediated events of the skin (rash, pruritus) or endocrine systems (hypothyroidism, hyperthyroidism, hypopituitarism, adrenal insufficiency, hypogonadism and Cushingoid syndrome) that resolve to Grade 1 or baseline within 3 weeks with or without the administration of steroids. Hepatic immune toxicity is defined as Grade 3 or higher elevation in aspartate aminotransferase, alanine aminotransferase or total bilirubin. A significant D-dimer increase (20% increase with at least a 1 μg/mL from baseline) in combination with a > grade 2 change in INR, PT, PTT, platelets, or fibrinogen lasting for > 7 days is considered a DLT. In addition, clinically significant thrombosis or bleeding related to CG0070 treatment is considered a DLT. Patients with a treatment delay extending beyond 21 days due to toxicity related to study treatment are considered as having a treatment related DLT. For reasons other than treatment related toxicity, patients with a treatment delay extending beyond 7 days or who withdraw from the study before 3 administrations are replaced within the cohort. The maximum tolerated dose (MTD) is the dose immediately preceding that resulting in 2 DLT. If the MTD is not defined, the highest dose administered without 2 DLT will be the Maximum Feasible Dose (MFD). Dose reduction for patients in this study is not allowed. However, if at least 2 out of 6 patients in dose level 1 experience a DLT, three patients will be enrolled at dose level 1. Furthermore, if at least 2 out of 6 patients in dose level 1 experience a DLT, three patients will be enrolled at dose level 2.

For example, Dose Level I includes intravesical administration of CG0070 alone at a dose of $1 \times 10^{12}$ viral particles (vp) once weekly for four weeks. Dose Level II includes: (1) intravesical administration of CG0070 at a dose of $1 \times 10^{12}$ viral particles (vp) once weekly for four weeks; and (2) immediately after CG0070 installation and drainage, intravesical administration of CTLA-4 inhibitor (e.g., Ipilimumab) at a dose of 0. 1mg/Kg but not exceeding 20 mg in total per dose, weekly for three weeks, starting from week 2 and ending on week 4. Dose Level III includes: (1) intravesical administration of CG0070 at a dose of $1 \times 10^{12}$ viral particles (vp) once weekly for four weeks; and (2) immediately after CG0070 installation and drainage, intravesical administration of CTLA-4 inhibitor (e.g., Ipilimumab) at a dose of 0.2mg/Kg but not exceeding 20 mg in total per dose, weekly for three weeks, starting from week 2 and ending on week 4. Dose Level IV includes: (1) intravesical administration of CG0070 at a dose of $1 \times 10^{12}$ viral particles (vp) once weekly for four weeks; and (2) immediately after CG0070 installation and drainage, intravesical administration of CTLA-4 inhibitor (e.g., Ipilimumab) at a dose of 0.3mg/Kg but not exceeding 20 mg in total per dose, weekly for three weeks, starting from week 2 and ending on week 4.

In the Phase II portion of the study, each patient is administered intravesically CG0070 in combination with the CTLA-4 inhibitor at a dose level determined in the Phase I portion of the study for a four-week treatment course. During both Phase I and Phase II portions of the study, prior to administration of the intravesical therapy, each patient is assessed for adverse events, and samples (such as blood and urine samples) are collected for laboratory assessment. For example, prior to the first intravesical administration of CG0070, blood and urine samples are collected from each patient to assess GM-CSF level, as well as CG0070 and wildtype adenovirus levels. Prior to each of the week 2, 3, and 4 administrations, samples from patients are collected to for laboratory assessment in hematology (such as CBC with differential, chemistry and coagulation), serum chemistry (such as sodium, potassium, chloride, BUN, creatinine, glucose, total protein, albumin, calcium, total bilirubin, direct bilirubin, alkaline phosphate, LDH, AST, ALT, and thyroid functions), and urinalysis. Vital signs, including blood pressure, pulse, respirations and temperature are recorded prior to each CG0070 treatment and every hour for 2 hours total during the treatment to ensure the patient is clinically stable.

CG0070 and the CTLA-4 inhibitor can be administered as follows. Patients are advised not to drink fluids for 4 hours before treatment and should empty their bladder prior to treatment administration. On the study day, each patient receives pretreatment with a transduction enhancing agent (DDM) administered intravesically via a catheter (Rusch 173430 Foley Catheter & BARD LUBRI-SIL Foley Catheter #70516SI). Pretreatment consists of an intravesical wash with 100 mL normal saline, followed by an intravesical wash with 75 mL of 0.1 % DDM. The patient then receive intravesical instillation of 100 mL of 0.1 % DDM, which is retained in the bladder for 12 - 15 minutes and subsequently rinsed with 100 mL of saline. If a patient is unable to tolerate at least 5 minutes of DDM pretreatment, further treatment with CG0070 and CTLA-4 inhibitor should be discontinued for that treatment. If the intravesical infusion of CG0070 is delayed for more than two hours after DDM pretreatment, the patient will not receive CG0070 and must be rescheduled for DDM and CG0070 treatment no sooner than 2 days later. If treatment is delayed for more than 2 weeks, patients must continue to meet eligibility criteria prior to retreatment. Following pretreatment with DDM, each patient receives a single intravesical instillation via catheter (e.g., Rusch 173430 Foley Catheter & BARD LUBRI-SII, Foley Catheter #70516SI) of 100 mL of CG0070 at a concentration of $1.0 \times 10^{10}$ vp/mL with a 45 to 50 minute dwell time. Treatment must occur at least 14 days following any prior bladder biopsy. Patients who experience bleeding during catheter insertion (traumatic catheterization) should not be treated with CG0070. While CG0070 is held in the bladder, the patient should be repositioned from left side to right side and also should lie upon the back and the abdomen to maximize bladder surface exposure to CG0070. The patient position is changed every 10-12 minutes for a total of 45 to 50 minutes. CG0070 is then be drained through the catheter into a disposal bag. As soon as the CG0070 solution has been drained from the bladder, the CTLA-4 inhibitor (for example, Ipilimumab, such as YERVOY®) at the appropriate dosage (e.g., Dose Level I of Phase I study does not include any CTLA-4) is diluted into 100 ml of normal saline, and is instilled into the bladder. After instillation, urethral catheter is then withdrawn and patient is asked to hold for another 45 min to 1 hour (or as long as possible) before emptying by urination.

After the 6-week treatment course in the Phase II portion of the study, each patient receives a cystectomy. Cystectomy is performed 10 to 14 days (e.g., about Day 40) after the last intravesical treatment or as soon as any treatment related toxicity has subsided and medical condition is suitable for surgery. After the cystectomy, tumor specimen is obtained from the patient and assessed in a pathology lab, and laboratory evaluation is performed to determine if the patient has responded to the treatment. This assessment includes pathological and immunological assessments of the resected tumor for: (1) tumor stage and grade, if present; (2) tumor immunological parameters, such as Treg, CD4, CD8 and other T cell subsets; (3) tumor PD-L1 expression status by immunohistochemistry methods; (4) lymph node involvement; (5) macroscopic photo comparison between pre- and post-treatment. Each patient is evaluated at months 3, 6, 12, 18, and 24 (plus or minus 2 weeks) from the date of cystectomy to monitor long-term response and toxicity of CG0070, disease recurrence or progression, and subsequent therapies and response. After 2 years, patients are contacted once a year for assessment of long-term toxicities related to gene therapy (such as new malignancies, autoimmune disease, neurologic and hematologic disorders, etc.), and survival for five years after the first intravesical CG0070 therapy. Patients are followed for up to 5 years in total post treatment with CG0070, or according to current FDA guidelines and the current standard of care.

Primary outcome measures of the study are determined as follows. Patients are followed throughout and upon completion of the study for assessment of AE, SAE, and SUSAR to determine safety and tolerability of the treatment. Additionally, at cystectomy, efficacy of the treatment is assessed by determining the rate of change in PD-L1 and PD-1 status, which is defined as the difference in proportions of patients that are PDL1 or PD1 positive before and after intervention for at least three or more completed intravesical instillations.

Secondary outcome measures of the study are determined as follows. At cystectomy, Pathological Complete Response Proportion at Cystectomy for each T stage (p0 proportion) is assessed by determining the proportion of patients with a pathological complete tumor response at the primary tumor site after intervention at cystectomy stratified further by T staging and for the whole group of patients. Also determined at the time of cystectomy are Pathological Down Staging Proportion at Cystectomy, defined as the proportion of patients with a downgrade of tumor stage or grade at the primary tumor site after intervention at cystectomy; and Organ Confined Disease Proportion at Cystectomy, defined as the proportion of patients with no positive lymph nodes found at cystectomy. Up to 2 years after the cystectomy, patients are followed to determine 2-year Disease Free Survival, defined as the number of months from the date of cystectomy to the earlier of disease recurrence or death (whatever the cause); and 2-year Progression Free Survival for patients with residual disease after cystectomy, defined as the number of months from the date of cystectomy to the earlier of disease progression or death (whatever the cause). Up to five years after the cystectomy, patients are followed to determine Overall Survival, defined as the number of months from the date of cystectomy to the date of death (whatever the cause).

Additionally, exploratory outcome measures to be assessed during the course of the study include, but are not limited to, changes in immune functions within the primary tumor site including assessment of changes in Treg (CD4+CD25+Foxp3+), CD4, CD8, CD4RO45 and CD4ICOShigh etc. before and after intervention; macroscopic changes in the primary tumor site by photographs taken before and after intervention; systemic absolute lymphocyte counts; and systemic cytokine patterns in the patients.

Patients must meet all of the following conditions to be eligible for the study:

1. 18 years of age or older;
2. Pathologically diagnosed transitional cell (urothelial) bladder cancer patients; where radical cystectomy with curative intent is indicated for muscle invasive disease (i.e., American Joint Committee on Cancer (AJCC) stage T2-4a, $N_{X-1}$, M0). Patients must be able to enter into the study within five weeks of their most recent diagnostic procedure, which is usually a diagnostic biopsy, a transurethral resection of bladder tumor (TURBT) procedure or other diagnostic scanning such as CT, MRI and PET procedures;
3. Histopathologically confirmed, transitional cell (urothelial) carcinoma. Urothelial tumors with mixed histology (but with <50% variant) are eligible;
4. Ineligible to receive neo-adjuvant chemotherapy due to a medical condition that can be confirmed by the investigator. (For example, renal impairment can be based on a calculated creatinine clearance of about <60ml/min OR hearing loss ≥ 25 dB by audiometry, averaged at 3 contiguous test frequencies in at least 1 ear; or other significant cardio dysfunction, vascular disease or chronic obstructive pulmonary disease etc.), or refuses to receive neo-adjuvant chemotherapy after a specific informed consent that addresses the increased risks of both recurrence and morbidity without neo-adjuvant chemotherapy;
5. Have an Eastern Cooperative Oncology Group (ECOG) performance status ≤2;
6. Not pregnant or lactating;
7. Agree to study informed consent and HIPAA authorization for release of personal health information;
8. Adequate baseline CBC and hepatic function, as defined as:

    a. WBC>3000 cells/mm3, ANC>1,000 cells/mm3, hemoglobin >9 g/dL, and platelet count >80,000/mm3;
    b. Bilirubin, AST and ALT less than 2.5x Upper Limit of Normal;
    c. Adequate coagulation with acceptable PT/INR, PTT, and fibrinogen (less than 1.5 of Upper Limit of Normal or according to institutional specifications);
    d. Absolute lymphocyte count ≥ 800/μL.

Patients who meet any of the following exclusion criteria are excluded from the study:

1. History of anaphylactic reaction following exposure to humanized or human therapeutic monoclonal antibodies, hypersensitivity to GM-CSF, clinically meaningful allergic reactions or any known hypersensitivity or prior reaction to any of the formulation excipients in the study drugs;
2. Known infection with HIV, HBV or HCV;
3. Anticipated use of chemotherapy or radiotherapy not specified in the study protocol while on study;
4. Any underlying medical condition that, in the Investigator's opinion, will make the administration of study drugs hazardous to the patient, would obscure the interpretation of adverse events, or surgical resection;
5. Systemic treatment on any investigational clinical trial within 28 days prior to registration;
6. Concurrent treatment with other immunosuppressive or immune-modulatory agents, including any systemic steroid (exception: inhaled or topically applied steroids, and acute and chronic standard dose NSAIDs, are permitted). Use of a short course (i.e., ≤ 1 day) of a glucocorticoid is acceptable to prevent a reaction to the IV contrast used for CT scans;
7. Immunosuppressive therapy, including: cyclosporine, antithymocyte globulin, or tacrolimus within 3 months of study entry;
8. History of stage III or greater cancer, excluding urothelial cancer. Basal or squamous cell skin cancers must have been adequately treated and the subject must be disease-free at the time of registration. Patients with a history of stage I or II cancer must have been adequately treated and have been disease-free for ≥ 2 years at the time of registration'
9. Concomitant active autoimmune disease (e.g., rheumatoid arthritis, multiple sclerosis, autoimmune thyroid disease, uveitis);
10. Progressive or current viral or bacterial infection. All infections must be resolved and the patient must remain afebrile for seven days without antibiotics prior to being placed on study.

**Example 2: A Phase I/II Clinical Study of Intratumoral Administration of CG0070 in Combination with a CTLA-4 Inhibitor for Patients with Refractory Injectable Solid Tumors**

This example describes a Phase I/II clinical study of CG0070 in combination with a CTLA-4 inhibitor (such as an anti-CTLA-4 monoclonal antibody or blocker) for patients with refractory injectable solid tumors. This study is a multi-center, single-arm, open-label, interventional study aimed at evaluating the safety and efficacy of the combination therapy comprising intratumoral administration of CG0070 and a CTLA-4 inhibitor in patients with solid tumor, including cutaneous or visceral lesions, such as head and neck squamous cell cancer, breast cancer, colorectal cancer, pancreatic adenocarcinoma, ovarian cancer, non-small cell lung cancer, prostate cancer, and melanoma. The CG0070 administration can include a pretreatment with a transducer, such as DDM.

The clinical study in Phase I is divided into three stages. In Stage 1, each subject is administered CG0070 via intratumoral injections weekly (e.g., on Day 1 of each week) for six weeks. Cohorts of (e.g., three to six) patients receive intratumoral CG0070 (e.g., with DDM pretreatment) at one of four dose levels. Dose escalation procedure is as described in Example 1, and MTD/MFD determined in Stage 1 is used for the beginning of the Stage 2.

In Stage 2 of Phase I, each subject is administered a CTLA-4 inhibitor (such as an anti-CTLA-4 mAb or blocker, e.g., Ipilimumab) via intratumoral injections weekly (e.g., on Day 1 of each week) for six weeks. Cohorts of (e.g., three to six patients) receive intratumoral CTLA-4 inhibitor at one of three dose levels. Dose escalation procedure is as described in Example 1, and MTD/MFD determined in Stage 1 is used for the beginning of the Stage 3.

In Stage 3 of Phase I, each subject is administered a combination of CG0070 (e.g., with DDM pretreatment) at a dose determined in Stage 1 of the study with the CTLA-4 inhibitor at a dose determined in Stage 2 of the study via intratumoral injections weekly (e.g., on Day 1 of each week) for a 6 weeks. Cohorts of (e.g., three to six) patients receive intratumoral CG0070 (e.g., with DDM pretreatment) followed by the CTLA-4 inhibitor at one of three dose levels for 6 weeks. Dose escalation procedure is as described in Example 1. Once the MTD or MFD has been reached, the patients receive repeated 6-week treatment course (once weekly for six weeks constitute one course) at 3 month after the first injection and subsequent courses every 3 months until complete response, disappearance of all injectable tumors, confirmed disease progression or intolerance of study treatment, whichever occurs first. Patients who are in the dose escalation phase of stage 1 or stage 2 portion of the study can be enrolled in the repeat MTD or MFD courses study after a period of three months from the last intervention with full successful enrollment evaluation.

There are two primary outcome measures for this study: (1) safety and tolerability; and (2) efficacy. Safety and tolerability are evaluated from the beginning of each stage until 3 months following enrollment of the last subject in each stage. Stage 1 determines the safety and tolerability of CG0070 (e.g., with DDM pretreatment) as assessed by incidence of dose-limiting toxicities (DLT) in patients with refractory solid tumors. Stage 2 determines the safety and tolerability of the CTLA-4 inhibitor (such as anti-CTLA-4 mAb or blocker) as assessed by incidence of dose-limiting toxicities (DLT) in patients with refractory solid tumors. Stage 3 determines the safety and tolerability of CG0070 (e.g., with DDM pretreatment) in combination with the CTLA-4 inhibitor (such as anti-CTLA-4 mAb or blocker) as assessed by incidence of dose-limiting toxicities (DLT) in patients with refractory solid tumors. Efficacy is evaluated from the beginning of each stage until 24 months following enrollment of the last subject at each stage. Efficacy is assessed by confirmed objective response rate (ORR) of the treatment with only CG0070 (e.g., with DDM pretreatment) in Stage 1, with only CTLA-4 inhibitor (such as anti-CTLA-4 mAb or blocker) in Stage 2, and with CG0070 (e.g., with DDM pretreatment) in combination with CTLA-4 inhibitor (such as anti-CTLA-4 mAb or blocker) in Stage 3 in patients with injectable refractory solid tumors.

The secondary outcome measures of this study are as follows. Safety secondary outcomes are assessed from the beginning of each stage until 24 months following enrollment of the last subject at each stage. For all three stages, safety secondary outcome measures include incidence of all Adverse Events (AEs), grade 3 or greater AEs, events requiring discontinuation of study drug(s), local effects on tumor, clinically significant laboratory changes and clinically significant changes in vital signs. The efficacy secondary outcomes are assessed from the beginning of each stage until 24 months following enrollment of the last subject at each stage. For all three stages, efficacy secondary outcome measures include Best Overall Response Rate (BOR), Disease Control Rate (DCR), Durable Response Rate (DRR), Duration of Response (DOR), Time to Response (TTR), Progression Free Survival (PFS), Overall Survival Rate (OS), 1 year and 2 year Survival Rate.

Eligibility of patients of both genders for the study is determined based on the following inclusion criteria:

1. Patients must have histologically confirmed solid tumors that have failed standard therapies (surgery, chemotherapy, radiotherapy, or endocrine therapy) and for which no curative options exist, including, but not limited to: squamous cell carcinoma of the head and neck, squamous cell carcinoma of the skin, carcinoma of the breast, malignant melanoma, colorectal cancer, pancreatic adenocarcinoma, ovarian cancer, non-small cell lung cancer and prostate cancer;
2. Patients may have had any kind and number of prior cancer therapies;
3. Patients must have measurable lesions that are evaluable by the RECIST method;
4. The tumor mass to be treated must be adequate for injections (i.e., more than 2 cm away from major vascular structures) and measurement by RECIST;
5. Patients must be ≥ 18 years of age;
6. Patients must have a life expectancy of ≥ 12 weeks;
7. Patients must have an Eastern Cooperative Oncology Group (ECOG) performance status of 0, 1, or 2;

8. Patients must have adequate hepatic function, as defined as:

a. Total bilirubin levels ≤ 1.5 x upper limit of normal (ULN); and
b. AST/ALT levels ≤ 2.5 x ULN, or ≤ 5 x ULN if liver metastases are present;

9. Patients must have adequate renal function as defined as serum creatinine ≤ 1.5 x ULN or creatinine clearance (calculated) ≥ 60 mL/min/1.73m2 for patients with creatinine > 1.5 x ULN;
10. Patients must have adequate bone marrow function, as defined as:

a. Absolute neutrophil count ≥ 1,200/μL; and
b. Platelet count ≥ 80,000/μL;

11. Patients must have no known bleeding diathesis or coagulopathy that would make intratumoral injection or biopsy unsafe;
12. Men and women of childbearing potential must agree to use adequate contraception prior to study entry and for up to six months;
13. Females of childbearing potential must have a negative urine or serum pregnancy test within one week prior to start of treatment; and
14. Patients must be able to understand and willing to sign a written informed consent document.

The following patients are excluded from the study:

1. Patients receiving chemotherapy, immunotherapy or radiotherapy within 4 weeks prior to screening, or adverse events > Grade 1, except alopecia, resulting from agents administered more than 4 weeks prior to screening;
2. Patients with a history of significant tumor bleeding, or coagulation or bleeding disorders;
3. Patients with target tumors that could potentially invade a major vascular structure(s) (e.g., innominate artery, carotid artery), based on unequivocal imaging findings, as determined by a radiologist;
4. Patients with Grade ≥ 1 pre-existing neurologic abnormalities (CTCAE version 4.0);
5. Patients who have been hospitalized for emergent conditions requiring inpatient evaluation, treatment or procedure during the 30 days prior to entry on study. In addition, emergent conditions requiring inpatient evaluation, treatment or procedure must have resolved or be medically stable and not severe for 30 days prior to entry on study;
6. Patients with clinically evident Human Immunodeficiency Virus (HIV), Hepatitis B Virus (HBV), Hepatitis C virus (HCV), or Epstein-Barr virus (EBV) infection. Patients are tested for HIV during pre-treatment screening
7. Patients receiving steroids or immunosuppressive agents, e.g., for rheumatoid arthritis;
8. Patients who have concurrent use of any other investigational agents;
9. Patients with presence or history of central nervous system metastasis;
10. Pregnant or breastfeeding women or women desiring to become pregnant within the timeframe of the study;
11. Patients with uncontrolled inter-current illness including, but not limited to, ongoing or active infection, symptomatic congestive heart failure, unstable angina pectoris, cardiac arrhythmia, or psychiatric illness/social situations that would limit compliance with study requirements.

**Example 3: A Phase I/II Clinical Study of Intratumoral Administration of CG0070 in Combination with a CTLA-4 Inhibitor and a CD40 agonist for Patients with Advanced Stage Solid Tumor (such as Melanoma)**

This example describes a Phase I/II clinical study of CG0070 in combination with a CTLA-4 inhibitor (such as an anti-CTLA-4 monoclonal antibody or blocker) and a CD40 agonist (such as an agonistic anti-CD40 antibody) for patients with solid or lymphatic tumors. Phase I study is a dose escalation study for patients with refractory solid tumors. Phase II study is a single-arm, open-label, interventional study aimed at evaluating the efficacy, safety and tolerability of repeated intratumoral injections of CG0070, the CTLA-4 inhibitor, and the CD40 agonist in patients with solid tumor, such as refractory unresectable, or metastatic stage III/IV malignant melanoma. The CG0070 administration can include a transduction enhancing agent, such as DDM.
The clinical study in Phase I is divided into three stages. Stage 1 is a dose escalation study for intratumoral injection of CG0070 alone. Cohorts (e.g., three to six) of patients receive weekly intratumoral injection of CG0070 (e.g., with DDM) for four weeks at one of the following four dose levels: $5 \times 10^{10}$ vp, $1 \times 10^{11}$ vp, $5 \times 10^{11}$ vp, or $1 \times 10^{12}$ vp. For example, the virus CG0070 is reconstituted in 0.1% of DDM in saline. The total volume of each dose is 2 mL. The concentration of the CG0070 solution is about $2.5 \times 10^{10}$vp/ml for the lowest dose and about $5 \times 10^{11}$ vp/ml for the highest dose. If the patient has a single lesion, which must be greater than 2 cm, the total volume of the CG0070 solution is injected into the lesion. If there are two or more lesions, the maximum injection volume based on the lesion size as shown in Table 1 is followed. Any remaining volume is injected into the largest lesion, if the largest lesion is at least 2 cm. If the largest lesion is less than 2 cm, then the remaining volume is divided between the two larger lesions. The maximum number of lesions injected is 3. The total dose is given regardless the total number and size of the lesions. Dose escalation procedure is as described in Example 1, and MTD/MFD is designated as Dose Level Stage 1, which is used at the beginning of Stage 2.

**Table 1. Injection volume per lesion based on tumor size**

| Tumor Size (longest dimension) | Maximum Injection Volume |
|---|---|
| ≥ 5.0 cm | 2.0 mL |
| ≥ 2.0 cm to 5.0 cm | 1.0 mL |
| > 0.5 cm to 2.0 cm | 0.5 mL |

Stage 2 of Phase I is a dose escalation of intratumoral injection of a CTLA-4 inhibitor (such as an anti-CTLA-4 mAb or blocker, e.g., Ipilimumab) in combination of CG0070 at Dose Level Stage 1. Cohorts (e.g., three to six) of patients receive weekly intratumoral injection of a fixed dose of CG0070 (e.g., with DDM) in combination with the CTLA-4 inhibitor (e.g., Ipilimumab) at one of the following three dose levels: 6 mg, 12 mg, or 18 mg, for six weeks. For each administration, CG0070 is first injected intratumorally according to the injection volume per lesion as defined in Stage 1. Immediately after each CG0070 injection, the CTLA-4 inhibitor is administered. The total volume at each dose level, and the maximum injection volumes based on lesion sizes for more than two injected lesions are listed in Table 2 below. The maximum number of injected lesions is 3, and the total dose of the CTLA-4 inhibitor is given regardless the total number and size of the lesions. Any remaining volume of the CTLA-4 inhibitor is administered subcutaneously around the injected lesion(s). In case lesions completely resolved prior to the last planned treatment, both CG0070 and the CTLA-4 inhibitor (e.g., Ipilimumab) can be administered to a previously un-injected lesion. If all lesions are resolved before the end of the treatment course, the CTLA-4 inhibitor (e.g., Ipilimumab) alone can be injected in the subcutaneous area at or around the former lesion. Dose escalation procedure is as described in Example 1, and MTD/MFD is designated as Dose Level Stage 2, which is used at the beginning of Stage 3.

**Table 2. Injection volume of immunomodulator per lesion based on tumor size**

| Dose level | 6.0 mg | | 12 mg | | 18 mg | |
|---|---|---|---|---|---|---|
| Tumor Size (longest dimension) | Max dose per lesion | Max Volume | Max dose per lesion | Max Volume | Max dose per lesion | Max Volume |
| ≥ 5.0 cm | 6.0 mg | 1.2 mL | 12 mg | 2.4 mL | 18 mg | 3.6 mL |
| ≥ 2.0 cm to 5.0 cm | 3.0 mg | 0.6 mL | 6.0 mg | 1.2 mL | 9 mg | 1.8 mL |
| > 0.5 cm to 2 cm | 1.5 mg | 0.3 mL | 3.0 mg | 0.6 mL | 4.5 mg | 0.9 mL |

Stage 3 of Phase I is a dose escalation of intratumoral injection of a CD40 agonist (such as a CD40 agonistic antibody, e.g., APX005M) in combination with the CTLA-4 inhibitor (such as an anti-CTLA-4 mAb or blocker, e.g., Ipilimumab) and CG0070 at Dose Level Stage 2. Cohorts (e.g., three to six) of patients receive weekly intratumoral injection of a fixed dose of CG0070 (e.g., with DDM) and the CTLA-4 inhibitor (e.g., Ipilimumab) in combination with the CD40 agonist (e.g., APX005M) at one of the following three dose levels: 6 mg, 12 mg, or 18 mg, for six weeks. For each administration, CG0070 and the CTLA-4 inhibitor (e.g., Ipilimumab) at Dose Level Stage 2 is adjusted to 2 mL and injected intratumorally according to the injection volume per lesion as defined in Table 1. Immediately after each CG0070/CTLA-4 inhibitor injection, the CD40 agonist (e.g., APX005M) is administered. The total volume at each dose level, and the maximum injection volumes based on lesion sizes for more than two injected lesions are listed in Table 2. The maximum number of injected lesions is 3, and the total dose of the CD40 agonist (e.g., APX005M) is given regardless the total number and size of the lesions. Any remaining volume of CD40 agonist (e.g., APX005M) is administered subcutaneously around the injected lesion(s). In case lesions completely resolved prior to the last planned treatment, CG0070, the CTLA-4 inhibitor (e.g., Ipilimumab) and CD40 agonist (e.g., APX005M) can be administered to a previously un-injected lesion. If all lesions are resolved before the end of the treatment course, the CD40 agonist (e.g., APX005M) alone can be injected in the subcutaneous area at or around the former lesion. Dose escalation procedure is as described in Example 1, and MTD/MFD is designated as the study dose, which is used in Phase II.

For Phase II of the study, the cohort of patients first receive a once weekly intratumoral injection of the three-component combination of CG0070 (e.g., with DDM), the CTLA-4 inhibitor (e.g., Ipilimumab), and the CD-40 agonist (e.g., APX005M) at the study dose determined in Stage 3 of Phase I for four weeks, followed by intratumoral injections of the three-component combination once every 2 weeks for four times. Afterwards, a monthly intratumoral injection of the three-component combination is administered for maintenance treatment until complete response, disappearance of all injectable tumors, confirmed disease progression or intolerance of study treatment, whichever occurs first. Patients who are in the dose escalation phase of Phase I (e.g., stage 1, 2 or 3) can be enrolled in the Phase II study as long as there is a rest period of at least four weeks from the last dose. For each administration, GC0070 is first injected to the lesions, followed by the CTLA-4 inhibitor (e.g., Ipilimumab) and the CD40 agonist (e.g., APX005M). The largest injectable tumor (as determined by PI) is the first tumor to be injected, and the injection volume and dose are according to Table 3 and Table 4. Any remaining volumes of the drugs are injected into the next largest injectable tumor (as determined by PI), and the injection volume and dose are according to Table 3 and Table 4. This procedure is repeated for the additional remaining volumes, until the entire total volumes and doses as determined in phase I are injected. CG0070 injection is omitted at a particular injection site when lesion at the site is no longer viable. However, the CTLA-4 inhibitor and the CD40 agonist injections are administered until the end of the treatment course into the same sites, even when a lesion disappears. Each patient receives a minimum of 8 injections of the CTLA-4 inhibitor and the CD40 agonist.

**Table 3. Injection volume per lesion based on tumor size**

| Tumor Size (longest dimension) | Maximum Injection Volume |
|---|---|
| ≥ 5.0 cm | 2.0 mL |
| ≥ 2.0 cm to 5.0 cm | 1.0 mL |
| > 0.75 cm to 2.0 cm | 0.5 mL |
| < 0.75 cm | 0.1 mL |

**Table 4. Dose of agents (immunomodulator(s) and/or immune-related molecule(s)) per lesion based on tumor size**

| Tumor Size (longest dimension) | Maximum Injection Volume |
|---|---|
| ≥ 5.0 cm | MTD/MFD agent # 1 dose and MTD/MFD agent #2 dose |
| ≥ 2.0 cm to 5.0 cm | 1/3 MTD/MFD agent #1dose and 1/3 MTD/MFD agent #2 dose |
| > 0.75 cm to 2.0 cm | 1/6 MTD/MFD agent #1 dose and 1/6 MTD/MFD agent #2 dose |
| < 0.75 cm | 1/10 MTD/MFD agent #1 dose and 1/10 MTD/MFD agent #2 dose |

There are two primary outcome measures for this study: (1) safety and tolerability; and (2) efficacy. Safety and tolerability are evaluated from the beginning of each stage until 3 months following enrollment of the last subject in each stage or Phase II. Stage 1 determines the safety and tolerability of CG0070 (e.g., with DDM) as assessed by incidence of dose-limiting toxicities (DLT) in patients with refractory solid tumor. Stage 2 determines the safety and tolerability of the CTLA-4 inhibitor (such as anti-CTLA-4 mAb or blocker, e.g., Ipilimumab) in combination with CG0070 as assessed by incidence of dose-limiting toxicities (DLT) in patients with refractory solid tumors. Stage 3 and Phase II determine the safety and tolerability of the CD40 agonist (agonistic anti-CD40 antibody, e.g., APX005M) in combination with CG0070 and the CTLA-4 inhibitor as assessed by incidence of dose-limiting toxicities (DLT) in patients with refractory solid tumors. Efficacy is evaluated from the beginning of each stage or Phase II until 24 months following enrollment of the last subject at each stage or Phase II. Efficacy is assessed by confirmed objective response rate (ORR) of the treatment with CG0070 (e.g., with DDM) alone in Stage 1, with the combination of CG0070 and the CTLA-4 inhibitor (such as anti-CTLA-4 mAb or blocker, e.g., Ipilimumab) in Stage 2, with the combination of CG0070, the CTLA-4 inhibitor and the CD40 agonist (such as agonistic anti-CD40 antibody, e.g., APX005M) in Stage 3 and in Phase II in patients with injectable refractory solid tumors.

The secondary outcome measures of this study are as follows. Safety secondary outcomes are assessed from the beginning of each stage until 24 months following enrollment of the last subject at each stage or Phase II. For all three stages and Phase II, safety secondary outcome measures include incidence of all Adverse Events (AEs), grade 3 or greater AEs, events requiring discontinuation of study drug(s), local effects on tumor, clinically significant laboratory changes and clinically significant changes in vital signs. The efficacy secondary outcomes are assessed from the beginning of each stage or Phase II until 24 months following enrollment of the last subject at each stage or Phase II. For all three stages and Phase II, efficacy secondary outcome measures include Best Overall Response Rate (BOR), Disease Control Rate (DCR), Durable Response Rate (DRR), Duration of Response (DOR), Time to Response (TTR), Progression Free Survival (PFS), Overall Survival Rate (OS), 1 year and 2 year Survival Rate.

Eligibility of patients of both genders for the study is determined based on the following inclusion criteria:

1. Patients must have histologically confirmed solid tumors that have failed standard therapies (surgery, chemotherapy, radiotherapy, or endocrine therapy) and for which no curative options exist, including, but not limited to: squamous cell carcinoma of the head and neck, squamous cell carcinoma of the skin, carcinoma of the breast, malignant melanoma, colorectal cancer, pancreatic adenocarcinoma, ovarian cancer, non-small cell lung cancer and prostate cancer;
2. Patients may have had any kind and number of prior cancer therapies;
3. Patients must have measurable lesions that are evaluable by the RECIST method;
4. The tumor mass to be treated must be assessable through a cutaneous route and adequate for injections (i.e., more than 2 cm away from major vascular structures) and measurement by RECIST;
5. Patients must be ≥ 18 years of age;
6. Patients must have a life expectancy of ≥ 12 weeks;
7. Patients must have an Eastern Cooperative Oncology Group (ECOG) performance status of 0, 1, or 2;
8. Patients must have adequate hepatic function, as defined as:

    a. Total bilirubin levels ≤ 1.5 x upper limit of normal (ULN); and
    b. AST/ALT levels ≤ 2.5 x ULN, or ≤ 5 x ULN if liver metastases are present;

9. Patients must have adequate renal function as defined as serum creatinine ≤ 1.5 x ULN or creatinine clearance (calculated) ≥ 60 mL/min/1.73m2 for patients with creatinine > 1.5 x ULN;
10. Patients must have adequate bone marrow function, as defined as:

    a. Absolute neutrophil count ≥ 1,200/μL; and
    b. Platelet count ≥ 80,000/μL;

11. Patients must have no known bleeding diathesis or coagulopathy that would make intratumoral injection or biopsy unsafe;
12. Men and women of childbearing potential must agree to use adequate contraception prior to study entry and for up to six months;
13. Females of childbearing potential must have a negative urine or serum pregnancy test within one week prior to start of treatment; and
14. Patients must be able to understand and willing to sign a written informed consent document.

The following patients are excluded from the study:

1. Patients receiving chemotherapy, immunotherapy or radiotherapy within 4 weeks prior to screening, or adverse events > Grade 1, except alopecia, resulting from agents administered more than 4 weeks prior to screening;
2. Patients with a history of significant tumor bleeding, or coagulation or bleeding disorders;
3. Patients with target tumors that could potentially invade a major vascular structure(s) (e.g., innominate artery, carotid artery), based on unequivocal imaging findings, as determined by a radiologist;
4. Patients with Grade ≥ 1 pre-existing neurologic abnormalities (CTCAE version 4.0);
5. Patients who have been hospitalized for emergent conditions requiring inpatient evaluation, treatment or procedure during the 30 days prior to entry on study. In addition, emergent conditions requiring inpatient evaluation, treatment or procedure must have resolved or be medically stable and not severe for 30 days prior to entry on study;
6. Patients with clinically evident Human Immunodeficiency Virus (HIV), Hepatitis B Virus (HBV), Hepatitis C virus (HCV), or Epstein-Barr virus (EBV) infection. Patients are tested for HIV during pre-treatment screening
7. Patients receiving steroids or immunosuppressive agents, e.g., for rheumatoid arthritis;
8. Patients who have concurrent use of any other investigational agents;
9. Patients with presence or history of central nervous system metastasis;
10. Pregnant or breastfeeding women or women desiring to become pregnant within the timeframe of the study;
11. Patients with uncontrolled inter-current illness including, but not limited to, ongoing or active infection, symptomatic congestive heart failure, unstable angina pectoris, cardiac arrhythmia, or psychiatric illness/social situations that would limit compliance with study requirements.

**Example 4: Preparation of inactivated tumor cells.**

This example describes an exemplary method of preparing inactivated tumor cells that can be used for local administration to the tumor site (such as by intratumoral injection) in combination with the infectious agent and immunomodulator.

Tumor cells can be from tumor biopsy or resection from an autologous or an allogeneic source. Alternatively, they can be harvested from established tumor cell lines or from individually developed tumor cell lines, coming from either an autologous or an allogeneic source. The tumor cells are typically isolated by gradient density centrifugation, plastic adherence and trypsinization. The isolated tumor cells are expanded through many passages to provide enough cells for the treatment. In the case of tumor cells harvested from cell lines, the cells are further washed, filtered and assayed for characterization (e.g., expression of tumor antigens), sterility and viability. The tumor cells are cryopreserved in the cell bank, or stored as aliquots ready for administration.

Preparation of Tumor Cells from a Surgical Specimen

For the usual surgical specimen, a piece of the tumor is removed for pathological classification and the main tumor cell mass is then placed into a tube with HBSS containing gentamycin and stored at 8°C. Within about 8-12 hours, the fresh tumor specimens are carried to the laboratory, where they are further dissociated. The tumor specimens are cut into smaller pieces, usually in 1 cm cubes with a scalpel. They are then incubated in an enzyme solution at 37°C. The usual enzymatic solution most effective is a mixture of collagenase, DNase, and hyaluronidase. After incubation the resulting suspension is filtered through a nylon mesh with a pore of 40μm. These steps are repeated until all the main fraction of the tumor specimen has been dissolved. The resulting cell suspension is then washed three times in HBSS and then ready for cryopreservation.

Cryopreservation and Thawing of Tumor Cells

Tumor cells isolated in this manner are then frozen in 10% human serum albumin and 10% DMSO and stored in aliquots of $10^7$ cells in liquid nitrogen. Cell freezing can be performed in a freezing computer Kryo 10 series II (Messer-Griesheim). On the day of the planned administration, the cells are carefully thawed in warm medium with the addition of 10% human serum albumin and then washed three times in this medium.

Inactivation of Tumor Cells

The tumor cells' proliferative capacity is inactivated with 200Gy using a telecobalt source prior to administration.

Preparation of Inactivated Tumor Cells for Injection to Tumor Sites

Aliquots of $10^7$ inactivated tumor cells are adjusted to an appropriate volume for intratumoral injections. For example, see Example 5. Typically, the inactivated cells can be centrifuged and reconstituted in 37C medium with 10% albumin to a volume of about 2 mL.

**Example 5: A Phase I/II Clinical Study of Intratumoral Administration of CG0070 in Combination with a CTLA-4 Inhibitor, an 4-1BB Agonist, and Inactivated Tumor Cells in Patients with Hepatocellular Carcinoma**

This example describes a Phase I/II, multicenter, open-label clinical study to evaluate the efficacy, safety and tolerability of a combination therapy including CG0070, a CTLA-4 inhibitor, a 4-1BB agonist and inactivated tumor cells for treating patients with refractory injectable liver tumors. The combination of CG0070, the CTLA-4 inhibitor, the 4-1BB agonist, and the inactivated tumor cells are administered intrahepatically into liver tumors with known progression in patients having hepatocellular carcinoma or patients having liver metastases from breast adenocarcinoma, colorectal adenocarcinoma, gastroesophageal cancer (adenocarcinoma or squamous cell carcinoma), melanoma, non-small cell lung cancer, or clear cell renal cell carcinoma.

In this study, the inactivated tumor cells are from an allogenic source. The combination of CG0070 with the inactivated tumor cells (referred to hereinafter as "VC" or "VC combination") has a fixed composition, wherein the number of the inactivated tumor cells is about at least 4 logs lower than the number of the CG0070 viral particles. For example, in combination with CG0070 at a dose of $1\times 10^{12}$ vp, about $1\times10^8$ or lower number of the inactivated tumor cells are injected per patient for each administration. The CTLA-4 inhibitor can be an anticalin that specifically recognizes CTLA-4. The anticalin can be formulated in LPGA. For example, a CTLA-4 specific anticalin in LPGA formulation at 75:25 weight ratio (referred hereinafter as anticalin/LPGA 75:25) can be used. The 4-1BB agonist can be an agonistic anti-4-1BB antibody, such as PF-05082566.

Phase I of the clinical study is divided into three stages. In Stage 1, each patient is administered the VC combination including CG0070 and the inactivated tumor cells (e.g., inactivated allogenic tumor cells) via intratumoral injections weekly (e.g., on Day 1 of each week) for six weeks. Cohorts of (e.g., three to six) patients receive the intratumoral VC at one of four dose levels. Cohorts (e.g., three to six) of patients receive weekly intratumoral injection of CG0070 (e.g., with DDM) for four weeks at one of the following four dose levels: $5 \times 10^{10}$ vp of CG0070 and $5 \times 10^{6}$ inactivated tumor cells, $1 \times 10^{11}$ vp of CG0070 and $1 \times 10^{7}$ inactivated tumor cells, $5 \times 10^{11}$ vp of CG0070 and $5 \times 10^{7}$ inactivated tumor cells, or $1 \times 10^{12}$ vp of CG0070 and $1 \times 10^{8}$ inactivated tumor cells. For example, CG0070 and the inactivated tumor cells are admixed immediately before administration in saline (e.g., with 0.1% DDM) in a total volume of 2 mL. If the patient has a single lesion, which must be greater than 2 cm, the total volume of the VC solution is injected into the lesion. If there are two or more lesions, the maximum injection volume based on the lesion size as shown in Table 1 is followed. Any remaining volume is injected into the largest lesion, if the largest lesion is at least 2 cm. If the largest lesion is less than 2 cm, then the remaining volume is divided between the two larger lesions. The maximum number of lesions injected is 3. The total dose is given regardless the total number and size of the lesions. Dose escalation procedure is as described in Example 1, and MTD/MFD is designated as Dose Level Stage 1, which is used at the beginning of Stage 2.

Stage 2 of Phase I is a dose escalation of intratumoral injection of a CTLA-4 inhibitor (e.g., anticalin/LPGA 75:25) in combination of the VC combination at Dose Level Stage 1. Cohorts (e.g., three to six) of patients receive weekly intratumoral injection of a fixed dose of CG0070 and inactivated tumor cells in combination with the CTLA-4 inhibitor (e.g., anticalin/LPGA 75:25) at one of the following three dose levels: 1.2 mg, 2.4 mg, or 3.6 mg, for six weeks. For each administration, the VC combination is first injected intratumorally according to the injection volume per lesion as defined in Stage 1. Immediately after each VC injection, the CTLA-4 inhibitor (e.g., anticalin/LPGA 75:25) is administered to the same injection sites with volumes according to Table 5. The maximum number of injected lesions is 3, and the total dose of the CTLA-4 inhibitor is given regardless the total number and size of the lesions. Any remaining volume of the CTLA-4 inhibitor is administered subcutaneously around the injected lesion(s). In case lesions completely resolved prior to the last planned treatment, both VC combination and the CTLA-4 inhibitor (e.g., anticalin/LPGA 75:25) can be administered to a previously un-injected lesion. If all lesions are resolved before the end of the treatment course, the CTLA-4 inhibitor (e.g., anticalin/LPGA 75:25) alone can be injected in the subcutaneous area at or around the former lesion. Dose escalation procedure is as described in Example 1, and MTD/MFD is designated as Dose Level Stage 2, which is used at the beginning of Stage 3.

**Table 5. Injection volume of immunomodulator per lesion based on tumor size**

| Dose level | 1.2 mg | | 2.4 mg | | 3.6 mg | |
|---|---|---|---|---|---|---|
| Tumor Size (longest dimension) | Max dose per lesion | Max Volume | Max dose per lesion | Max Volume | Max dose per lesion | Max Volume |
| ≥ 5.0 cm | 1.2 mg | 1.2 mL | 2.4 mg | 2.4 mL | 3.6 mg | 3.6 mL |
| ≥ 2.0 cm to 5.0 cm | 0.6 mg | 0.6 mL | 1.2 mg | 1.2 mL | 1.8 mg | 1.8 mL |
| > 0.5 cm to 2 cm | 0.3 mg | 0.3 mL | 0.6 mg | 0.6 mL | 0.9 mg | 0.9 mL |

Stage 3 of Phase I is a dose escalation of intratumoral injection of a 4-1BB agonist (such as a 4-1BB agonistic antibody, e.g., PF-05082566) in combination with the VC combination and the CTLA-4 inhibitor (e.g., anticalin/LPGA 75:25) at Dose Level Stage 2. Cohorts (e.g., three to six) of patients receive weekly intratumoral injection of a fixed dose of the VC combination and the CTLA-4 inhibitor (e.g., anticalin/LPGA 75:25) in combination with the 4-1BB agonist (e.g., PF-05082566) at one of the following three dose levels: 6 mg, 12 mg, or 18 mg, for six weeks. For each administration, the VC combination and the CTLA-4 inhibitor (e.g., anticalin/LPGA 75:25) at Dose Level Stage 2 is adjusted to 2 mL and injected intratumorally according to the injection volume per lesion as defined in Table 1. Immediately after each VC/CTLA-4 inhibitor injection, the 4-1BB agonist (e.g., PF-05082566) is administered. The total volume at each dose level, and the maximum injection volumes based on lesion sizes for more than two injected lesions are listed in Table 2. The maximum number of injected lesions is 3, and the total dose of the 4-1BB agonist (e.g., PF-05082566) is given regardless the total number and size of the lesions. Any remaining volume of the 4-1BB agonist (e.g., PF-05082566) is administered subcutaneously around the injected lesion(s). In case lesions completely resolved prior to the last planned treatment, the VC combination, the CTLA-4 inhibitor (e.g., anticalin/LPGA 75:25) and the 4-1BB agonist (e.g., PF-05082566) can be administered to a previously un-injected lesion. If all lesions are resolved before the end of the treatment course, the 4-1BB agonist (e.g., PF-05082566) alone can be injected in the subcutaneous area at or around the former lesion. Dose escalation procedure is as described in Example 1, and MTD/MFD is designated as the study dose, which is used in Phase II.

For Phase II of the study, the cohort of patients first receive a once weekly intratumoral injection of the four-component combination of CG0070 (e.g., with DDM), the inactivated tumor cells (e.g., inactivated allogenic tumor cells), the CTLA-4 inhibitor (e.g., anticalin/LPGA 75:25), and the 4-1BB agonist (e.g., PF-05082566) at the study dose determined in Stage 3 of Phase I for four weeks, followed by intratumoral injections of the four-component combination once every 2 weeks for four times. Afterwards, a monthly intratumoral injection of the four-component combination is administered for maintenance treatment until complete response, disappearance of all injectable tumors, confirmed disease progression or intolerance of study treatment, whichever occurs first. Patients who are in the dose escalation phase of Phase I (e.g., stage 1, 2 or 3) can be enrolled in the Phase II study as long as there is a rest period of at least four weeks from the last dose. For each administration, GC0070 and the inactivated tumor cells (e.g., allogenic inactivated tumor cells) are first admixed immediately prior to administration, injected to the lesion sites, followed by the CTLA-4 inhibitor (e.g., anticalin/LPGA 75:25) and the 4-1BB agonist (e.g., PF-05082566). The largest injectable tumor (as determined by PI) is the first tumor to be injected, and the injection volume and dose are according to Table 3 and Table 4. Any remaining volumes of the drugs are injected into the next largest injectable tumor (as determined by PI), and the injection volume and dose are according to Table 3 and Table 4. This procedure is repeated for the additional remaining volumes, until the entire total volumes and doses as determined in phase I are injected. VC combination injection is omitted at a particular injection site when lesion at the site is no longer viable. However, the CTLA-4 inhibitor and the 4-1BB agonist injections are administered until the end of the treatment course into the same sites, even when a lesion disappears. Each patient receives a minimum of 8 injections of the CTLA-4 inhibitor and the 4-1BB agonist.

There are two primary outcome measures for this study: (1) safety and tolerability; and (2) efficacy. Safety and tolerability are evaluated from the beginning of each stage or Phase II until 3 months following enrollment of the last subject in each stage or Phase II. Stage 1 determines the safety and tolerability of the combination of CG0070 (e.g., with DDM) and the inactivated tumor cells (e.g., inactivated allogenic tumor cells) as assessed by incidence of dose-limiting toxicities (DLT) in patients with refractory liver tumors. Stage 2 determines the safety and tolerability of the combination of CG0070 (e.g., with DDM), the inactivated tumor cells (e.g., inactivated allogenic tumor cells) and the CTLA-4 inhibitor (e.g., anticalin/LPGA 75:25) as assessed by incidence of dose-limiting toxicities (DLT) in patients with refractory liver tumors. Stage 3 determines the safety and tolerability of the combination of CG0070 (e.g., with DDM), the inactivated tumor cells (e.g., inactivated allogenic tumor cells), the CTLA-4 inhibitor (e.g., anticalin/LPGA 75:25), and the 4-1BB agonist (e.g., PF-05082566) as assessed by incidence of dose-limiting toxicities (DLT) in patients with refractory liver tumors. Phase II determines the safety and tolerability of the combination of CG0070 (e.g., with DDM), the inactivated tumor cells (e.g., inactivated allogenic tumor cells), the CTLA-4 inhibitor (e.g., anticalin/LPGA 75:25), and the 4-1BB agonist (e.g., PF-05082566) by incidence of dose-limiting toxicities (DLT) in patients with refractory liver tumors. Efficacy is evaluated from the beginning of each stage of Phase II until 24 months following enrollment of the last subject at each stage. Efficacy is assessed by confirmed objective response rate (ORR) of the treatment with the combination of CG0070 (e.g., with DDM) and the inactivated tumor cells (e.g., inactivated allogenic tumor cells) in Stage 1, with the combination of CG0070 (e.g., with DDM), the inactivated tumor cells (e.g., inactivated allogenic tumor cells) and the CTLA-4 inhibitor (e.g., anticalin/LPGA 75:25) in Stage 2, and with the combination of CG0070 (e.g., with DDM), the inactivated tumor cells (e.g., inactivated allogenic tumor cells), the CTLA-4 inhibitor (e.g., anticalin/LPGA 75:25), and the 4-1BB agonist (e.g., PF-05082566) in Stage 3 and in Phase II in patients with injectable refractory liver tumors.

The secondary outcome measures of this study are as follows. Safety secondary outcomes are assessed from the beginning of each stage or Phase II until 24 months following enrollment of the last subject at each stage or phase II. For all three stages and phase II, safety secondary outcome measures include incidence of all Adverse Events (AEs), grade 3 or greater AEs, events requiring discontinuation of study drug(s), local effects on tumor, clinically significant laboratory changes and clinically significant changes in vital signs. The efficacy secondary outcomes are assessed from the beginning of each stage until 24 months following enrollment of the last subject at each stage or Phase II. For all three stages and Phase II, efficacy secondary outcome measures include Best Overall Response Rate (BOR), Disease Control Rate (DCR), Durable Response Rate (DRR), Duration of Response (DOR), Time to Response (TTR), Progression Free Survival (PFS), Overall Survival Rate (OS), 1 year and 2 year Survival Rate.

Eligibility of patients of both genders for the study is determined based on the following inclusion criteria: (1) Subjects must have histologically confirmed breast adenocarcinoma, colorectal adenocarcinoma, gastroesophageal cancer (adenocarcinoma or squamous cell carcinoma), melanoma, non-small cell lung cancer, or clear cell renal cell carcinoma with liver metastases or hepatocellular carcinoma with known disease progression; (2) Non-hepatocellular carcinoma subjects must have received at least 1 prior standard of care systemic anti-cancer therapy for their metastatic disease; (3) Subjects must have measurable liver tumors that are suitable for injection; (4) Eastern Cooperative Oncology Group performance status must be 0 or 1, and life expectancy should be approximately 5 months or more. Adequate hematological, renal, hepatic and coagulation function is required; (5) Child-Pugh score must be A to B7.

The following patients are excluded from the study: (1) Subjects must not be candidates for hepatic surgery or locoregional therapy of liver tumors with curative intent or planned systemic anti-cancer therapy; (2) Liver tumors must not be estimated to invade approximately more than one third of the liver; (7) Liver tumor-directed therapy, hepatic surgery, antibody-based therapy, or immunotherapy must not have been performed < 28 days, chemotherapy < 21 days, and targeted small molecule therapy or hormonal therapy < 14 days prior to enrollment; (8) Subjects must have either no central nervous system metastasis or irradiated, stable cerebral metastases from breast adenocarcinoma, non-small cell lung cancer, clear cell renal cell carcinoma, or melanoma; (9) Subjects must not have history or evidence of symptomatic autoimmune pneumonitis, glomerulonephritis, vasculitis, or other symptomatic autoimmune disease; (10) Subjects must not have symptomatic auto-immune disease or be immunosuppressed; (11) Subjects must not have a history of solid organ transplantation; (12) For non-hepatocellular carcinoma, there must not be acute or chronic hepatitis B virus or hepatitis C virus infection; (13) For hepatocellular carcinoma, hepatitis B virus and hepatitis C virus viral load must be undetectable, and they must not have had recent treatment with certain antiviral medications; (14) There should be no macroscopic intravascular invasion of tumors into the main portal vein, hepatic vein, or vena cava; (15) Subjects must not have active herpetic skin lesions or prior complications of herpetic infection (e.g., herpetic keratitis or encephalitis) and must not require treatment with an antiherpetic drug; (16) Subjects must not require concomitant treatment with warfarin; (17) Female subjects of childbearing potential who is unwilling to use acceptable method(s) of effective contraception during protocol treatment and through 3 months after the last dose of intervention.

**Example 6: A Phase I/II Clinical Study of Radiation Pre-Treatment followed by Intratumoral Administration of CG0070 in Combination with a CTLA-4 Inhibitor and a CD40 agonist for Patients with Refractory Non Hodgkin Lymphoma, Nasopharyngeal Carcinoma and Melanoma**

This study is a multi-center, single-arm, open-label, interventional study aimed at evaluating the safety and efficacy of the combination therapy comprising radiation pre-treatment followed by intratumoral administration of CG0070, a CTLA-4 inhibitor and a CD40 agonist in patients with solid or lymphatic tumor, such as non-Hodgkin lymphoma, nasopharyngeal carcinoma or melanoma.

The radiation a pre-treatment is carried out as follows. Two days prior to each administration of the therapy (e.g., CG0070, combination of CG0070 with the CTLA-4 inhibitor, or combination of CG0070, the CTLA-4 inhibitor and the CD40 agonist), external radiation of a single dose of 2 Gy is administered to each treated tumor site of the patient daily for 2 days. The maximum dose of radiation is limited to one radiation course (2 Gy for 2 days) per month for a maximum of 4 months. After this maximum dose, all radiation will be stopped. Total radiation received should not exceed 16 Gy over the 4-month course of treatment.

The clinical study in Phase I is divided into three stages. Stage 1 is a dose escalation study for intratumoral injection of CG0070 in combination with the radiation pre-treatment. Cohorts (e.g., three to six) of patients receive weekly radiation pre-treatment followed by intratumoral injection of CG0070 (e.g., with DDM) for four weeks at one of the following four dose levels: $5 \times 10^{10}$ vp, $1 \times 10^{11}$ vp, $5 \times 10^{11}$ vp, or $1 \times 10^{12}$ vp. For example, the virus CG0070 is reconstituted in 0.1% of DDM in saline. The total volume of each dose is 2 mL. The concentration of the CG0070 solution is about $2.5 \times 10^{10}$vp/ml for the lowest dose, and about $5 \times 10^{11}$ vp/ml for the highest dose. If the patient has a single lesion, which must be greater than 2 cm, the total volume of the CG0070 solution is injected into the lesion. If there are two or more lesions, the maximum injection volume based on the lesion size as shown in Table 1 is followed. Any remaining volume is injected into the largest lesion, if the largest lesion is at least 2 cm. If the largest lesion is less than 2 cm, then the remaining volume is divided between the two larger lesions. The maximum number of lesions injected is 3. The total dose is given regardless the total number and size of the lesions. Dose escalation procedure is as described in Example 1, and MTD/MFD is designated as Dose Level Stage 1, which is used at the beginning of Stage 2.

Stage 2 of Phase I is a dose escalation of intratumoral injection of a CTLA-4 inhibitor (such as an anti-CTLA-4 mAb or blocker, e.g., Ipilimumab) in combination of CG0070 and the radiation pre-treatment at Dose Level Stage 1. Cohorts (e.g., three to six) of patients receive weekly radiation pre-treatment followed by intratumoral injection of a fixed dose of CG0070 (e.g., with DDM) in combination with the CTLA-4 inhibitor (e.g., Ipilimumab) at one of the following three dose levels: 6 mg, 12 mg, or 18 mg, for six weeks. For each administration, CG0070 is first injected intratumorally according to the injection volume per lesion as defined in Stage 1. Immediately after each CG0070 injection, the CTLA-4 inhibitor is administered. The total volume at each dose level, and the maximum injection volumes based on lesion sizes for more than two injected lesions are listed in Table 2. The maximum number of injected lesions is 3, and the total dose of the CTLA-4 inhibitor is given regardless the total number and size of the lesions. Any remaining volume of the CTLA-4 inhibitor is administered subcutaneously around the injected lesion(s). In case lesions completely resolved prior to the last planned treatment, both CG0070 and the CTLA-4 inhibitor (e.g., Ipilimumab) can be administered to a previously un-injected lesion. If all lesions are resolved before the end of the treatment course, the CTLA-4 inhibitor (e.g., Ipilimumab) alone can be injected in the subcutaneous area at or around the former lesion. Dose escalation procedure is as described in Example 1, and MTD/MFD is designated as Dose Level Stage 2, which is used at the beginning of Stage 3.

Stage 3 of Phase I is a dose escalation of intratumoral injection of a CD40 agonist (such as a CD40 agonistic antibody, e.g., APX005M) in combination with the CTLA-4 inhibitor (such as an anti-CTLA-4 mAb or blocker, e.g.,

Ipilimumab) CG0070, and the radiation pre-treatment at Dose Level Stage 2. Cohorts (e.g., three to six) of patients receive weekly radiation pre-treatment followed by intratumoral injection of a fixed dose of CG0070 (e.g., with DDM) and the CTLA-4 inhibitor (e.g., Ipilimumab) in combination with the a CD40 agonist (e.g., APX005M) at one of the following three dose levels: 6 mg, 12 mg, or 18 mg, for six weeks. For each administration, CG0070 and the CTLA-4 inhibitor (e.g., Ipilimumab) at Dose Level Stage 2 are adjusted to 2 mL and injected intratumorally according to the injection volumes per lesion as defined in Table 1. Immediately after each CG0070/CTLA-4 inhibitor injection, the CD40 agonist (e.g., APX005M) is administered. The total volume at each dose level, and the maximum injection volumes based on lesion sizes for more than two injected lesions are listed in Table 2. The maximum number of injected lesions is 3, and the total dose of the CD40 agonist (e.g., APX005M) is given regardless the total number and size of the lesions. Any remaining volume of CD40 agonist (e.g., APX005M) is administered subcutaneously around the injected lesion(s). In case lesions completely resolved prior to the last planned treatment, CG0070, the CTLA-4 inhibitor (e.g., Ipilimumab) and CD40 agonist (e.g., APX005M) can be administered to a previously un-injected lesion. If all lesions are resolved before the end of the treatment course, the CD40 agonist (e.g., APX005M) alone can be injected in the subcutaneous area at or around the former lesion. Dose escalation procedure is as described in Example 1, and MTD/MFD is designated as the study dose, which is used in Phase II.

For Phase II of the study, the cohort of patients first receive a once weekly radiation pre-treatment followed by intratumoral injection of the three-component combination of CG0070 (e.g., with DDM), the CTLA-4 inhibitor (e.g., Ipilimumab), and the CD-40 agonist (e.g., APX005M) at the study dose determined in Stage 3 of Phase I for four weeks, followed by intratumoral injections of the three-component combination once every 2 weeks for four times. Afterwards, a monthly intratumoral injection of the three-component combination is administered for maintenance treatment until complete response, disappearance of all injectable tumors, confirmed disease progression or intolerance of study treatment, whichever occurs first. Patients who are in the dose escalation phase of Phase I (e.g., stage 1, 2 or 3) can be enrolled in the Phase II study as long as there is a rest period of at least four weeks from the last dose. For each administration, GC0070 is first injected to the lesions, followed by the CTLA-4 inhibitor (e.g., Ipilimumab) and the CD40 agonist (e.g., APX005M). The largest injectable tumor (as determined by PI) is the first tumor to be injected, and the injection volume and dose are according to Table 3 and Table 4. Any remaining volumes of the drugs are injected into the next largest injectable tumor (as determined by PI), and the injection volume and dose are according to Table 3 and Table 4. This procedure is repeated for the additional remaining volumes, until the entire total volumes and doses as determined in phase I are injected. CG0070 injection is omitted at a particular injection site when lesion at the site is no longer viable. However, the CTLA-4 inhibitor and the CD40 agonist injections are administered until the end of the treatment course into the same sites, even when a lesion disappears. Each patient receives a minimum of 8 injections of the CTLA-4 inhibitor and the CD40 agonist.

There are two primary outcome measures for this study: (1) safety and tolerability; and (2) efficacy. Safety and tolerability are evaluated from the beginning of each stage until 3 months following enrollment of the last subject in each stage or Phase II. Stage 1 determines the safety and tolerability of CG0070 (e.g., with DDM) with radiation pre-treatment as assessed by incidence of dose-limiting toxicities (DLT) in patients with refractory solid or lymphatic tumors. Stage 2 determines the safety and tolerability of the CTLA-4 inhibitor (such as anti-CTLA-4 mAb or blocker, e.g., Ipilimumab) in combination with CG0070 with radiation pre-treatment as assessed by incidence of dose-limiting toxicities (DLT) in patients with refractory solid or lymphatic tumors. Stage 3 and Phase II studies determine the safety and tolerability of the CD40 agonist (agonistic anti-CD40 antibody, e.g., APX005M) in combination with CG0070 and the CTLA-4 inhibitor with radiation pre-treatment as assessed by incidence of dose-limiting toxicities (DLT) in patients with refractory solid or lymphatic tumors. Efficacy is evaluated from the beginning of each stage or Phase II until 24 months following enrollment of the last subject at each stage or Phase II. Efficacy is assessed by confirmed objective response rate (ORR) of the treatment with CG0070 (e.g., with DDM) with radiation pre-treatment in Stage 1, with the combination of CG0070 and the CTLA-4 inhibitor (such as anti-CTLA-4 mAb or blocker, e.g., Ipilimumab) with radiation pre-treatment in Stage 2, with the combination of CG0070, the CTLA-4 inhibitor and the CD40 agonist (such as agonistic anti-CD40 antibody, e.g., APX005M) with radiation pre-treatment in Stage 3 and in Phase II in patients with injectable refractory solid or lymphatic tumors.

The secondary outcome measures of this study are as follows. Safety secondary outcomes are assessed from the beginning of each stage until 24 months following enrollment of the last subject at each stage or Phase II. For all three stages and Phase II, safety secondary outcome measures include incidence of all Adverse Events (AEs), grade 3 or greater AEs, events requiring discontinuation of study drug(s), local effects on tumor, clinically significant laboratory changes and clinically significant changes in vital signs. The efficacy secondary outcomes are assessed from the beginning of each stage or Phase II until 24 months following enrollment of the last subject at each stage or Phase II. For all three stages and Phase II, efficacy secondary outcome measures include Best Overall Response Rate (BOR), Disease Control Rate (DCR), Durable Response Rate (DRR), Duration of Response (DOR), Time to Response (TTR), Progression Free Survival (PFS), Overall Survival Rate (OS), 1 year and 2 year Survival Rate.

Eligibility of patients of both genders for the study is determined based on the following inclusion criteria: (1) Patients must be ≥ 18 years of age; (2) Patients must be able to understand and willing to sign a written informed consent document; (3) Patients must have histologically confirmed Histologically confirmed malignancy (i.e., Phase I: histologically confirmed melanoma, or metastatic nasopharyngeal carcinoma; Phase II: histologically confirmed melanoma, non-Hodgkin lymphoma, or metastatic nasopharyngeal carcinoma); (4) Patients must have failed at least one systemic therapy or be intolerant to at least one prior systemic treatment; (5) Patients Must have at least two lesions of evaluable size by modified World Health Organization (mWHO)/Cheson criteria; one of two lesions must be amenable to biopsy (core or fine needle aspirate) and intratumoral injection of up to 5ml (diameter >= 10mm);(6) Patients with asymptomatic brain metastases are eligible; (systemic steroids should be avoided if possible, or the subject should be stable on the lowest clinically effective dose); (7) Patients must have an Eastern Cooperative Oncology Group (ECOG) performance status of 0, 1, or 2; (8) Patients must have a life expectancy of no less than 16 weeks; (9) Patients must have baseline (screening/baseline) radiographic images, (e.g., brain, chest, abdomen, pelvis, and bone scans with specific imaging tests to be determined by the attending physician) within 6 weeks of initiation of study; (10) Patients must have the following laboratory results: White blood cell (WBC) >= 2000/uL (~2 × 10^9/L); Absolute neutrophil count >= 1000/uL (~0.5 × 10^9/L); Platelet count >= 75 × 10^3/uL (~75 × 10^9/L); Hemoglobin >= 9 g/dL (may be transfused); Creatinine =< 2.0 x upper limit of normal (ULN); Aspartate aminotransferase (AST)/alanine aminotransferase (ALT) =< 2.5 x ULN for subjects without liver metastasis =< 5 times for liver metastases; and Bilirubin =< 2.0 x ULN (except for subjects with Gilbert's syndrome, who must have a total bilirubin of less than 3.0 mg/dL); (11) Patients must have no active or chronic infection with human immunodeficiency virus (HIV), hepatitis B, or hepatitis C; (12) Women of childbearing potential (WOCBP) must be using an adequate method of contraception to avoid pregnancy throughout the study and for up to 26 weeks after the last dose of investigational product, in such a manner that the risk of pregnancy is minimized; and (13) Men of fathering potential must be using an adequate method of contraception to avoid conception throughout the study (and for up to 26 weeks after the last dose of investigational product) in such a manner that the risk of pregnancy is minimized.

The following patients are excluded from the study: (1) Patients with any other malignancy from which the patient has been disease-free for less than 5 years, with the exception of adequately treated and cured basal or squamous cell skin cancer, superficial bladder cancer or carcinoma in situ of the cervix; (2) Patients with a history of significant tumor bleeding, or coagulation or bleeding disorders; (3) Patients with a history of inflammatory bowel disease, including ulcerative colitis and Crohn's disease, are excluded from this study, as are patients with a history of symptomatic disease (e.g., rheumatoid arthritis, systemic progressive sclerosis [scleroderma], systemic lupus erythematosus, autoimmune vasculitis [e.g., Wegener's Granulomatosis]); motor neuropathy considered of autoimmune origin (e.g., Guillain-Barre Syndrome and Myasthenia Gravis); (4) Patients with any underlying medical or psychiatric condition, which in the opinion of the investigator will make the administration of the interventional drugs hazardous or obscure the interpretation of adverse events (AEs), such as a condition associated with frequent diarrhea; (5) Patients with underlying heart conditions who are deemed ineligible for surgery by cardiology consult; (6) Patients with concomitant therapy with any of the following: interleukin-2 (IL 2), interferon, or other non-study immunotherapy regimens; cytotoxic chemotherapy; immunosuppressive agents; other investigation therapies; or chronic use of systemic corticosteroids (A history of AEs with prior IL-2 or Interferon will not preclude subjects from entering the current study); (7) Patients receiving any investigational agents; (8)Patients receiving immunosuppressive agents (unless required for treating potential AEs); and (9) Women of childbearing potential (WOCBP) who are unwilling or unable to use an acceptable method of contraception to avoid pregnancy for their entire study period and for at least 8 weeks after cessation of study drug; have a positive pregnancy test at baseline, or are pregnant or breastfeeding.

### Example 7: A Phase I/II Clinical Study of Intratumoral CCL21 Pre-Treatment Followed by Intratumoral Administration of CG0070 in Combination with a CTLA-4 Inhibitor and a CD40 agonist for Patients with Refractory Solid Tumors

This study is a multi-center, single-arm, open-label, interventional study aimed at evaluating the safety and efficacy of the combination therapy comprising an intratumoral CCL21 pre-treatment followed by intratumoral administration of CG0070, a CTLA-4 inhibitor and a CD40 agonist in patients with refractory solid tumors.

The intratumoral CCL21 pre-treatment is carried out as follows. Two days prior to each administration of the therapy (e.g., CG0070, combination of CG0070 with the CTLA-4 inhibitor, or combination of CG0070, the CTLA-4 inhibitor and the CD40 agonist), an intratumoral CCL21 nanocapsule is administered at a dose of about 200 μg/mL into each targeted tumor site. The dosage of the intratumoral CCL21 nanocapsule is about 2mL for tumors with the longest dimension exceeding 5 cm; about 1 mL for tumors with the longest dimension of 2 cm to 5 cm; and about 0.5 mL for tumors with the longest dimension of 0.5 cm to 2 cm. Intratumoral CCL21 nanocapsule is administered either weekly for six weeks in Phase I of the study, or weekly for four weeks in Phase II of the study, followed by once every 2 weeks for 4 more cycles. Afterwards, the CCL21 nanocapsule is administered intratumorally once every month until progression of disease or occurrence of toxicity events.

The clinical study in Phase I is divided into three stages. Stage 1 is a dose escalation study for intratumoral injection of CG0070 in combination with the intratumoral CCL21 nanocapsule pre-treatment. Cohorts (e.g., three to six) of patients receive weekly intratumoral CCL21 nanocapsule pre-treatment followed by intratumoral injection of CG0070 (e.g., with DDM) for four weeks at one of the following four dose levels: $5 \times 10^{10}$ vp, $1 \times 10^{11}$ vp, $5 \times 10^{11}$ vp, or $1 \times 10^{12}$ vp. For example, the virus CG0070 is reconstituted in 0.1% of DDM in saline. The total volume of each dose is 2 mL. The concentration of the CG0070 solution is about 2.5 x $10^{10}$vp/ml for the lowest dose, and about $5 \times 10^{11}$ vp/ml for the highest dose. If the patient has a single lesion, which must be greater than 2 cm, the total volume of the CG0070 solution is injected into the lesion. If there are two or more lesions, the maximum injection volume based on the lesion size as shown in Table 1 is followed. Any remaining volume is injected into the largest lesion, if the largest lesion is at least 2 cm. If the largest lesion is less than 2 cm, then the remaining volume is divided between the two larger lesions. The maximum number of lesions injected is 3. The total dose is given regardless the total number and size of the lesions. Dose escalation procedure is as described in Example 1, and MTD/MFD is designated as Dose Level Stage 1, which is used at the beginning of Stage 2.

Stage 2 of Phase I is a dose escalation of intratumoral injection of a CTLA-4 inhibitor (such as an anti-CTLA-4 mAb or blocker, e.g., Ipilimumab) in combination of CG0070 and the intratumoral CCL21 nanocapsule pre-treatment at Dose Level Stage 1. Cohorts (e.g., three to six) of patients receive weekly intratumoral CCL21 nanocapsule pre-treatment followed by intratumoral injection of a fixed dose of CG0070 (e.g., with DDM) in combination with the CTLA-4 inhibitor (e.g., Ipilimumab) at one of the following three dose levels: 6 mg, 12 mg, or 18 mg, for six weeks. For each administration, CG0070 is first injected intratumorally according to the injection volume per lesion as defined in Stage 1. Immediately after each CG0070 injection, the CTLA-4 inhibitor is administered. The total volume at each dose level, and the maximum injection volumes based on lesion sizes for more than two injected lesions are listed in Table 2. The maximum number of injected lesions is 3, and the total dose of the CTLA-4 inhibitor is given regardless the total number and size of the lesions. Any remaining volume of the CTLA-4 inhibitor is administered subcutaneously around the injected lesion(s). In case lesions completely resolved prior to the last planned treatment, both CG0070 and the CTLA-4 inhibitor (e.g., Ipilimumab) can be administered to a previously un-injected lesion. If all lesions are resolved before the end of the treatment course, the CTLA-4 inhibitor (e.g., Ipilimumab) alone can be injected in the subcutaneous area at or around the former lesion. Dose escalation procedure is as described in Example 1, and MTD/MFD is designated as Dose Level Stage 2, which is used at the beginning of Stage 3.

Stage 3 of Phase I is a dose escalation of intratumoral injection of a CD40 agonist (such as a CD40 agonistic antibody, e.g., APX005M) in combination with the CTLA-4 inhibitor (such as an anti-CTLA-4 mAb or blocker, e.g., Ipilimumab) CG0070, and the intratumoral CCL21 nanocapsule pre-treatment at Dose Level Stage 2. Cohorts (e.g., three to six) of patients receive weekly intratumoral CCL21 nanocapsule pre-treatment followed by intratumoral injection of a fixed dose of CG0070 (e.g., with DDM) and the CTLA-4 inhibitor (e.g., Ipilimumab) in combination with the a CD40 agonist (e.g., APX005M) at one of the following three dose levels: 6 mg, 12 mg, or 18 mg, for six weeks. For each administration, CG0070 and the CTLA-4 inhibitor (e.g., Ipilimumab) at Dose Level Stage 2 are adjusted to 2 mL and injected intratumorally according to the injection volumes per lesion as defined in Table 1. Immediately after each CG0070/CTLA-4 inhibitor injection, the CD40 agonist (e.g., APX005M) is administered. The total volume at each dose level, and the maximum injection volumes based on lesion sizes for more than two injected lesions are listed in Table 2. The maximum number of injected lesions is 3, and the total dose of the CD40 agonist (e.g., APX005M) is given regardless the total number and size of the lesions. Any remaining volume of CD40 agonist (e.g., APX005M) is administered subcutaneously around the injected lesion(s). In case lesions completely resolved prior to the last planned treatment, CG0070, the CTLA-4 inhibitor (e.g., Ipilimumab) and CD40 agonist (e.g., APX005M) can be administered to a previously un-injected lesion. If all lesions are resolved before the end of the treatment course, the CD40 agonist (e.g., APX005M) alone can be injected in the subcutaneous area at or around the former lesion. Dose escalation procedure is as described in Example 1, and MTD/MFD is designated as the study dose, which is used in Phase II.

For Phase II of the study, the cohort of patients first receive a once weekly intratumoral CCL21 nanocapsule pre-treatment followed by intratumoral injection of the three-component combination of CG0070 (e.g., with DDM), the CTLA-4 inhibitor (e.g., Ipilimumab), and the CD-40 agonist (e.g., APX005M) at the study dose determined in Stage 3 of Phase I for four weeks, followed by intratumoral injections of the three-component combination once every 2 weeks for four times. Afterwards, a monthly intratumoral injection of the three-component combination is administered for maintenance treatment until complete response, disappearance of all injectable tumors, confirmed disease progression or intolerance of study treatment, whichever occurs first. Patients who are in the dose escalation phase of Phase I (e.g., stage 1, 2 or 3) can be enrolled in the Phase II study as long as there is a rest period of at least four weeks from the last dose. For each administration, GC0070 is first injected to the lesions, followed by the CTLA-4 inhibitor (e.g., Ipilimumab) and the CD40 agonist (e.g., APX005M). The largest injectable tumor (as determined by PI) is the first tumor to be injected, and the injection volume and dose are according to Table 3 and Table 4. Any remaining volumes of the drugs are injected into the next largest injectable tumor (as determined by PI), and the injection volume and dose are according to Table 3 and Table 4. This procedure is repeated for the additional remaining volumes, until the entire total volumes and doses as determined in phase I are injected. CG0070 injection is omitted at a particular injection site when lesion at the site is no longer viable. However, the CTLA-4 inhibitor and the CD40 agonist injections are administered until the end of the treatment course into the same sites, even when a lesion disappears. Each patient receives a minimum of 8 injections of the CTLA-4 inhibitor and the CD40 agonist.

There are two primary outcome measures for this study: (1) safety and tolerability; and (2) efficacy. Safety and tolerability are evaluated from the beginning of each stage until 3 months following enrollment of the last subject in each stage or Phase II. Stage 1 determines the safety and tolerability of CG0070 (e.g., with DDM) with the intratumoral CCL21 pre-treatment as assessed by incidence of dose-limiting toxicities (DLT) in patients with refractory solid or lymphatic tumors. Stage 2 determines the safety and tolerability of the CTLA-4 inhibitor (such as anti-CTLA-4 mAb or blocker, e.g., Ipilimumab) in combination with CG0070 with the intratumoral CCL21 pre-treatment as assessed by incidence of dose-limiting toxicities (DLT) in patients with refractory solid or lymphatic tumors. Stage 3 and Phase II studies determine the safety and tolerability of the CD40 agonist (agonistic anti-CD40 antibody, e.g., APX005M) in combination with CG0070 and the CTLA-4 inhibitor with the intratumoral CCL21 pre-treatment as assessed by incidence of dose-limiting toxicities (DLT) in patients with refractory solid or lymphatic tumors. Efficacy is evaluated from the beginning of each stage or Phase II until 24 months following enrollment of the last subject at each stage or Phase II. Efficacy is assessed by confirmed objective response rate (ORR) of the treatment with CG0070 (e.g., with DDM) with the intratumoral CCL21 pre-treatment in Stage 1, with the combination of CG0070 and the CTLA-4 inhibitor (such as anti-CTLA-4 mAb or blocker, e.g., Ipilimumab) with the intratumoral CCL21 pre-treatment in Stage 2, with the combination of CG0070, the CTLA-4 inhibitor and the CD40 agonist (such as agonistic anti-CD40 antibody, e.g., APX005M) with the intratumoral CCL21 pre-treatment in Stage 3 and in Phase II in patients with injectable refractory solid or lymphatic tumors.

The secondary outcome measures of this study are as follows. Safety secondary outcomes are assessed from the beginning of each stage until 24 months following enrollment of the last subject at each stage or Phase II. For all three stages and Phase II, safety secondary outcome measures include incidence of all Adverse Events (AEs), grade 3 or greater AEs, events requiring discontinuation of study drug(s), local effects on tumor, clinically significant laboratory changes and clinically significant changes in vital signs. The efficacy secondary outcomes are assessed from the beginning of each stage or Phase II until 24 months following enrollment of the last subject at each stage or Phase II. For all three stages and Phase II, efficacy secondary outcome measures include Best Overall Response Rate (BOR), Disease Control Rate (DCR), Durable Response Rate (DRR), Duration of Response (DOR), Time to Response (TTR), Progression Free Survival (PFS), Overall Survival Rate (OS), 1 year and 2 year Survival Rate.

Eligibility of patients of both genders for the study is determined based on the following inclusion criteria: (1) Patients must have histologically confirmed solid tumors that have failed standard therapies (surgery, chemotherapy, radiotherapy, or endocrine therapy) and for which no curative options exist, including, but not limited to: squamous cell carcinoma of the head and neck, squamous cell carcinoma of the skin, carcinoma of the breast, malignant melanoma, colorectal cancer, pancreatic adenocarcinoma, ovarian cancer, non-small cell lung cancer and prostate cancer; (2) Patients may have had any kind and number of prior cancer therapies; (3) Patients must have measurable lesions that are evaluable by the RECIST method; (4) The tumor mass to be treated must be adequate for injections (i.e., more than 2 cm away from major vascular structures) and measurement by RECIST; (5) Patients must be $\geq$ 18 years of age; (6) Patients must have a life expectancy of $\geq$ 12 weeks; (7) Patients must have an Eastern Cooperative Oncology Group (ECOG) performance status of 0, 1, or 2; (8) Patients must have adequate hepatic function, as defined as: Total bilirubin levels $\leq$ 1.5 x upper limit of normal (ULN); and AST/ALT levels $\leq$ 2.5 x ULN, or $\leq$ 5 x ULN if liver metastases are present; (9) Patients must have adequate renal function as defined as serum creatinine $\leq$ 1.5 x ULN or creatinine clearance (calculated) $\geq$ 60 mL/min/1.73m2 for patients with creatinine > 1.5 x ULN; (10) Patients must have adequate bone marrow function, as defined as: Absolute neutrophil count $\geq$ 1,200/$\mu$L; and Platelet count $\geq$ 80,000/$\mu$L; (11) Patients must have no known bleeding diathesis or coagulopathy that would make intratumoral injection or biopsy unsafe; (12) Men and women of childbearing potential must agree to use adequate contraception prior to study entry and for up to six months; (13) Females of childbearing potential must have a negative urine or serum pregnancy test within one week prior to start of treatment; and (14) Patients must be able to understand and willing to sign a written informed consent document.

The following patients are excluded from the study: (1) Patients receiving chemotherapy, immunotherapy or radiotherapy within 4 weeks prior to screening, or adverse events > Grade 1, except alopecia, resulting from agents administered more than 4 weeks prior to screening; (2) Patients with a history of significant tumor bleeding, or coagulation or bleeding disorders; Patients with target tumors that could potentially invade a major vascular structure(s) (e.g., innominate artery, carotid artery), based on unequivocal imaging findings, as determined by a radiologist; (3) Patients with Grade $\geq$ 1 pre-existing neurologic abnormalities (CTCAE version 4.0); (4) Patients who have been hospitalized for emergent conditions requiring inpatient evaluation, treatment or procedure during the 30 days prior to entry on study. In addition, emergent conditions requiring inpatient evaluation, treatment or procedure must have resolved or be medically stable and not severe for 30 days prior to entry on study; (5) Patients with clinically evident Human Immunodeficiency Virus (HIV), Hepatitis B Virus (HBV), Hepatitis C virus (HCV), or Epstein-Barr virus (EBV) infection. Patients are tested for HIV during pre-treatment screening; (6) Patients receiving steroids or immunosuppressive agents, e.g., for rheumatoid arthritis; (7) Patients who have concurrent use of any other investigational agents; (8) Patients with presence or history of central nervous system metastasis; (9) Pregnant or breastfeeding women or women desiring to become pregnant within the timeframe of the study;(10) Patients with uncontrolled inter-current illness including, but not limited to, ongoing or active infection, symptomatic congestive heart failure, unstable angina pectoris, cardiac arrhythmia, or psychiatric illness/social situations that would limit compliance with study requirements.

**Example 8: A Phase I/II Clinical Study of Intratumoral CpG Pre-Treatment Followed by Intratumoral Administration of CG0070 in Combination with a CTLA-4 Inhibitor and an OX40 agonist for Patients with Refractory Solid Tumors**

This study is a multi-center, single-arm, open-label, interventional study aimed at evaluating the safety and efficacy of the combination therapy comprising an intratumoral CpG pre-treatment followed by intratumoral administration of CG0070 in combination with a CTLA-4 inhibitor and an OX40 agonist in patients with refractory solid tumors.

The intratumoral CpG pre-treatment is carried out as follows. Two days prior to each administration of the therapy (e.g., CG0070, combination of CG0070 with the CTLA-4 inhibitor, or combination of CG0070, the CTLA-4 inhibitor and the OX40 agonist), an intratumoral CpG (such as CpG 7909) is administered at a dose of about 1 mg /mL into each targeted tumor site. The injection volume of the intratumoral CpG is about 2 mL for tumors with the longest dimension exceeding 5 cm; about 1 mL for tumors with the longest dimension of 2 cm to 5 cm; and about 0.5 mL for tumors with the longest dimension of 0.5 cm to 2 cm. Intratumoral CpG is administered either weekly for six weeks in Phase I of the study, or weekly for four weeks in Phase II of the study, followed by once every 2 weeks for 4 more cycles. Afterwards, the CpG is administered intratumorally once every month until progression of disease or occurrence of toxicity events.

The clinical study in Phase I is divided into three stages. Stage 1 is a dose escalation study for intratumoral injection of CG0070 in combination with the intratumoral CpG (e.g., CpG 7909) pre-treatment. Cohorts (e.g., three to six) of patients receive weekly intratumoral CpG (e.g., CpG 7909) pre-treatment followed by intratumoral injection of CG0070 (e.g., with DDM) for four weeks at one of the following four dose levels: $5 \times 10^{10}$ vp, $1 \times 10^{11}$ vp, $5 \times 10^{11}$ vp, or $1 \times 10^{12}$ vp. For example, the virus CG0070 is reconstituted in 0.1% of DDM in saline. The total volume of each dose is 2 mL. The concentration of the CG0070 solution is about $2.5 \times 10^{10}$vp/ml for the lowest dose, and about $5 \times 10^{11}$ vp/ml for the highest dose. If the patient has a single lesion, which must be greater than 2 cm, the total volume of the CG0070 solution is injected into the lesion. If there are two or more lesions, the maximum injection volume based on the lesion size as shown in Table 1 is followed. Any remaining volume is injected into the largest lesion, if the largest lesion is at least 2 cm. If the largest lesion is less than 2 cm, then the remaining volume is divided between the two larger lesions. The maximum number of lesions injected is 3. The total dose is given regardless the total number and size of the lesions. Dose escalation procedure is as described in Example 1, and MTD/MFD is designated as Dose Level Stage 1, which is used at the beginning of Stage 2.

Stage 2 of Phase I is a dose escalation of intratumoral injection of a CTLA-4 inhibitor (such as an anti-CTLA-4 mAb or blocker, e.g., Ipilimumab) in combination of CG0070 and the intratumoral CpG pre-treatment at Dose Level Stage 1. Cohorts (e.g., three to six) of patients receive weekly intratumoral CpG (e.g., CpG 7909) pre-treatment followed by intratumoral injection of a fixed dose of CG0070 (e.g., with DDM) in combination with the CTLA-4 inhibitor (e.g., Ipilimumab) at one of the following three dose levels: 6 mg, 12 mg, or 18 mg, for six weeks. For each administration, CG0070 is first injected intratumorally according to the injection volume per lesion as defined in Stage 1. Immediately after each CG0070 injection, the CTLA-4 inhibitor is administered. The total volume at each dose level, and the maximum injection volumes based on lesion sizes for more than two injected lesions are listed in Table 2. The maximum number of injected lesions is 3, and the total dose of the CTLA-4 inhibitor is given regardless the total number and size of the lesions. Any remaining volume of the CTLA-4 inhibitor is administered subcutaneously around the injected lesion(s). In case lesions completely resolved prior to the last planned treatment, both CG0070 and the CTLA-4 inhibitor (e.g., Ipilimumab) can be administered to a previously un-injected lesion. If all lesions are resolved before the end of the treatment course, the CTLA-4 inhibitor (e.g., Ipilimumab) alone can be injected in the subcutaneous area at or around the former lesion. Dose escalation procedure is as described in Example 1, and MTD/MFD is designated as Dose Level Stage 2, which is used at the beginning of Stage 3.

Stage 3 of Phase I is a dose escalation of intratumoral injection of an OX40 agonist (such as an OX40 agonistic antibody, e.g., MEDI-6469) in combination with the CTLA-4 inhibitor (such as an anti-CTLA-4 mAb or blocker, e.g., Ipilimumab) CG0070, and the intratumoral CpG (e.g., CpG 7909) pre-treatment at Dose Level Stage 2. Cohorts (e.g., three to six) of patients receive weekly intratumoral CpG pre-treatment followed by intratumoral injection of a fixed dose of CG0070 (e.g., with DDM) and the CTLA-4 inhibitor (e.g., Ipilimumab) in combination with the OX40 agonist (e.g., MEDI-6469) at one of the following three dose levels: 6 mg, 12 mg, or 18 mg, for six weeks. For each administration, CG0070 and the CTLA-4 inhibitor (e.g., Ipilimumab) at Dose Level Stage 2 are adjusted to 2 mL and injected intratumorally according to the injection volumes per lesion as defined in Table 1. Immediately after each CG0070/CTLA-4 inhibitor injection, the OX40 agonist (e.g., MEDI-6469) is administered. The total volume at each dose level, and the maximum injection volumes based on lesion sizes for more than two injected lesions are listed in Table 2. The maximum number of injected lesions is 3, and the total dose of the OX40 agonist (e.g., MEDI-6469) is given regardless the total number and size of the lesions. Any remaining volume of OX40 agonist (e.g., MEDI-6469) is administered subcutaneously around the injected lesion(s). In case lesions completely resolved prior to the last planned treatment, CG0070, the CTLA-4 inhibitor (e.g., Ipilimumab) and OX40 agonist (e.g., MEDI-6469) can be administered to a previously un-injected lesion. If all lesions are resolved before the end of the treatment course, the OX40 agonist (e.g., MEDI-6469) alone can be injected in the subcutaneous area at or around the former lesion. Dose escalation procedure is as described in Example 1, and MTD/MFD is designated as the study dose, which is used in Phase II.

For Phase II of the study, the cohort of patients first receive a once weekly intratumoral CpG pre-treatment followed by intratumoral injection of the three-component combination of CG0070 (e.g., with DDM), the CTLA-4 inhibitor (e.g., Ipilimumab), and the OX-40 agonist (e.g., MEDI-6469) at the study dose determined in Stage 3 of Phase I for four weeks, followed by intratumoral injections of the three-component combination once every 2 weeks for four times. Afterwards, a monthly intratumoral injection of the three-component combination is administered for maintenance treatment until complete response, disappearance of all injectable tumors, confirmed disease progression or intolerance of study treatment, whichever occurs first. Patients who are in the dose escalation phase of Phase I (e.g., stage 1, 2 or 3) can be enrolled in the Phase II study as long as there is a rest period of at least four weeks from the last dose. For each administration, GC0070 is first injected to the lesions, followed by the CTLA-4 inhibitor (e.g., Ipilimumab) and the OX-40 agonist (e.g., MEDI-6469). The largest injectable tumor (as determined by PI) is the first tumor to be injected, and the injection volume and dose are according to Table 3 and Table 4. Any remaining volumes of the drugs are injected into the next largest injectable tumor (as determined by PI), and the injection volume and dose are according to Table 3 and Table 4. This procedure is repeated for the additional remaining volumes, until the entire total volumes and doses as determined in phase I are injected. CG0070 injection is omitted at a particular injection site when lesion at the site is no longer viable. However, the CTLA-4 inhibitor and the OX-40 agonist (e.g., MEDI-6469) injections are administered until the end of the treatment course into the same sites, even when a lesion disappears. Each patient receives a minimum of 8 injections of the CTLA-4 inhibitor and the OX-40 agonist (e.g., MEDI-6469).

There are two primary outcome measures for this study: (1) safety and tolerability; and (2) efficacy. Safety and tolerability are evaluated from the beginning of each stage until 3 months following enrollment of the last subject in each stage or Phase II. Stage 1 determines the safety and tolerability of CG0070 (e.g., with DDM) with the intratumoral CpG pre-treatment as assessed by incidence of dose-limiting toxicities (DLT) in patients with refractory solid or lymphatic tumors. Stage 2 determines the safety and tolerability of the CTLA-4 inhibitor (such as anti-CTLA-4 mAb or blocker, e.g., Ipilimumab) in combination with CG0070 with the intratumoral CpG pre-treatment as assessed by incidence of dose-limiting toxicities (DLT) in patients with refractory solid or lymphatic tumors. Stage 3 and Phase II studies determine the safety and tolerability of the OX40 agonist (agonistic anti- OX40 antibody, e.g., MEDI-6469) in combination with CG0070 and the CTLA-4 inhibitor with the intratumoral CpG pre-treatment as assessed by incidence of dose-limiting toxicities (DLT) in patients with refractory solid or lymphatic tumors. Efficacy is evaluated from the beginning of each stage or Phase II until 24 months following enrollment of the last subject at each stage or Phase II. Efficacy is assessed by confirmed objective response rate (ORR) of the treatment with CG0070 (e.g., with DDM) with the intratumoral CpG pre-treatment in Stage 1, with the combination of CG0070 and the CTLA-4 inhibitor (such as anti-CTLA-4 mAb or blocker, e.g., Ipilimumab) with the intratumoral CpG pre-treatment in Stage 2, with the combination of CG0070, the CTLA-4 inhibitor and the OX40 agonist (such as agonistic anti-OX40 antibody, e.g., MEDI-6469) with the intratumoral CpG pre-treatment in Stage 3 and in Phase II in patients with injectable refractory solid or lymphatic tumors.

The secondary outcome measures of this study are as follows. Safety secondary outcomes are assessed from the beginning of each stage until 24 months following enrollment of the last subject at each stage or Phase II. For all three stages and Phase II, safety secondary outcome measures include incidence of all Adverse Events (AEs), grade 3 or greater AEs, events requiring discontinuation of study drug(s), local effects on tumor, clinically significant laboratory changes and clinically significant changes in vital signs. The efficacy secondary outcomes are assessed from the beginning of each stage or Phase II until 24 months following enrollment of the last subject at each stage or Phase II. For all three stages and Phase II, efficacy secondary outcome measures include Best Overall Response Rate (BOR), Disease Control Rate (DCR), Durable Response Rate (DRR), Duration of Response (DOR), Time to Response (TTR), Progression Free Survival (PFS), Overall Survival Rate (OS), 1 year and 2 year Survival Rate.

Eligibility of patients of both genders for the study is determined based on the following inclusion criteria: (1) Patients must have histologically confirmed solid tumors that have failed standard therapies (surgery, chemotherapy, radiotherapy, or endocrine therapy) and for which no curative options exist, including, but not limited to: squamous cell carcinoma of the head and neck, squamous cell carcinoma of the skin, carcinoma of the breast, malignant melanoma, colorectal cancer, pancreatic adenocarcinoma, ovarian cancer, non-small cell lung cancer and prostate cancer; (2) Patients may have had any kind and number of prior cancer therapies; (3) Patients must have measurable lesions that are evaluable by the RECIST method; (4) The tumor mass to be treated must be adequate for injections (i.e., more than 2 cm away from major vascular structures) and measurement by RECIST; (5) Patients must be $\geq$ 18 years of age; (6)

Patients must have a life expectancy of ≥ 12 weeks; (7) Patients must have an Eastern Cooperative Oncology Group (ECOG) performance status of 0, 1, or 2; (8) Patients must have adequate hepatic function, as defined as: Total bilirubin levels ≤ 1.5 x upper limit of normal (ULN); and AST/ALT levels ≤ 2.5 x ULN, or ≤ 5 x ULN if liver metastases are present; (9) Patients must have adequate renal function as defined as serum creatinine ≤ 1.5 x ULN or creatinine clearance (calculated) ≥ 60 mL/min/1.73m$^2$ for patients with creatinine > 1.5 x ULN; (10) Patients must have adequate bone marrow function, as defined as: Absolute neutrophil count ≥ 1,200/μL; and Platelet count ≥ 80,000/μL; (11) Patients must have no known bleeding diathesis or coagulopathy that would make intratumoral injection or biopsy unsafe; (12) Men and women of childbearing potential must agree to use adequate contraception prior to study entry and for up to six months; (13) Females of childbearing potential must have a negative urine or serum pregnancy test within one week prior to start of treatment; and (14) Patients must be able to understand and willing to sign a written informed consent document.

The following patients are excluded from the study: (1) Patients receiving chemotherapy, immunotherapy or radiotherapy within 4 weeks prior to screening, or adverse events > Grade 1, except alopecia, resulting from agents administered more than 4 weeks prior to screening; (2) Patients with a history of significant tumor bleeding, or coagulation or bleeding disorders; Patients with target tumors that could potentially invade a major vascular structure(s) (e.g., innominate artery, carotid artery), based on unequivocal imaging findings, as determined by a radiologist; (3) Patients with Grade ≥ 1 pre-existing neurologic abnormalities (CTCAE version 4.0); (4) Patients who have been hospitalized for emergent conditions requiring inpatient evaluation, treatment or procedure during the 30 days prior to entry on study. In addition, emergent conditions requiring inpatient evaluation, treatment or procedure must have resolved or be medically stable and not severe for 30 days prior to entry on study; (5) Patients with clinically evident Human Immunodeficiency Virus (HIV), Hepatitis B Virus (HBV), Hepatitis C virus (HCV), or Epstein-Barr virus (EBV) infection. Patients are tested for HIV during pre-treatment screening; (6) Patients receiving steroids or immunosuppressive agents, e.g., for rheumatoid arthritis; (7) Patients who have concurrent use of any other investigational agents; (8) Patients with presence or history of central nervous system metastasis; (9) Pregnant or breastfeeding women or women desiring to become pregnant within the timeframe of the study;(10) Patients with uncontrolled inter-current illness including, but not limited to, ongoing or active infection, symptomatic congestive heart failure, unstable angina pectoris, cardiac arrhythmia, or psychiatric illness/social situations that would limit compliance with study requirements.

**Example 9: *In vivo* study of intratumoral administration of Ar20-1004 in combination with anti-CTLA-4 antibody and/or anti-PD-L1 antibody in squamous cell lung carcinoma mouse allograft model.**

This example describes an *in vivo* study of efficacy of oncolytic adenovirus Ar20-1004, administered alone or in combination with anti-CTLA-4 antibody 9H10 and/or anti-PD-L1 antibody WBP315 in the KLN 205 murine squamous cell lung carcinoma mouse allograft model. Efficacy was assessed by monitoring tumor growth and metastasis. Ar20-1004 is a conditionally replicating oncolytic adenovirus having the same construct as CG0070 except for expressing mouse GM-CSF (CG0070 expresses human GM-CSF). Due to the presence of the tumor-selective E2F-1 promoter, Ar20-1004 selectively replicates in and selectively kills tumor cells with Rb-pathway defects. The cell death event and expressed GM-CSF can stimulate immune responses against distant uninfected metastases. Ar20-1004 has been described in US2008/0118470.

Materials and methods

Ar20-1004 (1.2 × 10^12 vp/mL) and anti-PD-L1 (WBP315) (5.6 mg/mL) were prepared at Cold Genesys Inc. and stored at -80 °C prior to sue. Anti-CTLA-4 9H10 and Hamster Polyclonal IgG, supplied as 6.15 mg/mL and 9.55 mg/mL stock solutions, respectively, were purchased from BioX cell (West Lebanon, NH). All dosing solutions were prepared freshly each day and solutions were combined for an entire group of animals prior to dosing. Anti-PD-L1 (WBP315), anti-CTLA-4 9H10, and hamster IgG isotype were each diluted in PBS to yield dosing solutions at 1 mg/mL.

KLN 205 tumor cells were inoculated in the right and left flanks of female DBA/2 mice. Tumors on the left side were implanted four days after the right side was implanted. Treatment began on Day (D) 1 in eight groups of mice (n = 10) with established subcutaneous KLN 205 tumors, when tumors in the right flank reached a group mean volume of 99 -102 mm$^3$. All agents were administered intratumorally on D1, D4, D7, and D10 to the right flank tumors. Ar20-1004 was administered at 1 × 10$^{10}$ pfu/animal. Anti-CTLA-4, hamster polyclonal IgG, and anti-PD-L1 were each administered at 20 μg/animal. Control animals were untreated. Animal groups and dosing schemes are summarized in FIG. 2.

Tumors were measured on both flanks twice per week. The study endpoint was defined as a mean tumor volume of 1000 mm$^3$ in the right flank of the control group or 35 days, whichever came first. The study ended on D23 when the control group reached tumor volume endpoint. Treatment outcome was based on percent tumor growth inhibition (%TGI), defined as the percent difference between the median tumor volumes (MTVs) of treated and control mice on D19 (total sum of bilateral tumors volumes). The results were analyzed using the Mann-Whitney U test, and were deemed statistically significant at $P < 0.05$.

Results were also analyzed by counting the lung metastatic foci on D23, the last day of the study. Animals were sacrificed at endpoint using isoflurane anesthesia and necropsies were performed to identify metastases. Total counts were obtained by adding the number of foci counted in the superior, middle, inferior, and post-caval lobes of the right lung to the number of foci counted in the left lung. Percent inhibition was defined as the difference between the number of metastatic foci of the designated control group and the number of metastatic foci of the drug-treated group, expressed as a percentage of the number of metastatic foci of the designated control.

$$\% \text{ Inhibition} = [1-(\#\text{Foci drug-treated}/\#\text{Foci control})] \times 100.$$

Results were analyzed using the Kruskal-Wallis test and were deemed statistically significant at $P < 0.05$. Treatment tolerability was assessed by body weight (BW) measurements and frequent observation for clinical signs of treatment-related (TR) side effects.

Results

This study characterized the antitumor responses induced by Ar20-1004 in the KLN 205 murine squamous cell lung carcinoma allograft model. Responses were also evaluated when Ar20-1004 was administered in combination with anti-CTLA-4 and/or anti-PD-L1. Tumors were measured twice per week through D23, and TGI analysis was performed on D19. Lung metastatic foci were counted on D23. All treatments were well-tolerated.

Ar20-1004, anti-CTLA-4 and anti-PD-L1 alone or in combination showed no significant tumor growth inhibition when administered intratumorally in the KLN 205 murine squamous cell lung carcinoma model in female DBA/2 mice (FIG. 4). Ar20-1004, antiCTLA-4 and anti-PD-L1 monotherapies inhibited metastasis by 71%, 60% and 66% respectively, but these outcomes were not statistically significant compared to the untreated group. Similarly, Ar20-1004 in combination with anti-CTLA-4, or the dual therapy of anti-CTLA-4 and antiPD-L1 (without Ar20-1004) inhibited metastasis by 69% and 74% respectively, which were not statistically significant.

Notably, combination therapy including Ar20-1004 and anti-PD-L1 resulted in a significant 84% inhibition in metastases count, and the triple combination therapy including Ar20-1004, anti-PD-L1, and anti-CTLA-4 resulted in a significant 94% inhibition of metastasis foci (FIG. 3).

**Example 10: *In vivo* study of intratumoral administration of Ar20-1004 in combination with intratumoral anti-CTLA-4 9H10 and/or local irradiation in 4T1 syngeneic mouse model.**

This example describes an *in vivo* study that evaluates the anti-tumor immune responses induced by Ar20-1004 alone or in combination with CTLA-4-blockade and/or irradiation at the primary tumor site, as well as the treatment impact on metastasis in 4T1 syngeneic mouse model.

4T1 is a Rb-pathway defective mouse breast cancer cell line. As a preliminary step, the antitumor effect of Ar20-1004, a human adenovirus (Ad5) derivative, with mouse specific GM-CSF sequence, is assessed by 4T1 cell viability and toxicity (*i.e.*, GM-CSF production) assays. As a positive control, LNCap clone FGC (ATCC® CRL-1740™) human cell line is used in the same *in vitro* assays. Triplicate wells of cancer cells are infected with Ar20-1004 at MOI of 10, 100, and 1000 respectively for 24 hours. Cell viability is assessed via MTT assay at 24 hour, 72 hours, and 120 hours post infection. Cell supernatants are collected at 24 hours, 72 hours and 120 hours post infection, and tested for total GM-CSF protein by ELISA.

In the *in vivo* study, 8-12-week old female BALB/c mice are each injected with $10^4$ 4T1 tumor cells orthotopically in the $4^{th}$ inguinal mammary fat pad. A pair match is performed when tumors reach an average size of 50 - 100 $mm^3$, and mice are randomized into treatment groups as shown in Table 1 to begin treatment. The dosing scheme is as shown in FIG. 5. Irradiation is given at 5 Gy, 1 day before administering the $1^{st}$ dose of Ar20-1004 (treatment regimen 1) and optionally anti-CTLA-4 antibody 9H10 (BioXell) or Syrian Hamster IgG2 isotype control (BioXell, treatment regimen 2). The mice are treated 4 times at a 3-day interval with treatment regimens 1 and 2 as listed in Table 6. Each time, all agents are combined into one syringe for a single dose intratumoral administration. The total dose volume is no more than 50 $\mu$l/dose/mouse.

Table 6.

| Group | N | Treatment regimen 1 Ar20-1004 (IT) $1\times10^{10}$ pfu/dose | Treatment regimen 2 Anti-CTLA-4 (IT) or Syrian Hamster IgG2 (isotype control, IT) 30 $\mu$g/dose/animal | Treatment regimen 3 Irradiation at the primary tumor sites |
|---|---|---|---|---|
| 1 | Look-See Group | - | - | - |
| 2 | 10 | - | - | - |
| 3 | 10 | normal saline | Syrian Hamster IgG2 | - |
| 4 | 10 | + | Syrian Hamster IgG2 | - |
| 5 | 10 | normal saline | Anti-CTLA-4 | - |
| 6 | 10 | + | Anti-CTLA-4 | - |
| 7 | 10 | - | - | + |
| 8 | 10 | normal saline | Syrian Hamster IgG2 | + |
| 9 | 10 | + | Syrian Hamster IgG2 | + |
| 10 | 10 | normal saline | Anti-CTLA-4 | + |
| 11 | 10 | + | Anti-CTLA-4 | + |

The study has two major endpoints: (1) Tumor growth inhibition (TGI); and (2) metastasis count. Animals are also checked for any effects of treatments on normal behavior such as mobility, visual estimation of food and water consumption, body weight gain/loss (body weights are measured at the working day in the first week and then twice weekly after randomization), eye/hair matting and any other abnormal effect. Any adverse reactions or death are reported.

To monitor tumor growth, the volumes of the primary tumors are measured using a caliper. Individual animals with a single observation of > than 30% body weight loss or three consecutive measurements of >25% body weight loss are euthanized. Any group with a mean body weight loss of >20 % or >10% mortality is stopped dosing, but the group is not euthanized and recovery is allowed. Within a group with >20% weight loss, individuals hitting the individual body weight loss endpoint are euthanized. If the group treatment related body weight loss is recovered to within 10% of the original weights, dosing may resume at a lower dose or less frequent dosing schedule.

To determine the metastasis count, 2-3 animals from the Look See group are euthanized every two days beginning on Day 12. Lungs of the animals are removed from each mouse with minimal bronchus, and tumor foci on the surface of the lung are stained using India Ink and counted. When 50-100 metastasis foci per lung set are observed in the Look See group, the metastasis count endpoint is reached, and all mice are euthanized and their lung metastasis foci are counted. %TGI is also calculated based on tumor volume measurements taken on the last day of the study when all animals are assessed for metastasis. Gross necropsy is also performed on all animals at termination to identify any metastasis by India ink staining at the injection site, regional lymph nodes, lungs, liver, kidneys, spleen and brain.

## Claims

1. In combination, an infectious agent and immunomodulator for use in a method of treating a solid or lymphatic tumor in an individual, wherein said method comprises: a) locally administering to the site of the tumor an effective amount of said infectious agent, wherein the infectious agent is an oncolytic virus; and b) locally administering to the site of the tumor said effective amount of an immunomodulator, wherein the immunomodulator is an inhibitor of PD-L1.

2. The combination for use of claim 1, wherein the infectious agent is administered: (a) directly into the tumor; or (b) to the tissue having the tumor.

3. The combination of claim 1 or 2, wherein the infectious agent and the immunomodulator are administered sequentially or simultaneously.

4. The combination for use of any one of claims 1-3, wherein the method comprises local administration of at least two immunomodulators, wherein the method comprises local administration of a CTLA-4 inhibitor and a PD-L1 inhibitor.

5. The combination for use of any one of claims 1-4, further comprising locally administering to the site of the tumor an immune-related molecule, wherein the immune-related molecule is selected from the group consisting of GM-CSF, IL-2, IL-12, interferon, CCL4, CCL19, CCL21, CXCL13, TLR1, TLR2, TLR3, TLR4, TLR5, TLR6, TLR7, TLR8, TLR9, TLR10, RIG-I, MDA5, LGP2, LTαβ, cyclic dinucleotides, CpG oligodeoxynucleotides, Imiquimod, Poly I:C, GS-9620, AED-1419, CYT-003-QbG10, AVE-0675, and PF-7909.

6. The combination for use of claim 5, wherein the immune-related molecule is expressed by the infectious agent, wherein the infectious agent comprises a nucleic acid encoding the immune-related molecule.

7. The combination for use of any one of claims 1-6, wherein the infectious agent is an adenovirus serotype 5, wherein the endogenous E1a promoter and E3 19kD coding region of a native adenovirus is replaced by the human E2F-1 promoter and a nucleic acid encoding human GM-CSF.

8. The combination for use of any one of claims 1-7, wherein the infectious agent is CG0070.

9. The combination for use of any one of claims 1-8: (a) further comprising locally administering to the site of the tumor a pretreatment composition prior to the administration of the infectious agent; and/or (b) wherein the individual is subject to a prior therapy prior to the administration of the infectious agent and the immunomodulator.

10. The combination for use of any one of claims 1-9, wherein the solid or lymphatic tumor is bladder cancer, optionally wherein the infectious agent is administered intravesically.

11. The combination for use of any one of claims 1-10, wherein the method inhibits metastasis of the solid or lymphatic tumor in the individual.

12. An infectious agent for use in a method of treating a solid or lymphatic tumor in an individual, wherein said method comprises: a) locally administering to the site of the tumor an effective amount of said infectious agent, wherein the infectious agent is an oncolytic virus; and b) locally administering to the site of the tumor said effective amount of an immunomodulator, wherein the immunomodulator is an inhibitor of PD-L1.

13. An immunomodulator for use in a method of treating a solid or lymphatic tumor in an individual, wherein said method comprises: a) locally administering to the site of the tumor an effective amount of an infectious agent, wherein the infectious agent is an oncolytic virus; and b) locally administering to the site of the tumor said effective amount of said immunomodulator, wherein the immunomodulator is an inhibitor of PD-L1.

14. The combination for use of any one of claims 1 to 11, the infectious agent of claim 12, or the immunomodulator of claim 13, wherein the inhibitor of PD-L1 is an anti-PD-L1 antibody, optionally wherein the anti-PD-L1 antibody is selected from the group consisting of KY-1003, MCLA-145, RG7446, BMS935559, MPDL3280A, MEDI4736, Avelumab and STI-A1010.

15. The combination for use of claim 4, wherein the CTLA-4 inhibitor is an anti-CTLA-4 antibody, optionally wherein the anti-CTLA-4 antibody is selected from the group consisting of Ipilimumab, Tremilimumab, and a single chain anti-CTLA-4 antibody.

**Patentansprüche**

1. In Kombination ein Infektionserreger und Immunmodulator zur Verwendung in einem Verfahren des Behandelns eines soliden oder lymphatischen Tumors in einem Individuum, wobei das Verfahren Folgendes umfasst: a) lokales Verabreichen einer wirksamen Menge des Infektionserregers an die Stelle des Tumors, wobei der Infektionserreger ein onkolytisches Virus ist; und b) lokales Verabreichen der wirksamen Menge eines Immunmodulators an die Stelle des Tumors, wobei der Immunmodulator ein Hemmer von PD-L1 ist.

2. Kombination zur Verwendung nach Anspruch 1, wobei der Infektionserreger wie folgt verabreicht wird: (a) direkt in den Tumor; oder (b) an das Gewebe, das den Tumor aufweist.

3. Kombination nach Anspruch 1 oder 2, wobei der Infektionserreger und der Immunmodulator sequentiell oder simultan verabreicht werden.

4. Kombination zur Verwendung nach einem der Ansprüche 1-3, wobei das Verfahren die lokale Verabreichung von mindestens zwei Immunmodulatoren umfasst, wobei das Verfahren lokale Verabreichung eines CTLA-4-Hemmers und eines PD-L1-Hemmers umfasst.

5. Kombination zur Verwendung nach einem der Ansprüche 1-4, ferner umfassend das lokale Verabreichen eines immunvermittelten Moleküls an die Stelle des Tumors, wobei das immunvermittelte Molekül ausgewählt ist aus der Gruppe bestehend aus GM-CSF, IL-2, IL-12, Interferon, CCL4, CCL19, CCL21, CXCL13, TLR1, TLR2, TLR3, TLR4, TLR5, TLR6, TLR7, TLR8, TLR9, TLR10, RIG-I, MDA5, LGP2, LTαβ, zyklischen Dinukleotiden, CpG-Oligodesoxynukleotiden, Imiquimod, Poly I:C, GS-9620, AED-1419, CYT-003-QbG10, AVE-0675 und PF-7909.

6. Kombination zur Verwendung nach Anspruch 5, wobei das immunvermittelte Molekül durch den Infektionserreger exprimiert wird, wobei der Infektionserreger eine Nukleinsäure umfasst, die das immunvermittelte Molekül kodiert.

7. Kombination zur Verwendung nach einem der Ansprüche 1-6, wobei der Infektionserreger ein Adenovirus Serotyp 5 ist, wobei der endogene E1a-Promotor und die E3-19kD-kodierende Region eines nativen Adenovirus durch den menschlichen E2F-1-Promotor und eine Nukleinsäure ersetzt wird, die menschliches GM-CSF kodiert.

8. Kombination zur Verwendung nach einem der Ansprüche 1-7, wobei der Infektionserreger CG0070 ist.

9. Kombination zur Verwendung nach einem der Ansprüche 1-8: (a) ferner umfassend das lokale Verabreichen einer Vorbehandlungszusammensetzung an die Stelle des Tumors vor der Verabreichung des Infektionserregers; und/oder (b) wobei das Individuum einer vorherigen Therapie vor der Verabreichung des Infektionserregers und des Immunmodulators unterliegt.

10. Kombination zur Verwendung nach einem der Ansprüche 1-9, wobei der solide oder lymphatische Tumor Blasenkrebs ist, wobei optional der Infektionserreger intravesikal verabreicht wird.

11. Kombination zur Verwendung nach einem der Ansprüche 1-10, wobei das Verfahren Metastasenbildung des soliden oder lymphatischen Tumors in dem Individuum hemmt.

12. Infektionserreger zur Verwendung in einem Verfahren des Behandelns eines soliden oder lymphatischen Tumors in einem Individuum, wobei das Verfahren Folgendes umfasst: a) lokales Verabreichen einer wirksamen Menge des Infektionserregers an die Stelle des Tumors, wobei der Infektionserreger ein onkolytisches Virus ist; und b) lokales Verabreichen der wirksamen Menge eines Immunmodulators an die Stelle des Tumors, wobei der Immunmodulator ein Hemmer von PD-L1 ist.

13. Immunmodulator zur Verwendung in einem Verfahren des Behandelns eines soliden oder lymphatischen Tumors in einem Individuum, wobei das Verfahren Folgendes umfasst: a) lokales Verabreichen einer wirksamen Menge eines Infektionserregers an die Stelle des Tumors, wobei der Infektionserreger ein onkolytisches Virus ist; und b) lokales Verabreichen der wirksamen Menge des Immunmodulators an die Stelle des Tumors, wobei der Immunmodulator ein Hemmer von PD-L1 ist.

14. Kombination zur Verwendung nach einem der Ansprüche 1 bis 11, Infektionserreger nach Anspruch 12 oder Immunmodulator nach Anspruch 13, wobei der Hemmer von PD-L1 ein Anti-PD-L1-Antikörper ist, wobei optional der Anti-PD-L1-Antikörper ausgewählt ist aus der Gruppe bestehend aus KY-1003, MCLA-145, RG7446, BMS935559, MPDL3280A, MEDI4736, Avelumab und STI-A1010.

15. Kombination zur Verwendung nach Anspruch 4, wobei der CTLA-4-Hemmer ein Anti-CTLA-4-Antikörper ist, wobei optional der Anti-CTLA-4-Antikörper ausgewählt ist aus der Gruppe bestehend aus Ipilimumab, Tremelimumab und einem Einzelketten-Anti-CTLA-4-Antikörper.

## Revendications

1. En combinaison, un agent infectieux et un immunomodulateur destinés à être utilisés dans un procédé de traitement d'une tumeur solide ou lymphatique chez un individu, ledit procédé comprenant : a) l'administration locale au site de la tumeur d'une quantité efficace dudit agent infectieux, dans laquelle l'agent infectieux est un virus oncolytique ; et b) l'administration locale au site de la tumeur ladite quantité efficace d'un immunomodulateur, l'immunomodulateur étant un inhibiteur de PD-L1.

**2.** Combinaison à utiliser selon la revendication 1, dans laquelle l'agent infectieux est administré : (a) directement dans la tumeur ; ou (b) au tissu portant la tumeur.

**3.** Combinaison selon la revendication 1 ou 2, dans laquelle l'agent infectieux et l'immunomodulateur sont administrés séquentiellement ou simultanément.

**4.** Combinaison à utiliser selon l'une quelconque des revendications 1 à 3, dans laquelle le procédé comprend l'administration locale d'au moins deux immunomodulateurs, dans laquelle le procédé comprend l'administration locale d'un inhibiteur de CTLA-4 et d'un inhibiteur de PD-L1.

**5.** Combinaison à utiliser selon l'une quelconque des revendications 1 à 4, comprenant en outre l'administration locale au site de la tumeur d'une molécule à caractère immunitaire, dans laquelle la molécule à caractère immunitaire est sélectionnée dans le groupe constitué de GM-CSF, IL-2, IL-12, interféron, CCL4, CCL19, CCL21, CXCL13, TLR1, TLR2, TLR3, TLR4, TLR5, TLR6, TLR7, TLR8, TLR9, TLR10, RIG-I, MDA5, LGP2, LT$\alpha\beta$, dinucléotides cycliques, oligodésoxynucléotides CpG, Imiquimod , Poly I:C, GS-9620, AED-1419, CYT-003-QbG10, AVE-0675 et PF-7909.

**6.** Combinaison à utiliser selon la revendication 5, dans laquelle la molécule à caractère immunitaire est exprimée par l'agent infectieux, dans laquelle l'agent infectieux comprend un acide nucléique codant pour la molécule à caractère immunitaire.

**7.** Combinaison à utiliser selon l'une quelconque des revendications 1 à 6, dans laquelle l'agent infectieux est un adénovirus de sérotype 5, dans laquelle le promoteur E1a endogène et la région codante E3 19kD d'un adénovirus natif sont remplacés par le promoteur E2F-1 humain et un acide nucléique codant pour le GM-CSF humain.

**8.** Combinaison à utiliser selon l'une quelconque des revendications 1 à 7, dans laquelle l'agent infectieux est CG0070.

**9.** Combinaison à utiliser selon l'une quelconque des revendications 1 à 8 : (a) comprenant en outre l'administration locale au site de la tumeur d'une composition de prétraitement avant l'administration de l'agent infectieux ; et/ou (b) dans lequel l'individu est soumis à une thérapie préalable avant l'administration de l'agent infectieux et de l'immunomodulateur.

**10.** Combinaison à utiliser selon l'une quelconque des revendications 1 à 9, dans laquelle la tumeur solide ou lymphatique est un cancer de la vessie, éventuellement dans laquelle l'agent infectieux est administré par voie intravésicale.

**11.** Combinaison à utiliser selon l'une quelconque des revendications 1 à 10, dans laquelle le procédé inhibe les métastases de la tumeur solide ou lymphatique chez l'individu.

**12.** Agent infectieux à utiliser dans un procédé de traitement d'une tumeur solide ou lymphatique chez un individu, ledit procédé comprenant : a) l'administration locale au site de la tumeur d'une quantité efficace dudit agent infectieux, l'agent infectieux étant un virus oncolytique ; et b) l'administration locale au site de la tumeur ladite quantité efficace d'un immunomodulateur, l'immunomodulateur étant un inhibiteur de PD-L1.

**13.** Immunomodulateur à utiliser dans un procédé de traitement d'une tumeur solide ou lymphatique chez un individu, ledit procédé comprenant : a) l'administration locale au site de la tumeur d'une quantité efficace d'un agent infectieux, l'agent infectieux étant un virus oncolytique ; et b) l'administration locale au site de la tumeur ladite quantité efficace dudit immunomodulateur, l'immunomodulateur étant un inhibiteur de PD-L1.

**14.** Combinaison à utiliser selon l'une quelconque des revendications 1 à 11, agent infectieux selon la revendication 12 ou immunomodulateur selon la revendication 13, dans laquelle l'inhibiteur de PD-L1 est un anticorps anti-PD-L1, éventuellement dans laquelle l'anticorps anti-PD-L1 est sélectionné dans le groupe constitué de KY-1003, MCLA-145, RG7446, BMS935559, MPDL3280A, MEDI4736, Avelumab et STI-A1010.

**15.** Combinaison à utiliser selon la revendication 4, dans laquelle l'inhibiteur de CTLA-4 est un anticorps anti-CTLA-4, éventuellement dans laquelle l'anticorps anti-CTLA-4 est choisi dans le groupe constitué de l'Ipilimumab, du Tremilimumab et d'un anticorps anti-CTLA-4 à chaîne unique.

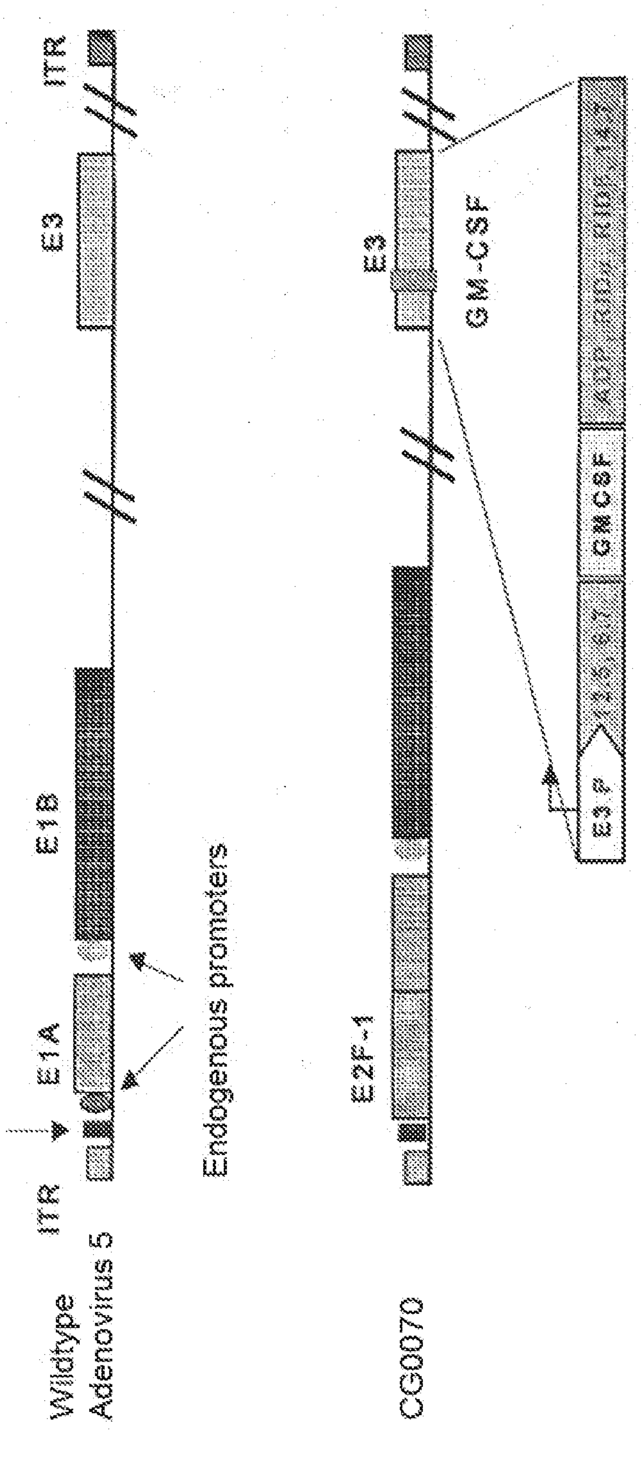
Wildtype
Adenovirus 5
ITR
E1A
E1B
E3
ITR
Endogenous promoters
CG0070
E2F-1
E3
GM-CSF
E3 P
GMCSF

FIG. 1

| Summary on groups and dosing scheme | | | | | | | | | | | | | |
|---|---|---|---|---|---|---|---|---|---|---|---|---|---|
| | | Regimen 1: Ar20-1004 | | | | Regimen 2: anti-CTLA4 | | | | Regimen 3: anti-PD-L1 | | | |
| Gr. | N | Agent | µg/animal | Route | Schedule | Agent | µg/animal | Route | Schedule | Agent | µg/animal | Route | Schedule |
| 1 | 10 | No Treatment | - | - | - | No Treatment | - | - | - | No Treatment | - | - | - |
| 2 | 10 | Normal Saline | - | IT | days 1,4,7,10 | anti-CTLA4 9H10 | 20 | IT | days 1,4,7,10 | Normal Saline | - | IT | days 1,4,7,10 |
| 3 | 10 | Ar20-1004 | 1E+10 | IT | days 1,4,7,10 | Hamster Polyclonal IgG | 20 | IT | days 1,4,7,10 | Normal Saline | - | IT | days 1,4,7,10 |
| 4 | 10 | Ar20-1004 | 1E+10 | IT | days 1,4,7,10 | anti-CTLA4 9H10 | 20 | IT | days 1,4,7,10 | Normal Saline | - | IT | days 1,4,7,10 |
| 5 | 10 | Normal Saline | - | IT | days 1,4,7,10 | Hamster Polyclonal IgG | 20 | IT | days 1,4,7,10 | anti-PD-L1 (WBP315) | 20 | IT | days 1,4,7,10 |
| 6 | 10 | Ar20-1004 | 1E+10 | IT | days 1,4,7,10 | Normal Saline | 20 | IT | days 1,4,7,10 | anti-PD-L1 (WBP315) | 20 | IT | days 1,4,7,10 |
| 7 | 10 | Normal Saline | - | IT | days 1,4,7,10 | anti-CTLA4 9H10 | 20 | IT | days 1,4,7,10 | anti-PD-L1 (WBP315) | 20 | IT | days 1,4,7,10 |
| 8 | 10 | Ar20-1004 | 1E+10 | IT | days 1,4,7,10 | anti-CTLA4 9H10 | 20 | IT | days 1,4,7,10 | anti-PD-L1 (WBP315) | 20 | IT | days 1,4,7,10 |

**FIG. 2**

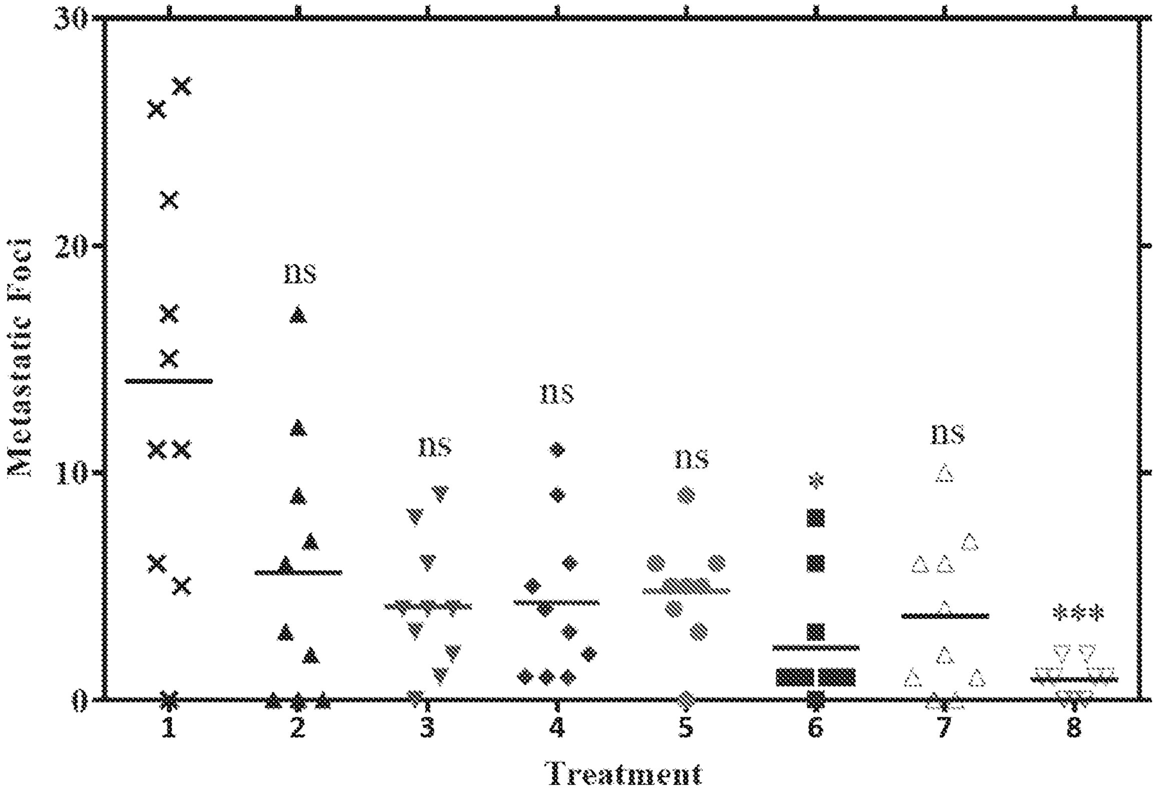


FIG. 3

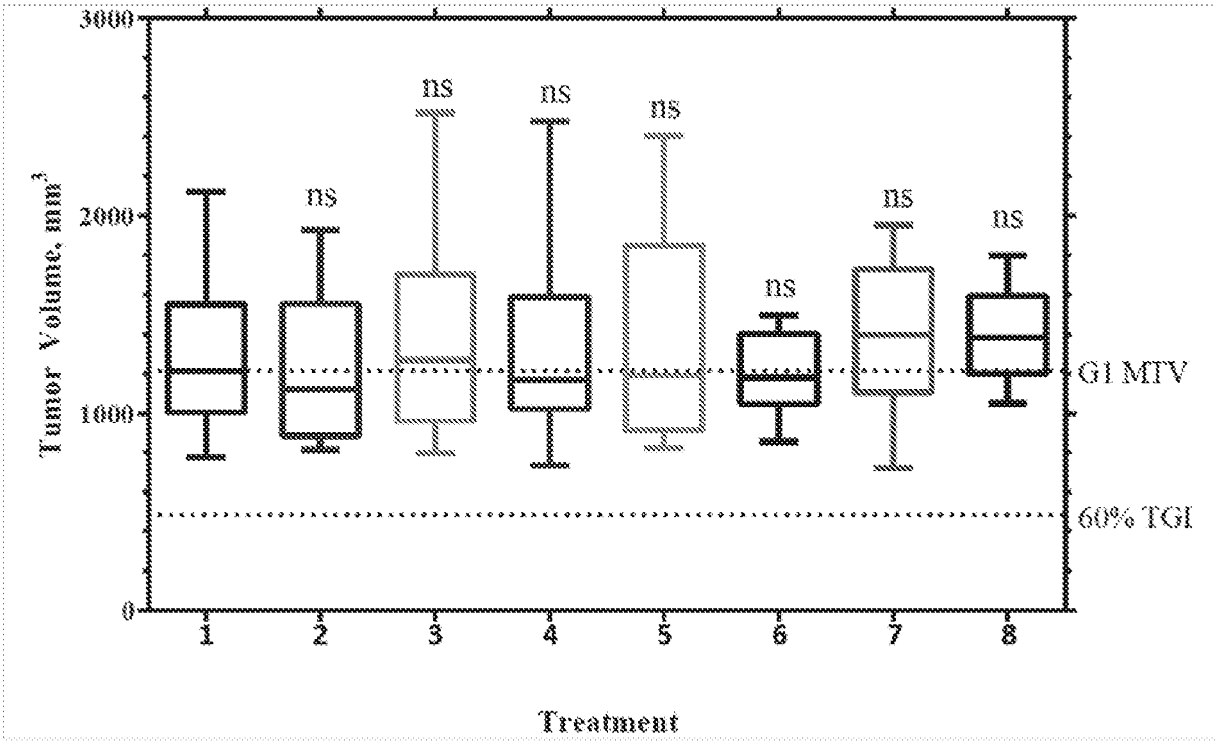
Tumor Volume, mm³
3000
2000
1000
0
ns
ns
ns
ns
ns
ns
ns
G1 MTV
60% TGI
1
2
3
4
5
6
7
8
Treatment

FIG. 4

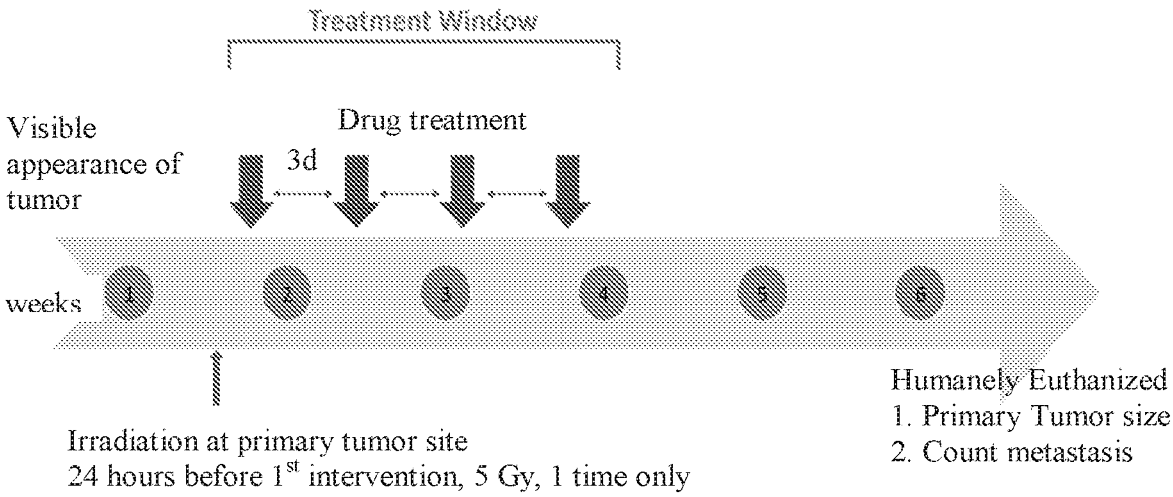
Treatment Window
Visible appearance of tumor
Drug treatment
3d
weeks
1
2
3
4
5
6
Irradiation at primary tumor site
24 hours before 1st intervention, 5 Gy, 1 time only
Humanely Euthanized
1. Primary Tumor size
2. Count metastasis

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2015190505 A
- EP 20120194977 A
- US 7709214 B
- US 7432059 B
- US 7722868 B
- US 8217149 B
- US 8383796 B
- US 9102725 B
- US 2001020964 W
- US 6984720 B
- US 7452535 B
- US 7605238 B
- US 8017114 B
- US 8142778 B
- US 668736 A
- US 7109003 B
- US 7132281 B
- US 7411057 B
- US 7807797 B
- US 7824679 B
- US 8143379 B
- US 5811097 A
- US 6051227 A
- US 7229628 B
- US 20110044953 A
- US 7250297 A
- WO 2012072806 A
- US 7101550 B
- US 5698520 A
- US 6808710 B
- US 7029674 B
- US 7794710 B
- US 7892540 B
- US 8008449 B
- US 8088905 B
- US 8163503 B
- US 8168757 B
- US 8354509 B
- US 8460927 B
- US 8609089 B
- US 8747833 B
- US 8779105 B
- US 8900587 B
- US 8952136 B
- US 8981063 B
- US 8993731 B
- US 9062112 B
- US 9067999 B
- US 9073994 B
- US 9084776 B
- US 9102728 B
- US 7488802 B
- US 20020055139 A
- US 20140044738 A
- US 7943743 B
- US 8552154 B
- US 20140341917
- US 20150203580 A
- US 20080118470 A


### Non-patent literature cited in the description


- **BURKE JM et al.** *Journal of Urology Dec,* 2012, vol. 188 (6), 2391-7
- **ISHIHARA M. et al.** *PLOS ONE,* 2014, vol. 9, e104669
- **BRAMANTE S.** *Faculty of Medicine of the University of Helsinki Academic Dissertation,* 02 October 2015, 1-101
- **TURNIS et al.** *OncoImmunology,* 2012, vol. 1 (7), 1172-1174